# EUROPEAN PATENT APPLICATION

(11) **EP 4 293 016 A1**
(43) Date of publication of application: **20.12.2023**
(21) Application number: 22770617.3
(22) Date of filing: 18.03.2022
(51) Int. Cl.: C07D 239/94, C07D 401/14, A61K 31/517, A61P 35/00, A61P 27/02, A61P 17/06, A61P 17/00, A61P 17/14, A61P 19/02, A61P 1/06, A61P 9/10, A61P 37/02, A61P 35/02

(54) **QUINAZOLINE-BASED COMPOUND, COMPOSITION, AND APPLICATION OF QUINAZOLINE-BASED COMPOUND**

(30) Priority: 19.03.2021 CN 202110296394
(71) Applicant: Beijing Scitech-MQ Pharmaceuticals Limited, Beijing 101320 (CN)
(72) Inventor: ZHANG, Qiang, Beijing 101320 (CN); YANG, Leifu, Beijing 101320 (CN)
(74) Representative: Maiwald GmbH
(86) International application number: PCT/CN2022/081599
(87) International publication number: WO 2022/194265

(57) **Abstract**

Provided are a quinazoline-based compound, a composition, and an application of the quinazoline-based compound, in particular, relating to a compound represented by formula (I), a stereoisomer thereof, a pharmaceutically acceptable salt or deuterated derivative thereof, a composition thereof, and an application thereof in the preparation of a drug that serves as a tyrosine kinase inhibitor. The compound has good inhibitory activity against EGFR, HER2 kinase, and their exon 20 mutations and EGFRviii mutations.

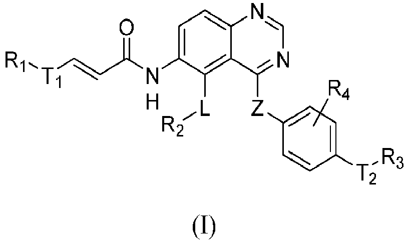

## Description

### FIELD OF THE INVENTION

The present disclosure belongs to the field of pharmaceutical technology and relates to quinazoline-based compounds, compositions and applications thereof.

### BACKGROUND OF THE INVENTION

Epidermal growth factor receptor (ErbB) tyrosine kinases can regulate cell proliferation, migration, differentiation, apoptosis, and cell movement through multiple pathways. ErbB family members, as well as some of their ligands, are commonly overexpressed, amplified, or mutated in many forms of malignancy, making them important therapeutic targets. This family of protein kinases includes ErbB1/EGFR/HER1, ErbB2/HER2, ErbB3/HER3, and ErbB4/HER4, of which EGFR and HER2 are important targets for the development of drugs for non-small cell lung cancer and breast cancer (Dienstmann R., et. al., (2001) Personalizing Therapy with Targeted Agents in Non-Small Cell Lung Cancer. ONCOTARGET. 2(3), 165.; Mitri Z., et. al. (2012) The HER2 Receptor in Breast Cancer: Pathophysiology, Clinical Use, and New Advances in Therapy., Chemotherapy Research & Practice., Volum 2012 (23), 743193.). Currently, several generations of EGFR and HER2 kinase inhibitors are also on the market.

However, the expression of EGFR and HER2 is not stable, and frequent amplification and rearrangement of genes occur, which changes the antigenic phenotype on the surface of tumor cells. The efficacy of the existing targeted drugs on different mutations of EGFR and HER2 varies greatly, wherein the inhibitory ability against Ins20 is the weakest, causing the Ins20 mutation to become a drug-resistant mutation for which the current multiple generations of targeted drugs are almost ineffective. The exon 20 mutations of EGFR and HER2 genes occur in similar positions, but there are many types of EGFR exon 20 insertion mutations, of which 122 kinds have been found; in contrast, there are fewer types of HER2 gene exon 20 insertion mutations, the most common being the A775_G776insYVMA site, which accounts for nearly 70% of the total number of mutations. Statistics also show that about 3% of NSCLC patients carry HER2 mutations, wherein about 90% are patients with exon 20 mutant types of the HER2 gene. For these EGFR/HER2 exon 20 mutation patients, the efficacy of existing targeted TKI drugs is very limited.

Meanwhile, there are also a small number of research projects targeting the EGFR/HER2 Ins20 mutation. Poziotinib is a broad-spectrum HER inhibitor developed by Hanmi. Clinical data show that Poziotinib also has certain effects on exon 20 mutations of EGFR/HER2, but has a high rate of adverse reactions. At the same time, the marketed pyrotinib is also undergoing clinical research on related exon 20 mutations.

In addition, in recent years, EGFRvIII, a class of mutants of the epidermal growth factor receptor EGFR expressed only on the surface of tumor cells rather than on the surface of cells of normal tissues, has also been identified, and is also a very common EGFR mutant. In contrast to the intact structure of EGFR, exons 2-7 encoding the extracellular ligand-binding region of EGFRvIII are deleted, resulting in an 801-base pair deletion that allows exons 1 and 8 to be linked and a new glycine to be generated at this binding site, causing the deletion of amino acids 6 to 273, and thus a loss of the ability to bind to the ligand EGF. EGFRvIII, in the absence of ligand binding, structurally activates tyrosine kinases in an unregulated manner by dimerization and autophosphorylation, inducing downstream signaling and stimulating tumor cell proliferation. Therefore, the development of new molecularly targeted therapeutics against EGFRvIII would provide more effective and cost-effective treatment options for tumor patients, and there is a huge unmet clinical need.

From the perspective of a chemical structure, many TKI drugs on the market, as well as poziotinib and pyrotinib as mentioned above, are all derived from the structure of quinazoline or quinoline. As shown in the figure below, when constructing a TKI inhibitor through quinazoline or quinoline, an aniline-based group is usually introduced at the 4-position, and substitutions are made at the 6-position and the 7-position at the same time. The pyrotinib, neratinib, and poziotinib shown in the figure below are all substituted with an alkoxy group at the 7-position, and have also a substituent at the 6-position. However, there are few attempts to construct TKI inhibitors by substitution at the 5-position. In this application, a series of quinazoline compounds have been synthesized by introducing a substituent at the 5-position and removing a substituent at the 7-position, which exhibit excellent inhibitory activity against HER2 and exon 20 mutations thereof, and are expected to be used in the treatment of HER2 exon 20 mutations.

It is worth mentioning that none of the currently marketed small molecule HER2 inhibitors can effectively penetrate the blood-brain barrier, and brain metastasis occurs in about 50% of HER2-positive breast cancer patients. In view of the fact that some compounds of the present application can effectively penetrate the blood-brain barrier and have an extremely strong inhibitory effect on HER2-positive tumors, the compounds of the present application are also expected to be used in the treatment of patients with brain metastasis from HER2-positive breast cancer.

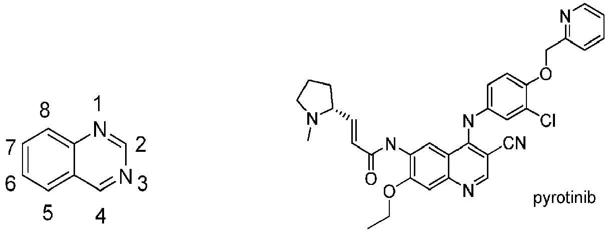

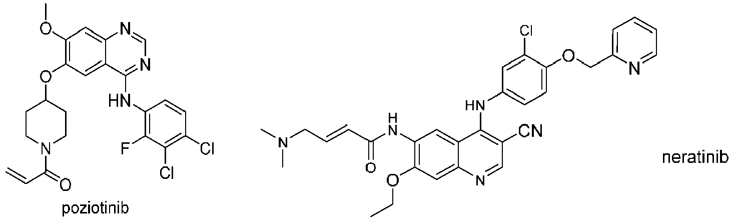

### SUMMARY OF THE INVENTION

One aspect of the present disclosure provides a compound represented by formula (I), a stereoisomer thereof, a pharmaceutically acceptable salt thereof, or a deuterated derivative thereof,

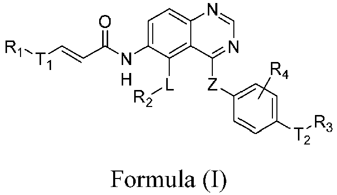

In formula (I), Z is -NH- or -O-;
T₁ is -(CH₂)ₙ-, wherein n is an integer from 0 to 3;
R₁ is hydrogen, hydroxy, 4- to 7-membered heteroalicyclic group or -NR^{a}R^{b},
R^{a} and R^{b} are each independently hydrogen, C₁-C₆ alkyl, C₃-C₆ cycloalkyl, hydroxy-substituted C₁-C₆ alkyl, C₁-C₃ alkoxy-substituted C₁-C₆ alkyl, or C₃-C₆ cycloalkyl-substituted C₁-C₆ alkyl;
the 4- to 7-membered heteroalicyclic group is a heteroalicyclic group containing 1-2 heteroatoms selected from N, O and S, wherein the heteroalicyclic group is unsubstituted or substituted with one or two of C₁-C₃ alkyl, C₁-C₄ alkylacyl, hydroxy, cyano, aminoacyl, mono- or di-substituted C₁-C₃ aminoacyl, C₁-C₃ alkoxy-substituted C₁-C₃ alkyl, hydroxy-substituted C₁-C₃ alkyl;
L is -O-, -S- or -NH-;
R₂ is C₁-C₆ alkyl, C₁-C₆ haloalkyl, hydroxy-substituted C₁-C₆ alkyl, C₁-C₃ alkoxy-substituted C₁-C₆ alkyl, or -(CH₂)_{M}-R₅,
R₅ is C₃-C₆ cycloalkyl or 4- to 6-membered heteroalicyclic group, and m is an integer from 0 to 3,
the 4- to 6-membered heteroalicyclic group is a heteroalicyclic group containing 1-2 heteroatoms selected from N, O and S;
T₂ is -M-(CH₂)p-, wherein M is O, S or NH, and p is an integer from 0 to 2,
R₃ is an aryl or heteroaryl group selected from phenyl, pyridyl, pyrimidinyl, and pyrrolyl, and the aryl or heteroaryl is unsubstituted or substituted with 1 to 3 substituents selected from halogen, cyano, hydroxy, C₁-C₃ alkyl, C₁-C₃ alkoxy, C₁-C₃ haloalkyl, C₃-C₄ cycloalkyl, C₂-C₃ alkynyl, C₂-C₃ alkenyl and -NR'R",
R', R" are each independently H or C₁-C₃ alkyl;
R₄ is hydrogen, halogen, C₁-C₃ alkyl or C₁-C₃ alkoxy.

According to an alternative embodiment, Z is -NH-.

According to an alternative embodiment, T₁ is -(CH₂)ₙ-, wherein n is an integer from 0 to 2,
R₁ is hydrogen, 4- to 6-membered heteroalicyclic group or -NR^{a}R^{b},
R^{a} and R^{b} are each independently hydrogen, C₁-C₃ alkyl, C₃-C₄ cycloalkyl, hydroxy-substituted C₁-C₃ alkyl, C₁-C₃ alkoxy-substituted C₁-C₃ alkyl, or C₃-C₆ cycloalkyl-substituted C₁-C₃ alkyl;
the 4- to 6-membered heteroalicyclic group is pyrrolidinyl, piperidinyl, piperazinyl, morpholinyl, tetrahydrofuranyl, tetrahydropyranyl, or thiomorpholinyl, and the above groups are unsubstituted or substituted with one or two of methyl, ethyl, propyl, isopropyl, aldehyde group, acetyl, propionyl, hydroxy, cyano, aminoacyl, methoxymethyl, methoxyethyl, methoxypropyl, ethoxymethyl, ethoxyethyl, ethoxypropyl, propoxymethyl, propoxyethyl, propoxypropyl, isopropoxymethyl, isopropoxyethyl, isopropoxypropyl, hydroxymethyl, hydroxyethyl and hydroxypropyl.

Still alternatively, T₁ is -(CH₂)ₙ-, wherein n is 0 or 1;
R₁ is dimethylamino, diethylamino, dipropylamino, diisopropylamino, methylethylamino, methylpropylamino, ethylpropylamino, methylcyclopropylamino, ethylcyclopropylamino, propylcyclopropylamino, isopropyl cyclopropylamino, methylcyclobutylamino, ethyl cyclobutyl amino, propylcyclobutylamino, isopropylcyclobutylamino, pyrrolidin-1-yl, pyrrolidin-2-yl, 1-methylpyrrolidin-2-yl, 1-ethylpyrrolidin-2-yl, 1-propylpyrrolidin-2-yl, 1-isopropylpyrrolidin-2-yl, 1-(2-methoxyethyl)pyrrolidin-2-yl, 1-(2-methoxypropyl)pyrrolidin-2-yl, 1-(2-ethoxyethyl)pyrrolidin-2-yl, 1-(2-ethoxypropyl)pyrrolidin-2-yl, 1-(2-hydroxymethyl)pyrrolidin-2-yl, 1-(2-hydroxyethyl)pyrrolidin-2-yl, 1-(2-hydroxypropyl)pyrrolidin-2-yl, piperidin-1-yl, 1-methylpiperazin-4-yl, 1-ethylpiperazin-4-yl, 1-propylpiperazin-4-yl, 1-isopropylpiperazin-4-yl, 1-(2-hydroxyethyl)piperazin-4-yl, morpholinyl, tetrahydrofuran-2-yl, tetrahydrofuran-3-yl, tetrahydropyran-2-yl, tetrahydropyran-3-yl, or thiomorpholinyl.

Still alternatively, R₁ is 1-methylpyrrolidin-2-yl, 1-ethylpyrrolidin-2-yl, 1-propylpyrrolidin-2-yl, 1-isopropylpyrrolidin-2-yl, 1-(2-methoxyethyl)pyrrolidin-2-yl, 1-(2-methoxypropyl)pyrrolidin-2-yl, 1-(2-ethoxyethyl)pyrrolidin-2-yl, 1-(2-ethoxypropyl)pyrrolidin-2-yl, 1-(2-hydroxymethyl)pyrrolidin-2-yl, 1-(2-hydroxyethyl)pyrrolidin-2-yl, or 1-(2-hydroxypropyl)pyrrolidin-2-yl.

According to another alternative embodiment, L is -O- or -NH-;
R₂ is C₁-C₄ alkyl, C₁-C₄ haloalkyl, hydroxy-substituted C₁-C₄ alkyl, C₁-C₃ alkoxy-substituted C₁-C₄ alkyl or -(CH₂)m-R₅,
R₅ is C₃-C₆ cycloalkyl or 4- to 6-membered heteroalicyclic group, and m is 0, 1 or 2,
the 4- to 6-membered heteroalicyclic group is a heteroalicyclic group containing 1-2 heteroatoms selected from N, O and S.

Still alternatively, L is -O-;
R₂ is methyl, ethyl, propyl, isopropyl, butyl, isobutyl, tert-butyl, fluoromethyl, 2-fluoroethyl, 3-fluoropropyl, trifluoromethyl, difluoromethyl, 3,3,3-trifluoropropyl, 3,3,3-trifluoroethyl, 2,2-difluoroethyl, 3,3-difluoropropyl, hydroxymethyl, hydroxyethyl, hydroxypropyl, hydroxybutyl, 2-hydroxy-2-methylpropyl, 3-hydroxy-3-methylbutyl, methoxymethyl, methoxyethyl, methoxypropyl, methoxybutyl, ethoxymethyl, ethoxyethyl, ethoxypropyl, ethoxybutyl, cyclopropyl, cyclopropylmethyl, cyclobutyl, tetrahydrofuran-2-yl, tetrahydrofuran-3 -yl.

In another alternative embodiment, T₂ is -O-(CH₂)p-, wherein p is 0 or 1;
R₃ is phenyl, pyridyl, or pyrimidyl, and the phenyl, pyridyl, and pyrimidyl are unsubstituted or substituted with 1 to 2 substituents selected from fluorine, chlorine, methyl, methoxy, ethyl, ethoxy, propyl, propoxy, isopropyl, isopropoxy, cyano, hydroxy, fluoromethyl, 2-fluoroethyl, trifluoromethyl, difluoromethyl, 3,3,3-trifluoroethyl, and 2,2-difluoroethyl.

Still alternatively, T₂ is -O-(CH₂)p-, wherein p is 1;
R₃ is phenyl, 2-fluorophenyl, 3-fluorophenyl, 4-fluorophenyl, 2-methylphenyl, 3-methylphenyl, 4-methylphenyl, 2-methoxyphenyl, 3-methoxyphenyl, 4-methoxyphenyl, pyridin-2-yl, pyridin-3-yl, pyridin-4-yl, 6-methylpyridin-2-yl, 6-methoxypyridin-2-yl, 5-methylpyridin-2-yl, 5-methoxypyridin-2-yl, 4-methylpyridin-2-yl, 4-methoxypyridin-2-yl, 3-methylpyridin-2-yl, 3-methoxypyridin-2-yl, 6-fluoropyridin-2-yl, or 6-chloropyridin-2-yl.

According to an alternative embodiment, R₄ is hydrogen, fluorine, chlorine, methyl, methoxy, still alternatively, hydrogen, fluorine, chlorine.

According to an alternative embodiment, the application provides a compound represented by the following formula, a stereoisomer thereof, or a pharmaceutically acceptable salt thereof,

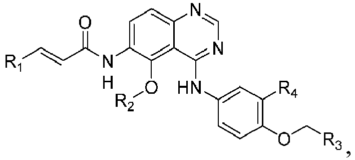

wherein R₁ is pyrrolidinyl, piperidinyl, piperazinyl, morpholinyl, tetrahydrofuranyl, tetrahydropyranyl, or thiomorpholinyl, and the above groups are unsubstituted or substituted with one or two of methyl, ethyl, propyl, isopropyl, aldehyde group, acetyl, propionyl, hydroxy, cyano, aminoacyl, methoxymethyl, methoxyethyl, methoxypropyl, ethoxymethyl, ethoxyethyl, ethoxypropyl, propoxymethyl, propoxyethyl, propoxypropyl, isopropoxymethyl, isopropoxyethyl, isopropoxypropyl, hydroxymethyl, hydroxyethyl and hydroxypropyl;
R₂ is C₁-C₄ alkyl, C₁-C₄ haloalkyl, hydroxy-substituted C₁-C₄ alkyl, C₁-C₃ alkoxy-substituted C₁-C₄ alkyl or -(CH₂)m-R₅,
R₅ is C₃-C₆ cycloalkyl or 4- to 6-membered heteroalicyclic group, and m is 0, 1 or 2,
the 4- to 6-membered heteroalicyclic group is a heteroalicyclic group containing 1-2 heteroatoms selected from N, O and S;
R₃ is phenyl, pyridyl, or pyrimidyl, and the phenyl, pyridyl, and pyrimidyl are unsubstituted or substituted with 1 to 2 substituents selected from fluorine, chlorine, methyl, methoxy, ethyl, ethoxy, propyl, propoxy, isopropyl, isopropoxy, cyano, hydroxy, fluoromethyl, 2-fluoroethyl, trifluoromethyl, difluoromethyl, 3,3,3-trifluoroethyl, and 2,2-difluoroethyl;
R₄ is hydrogen, fluorine, chlorine, methyl, methoxy.
Still alternatively, R₁ is pyrrolidin-1-yl, pyrrolidin-2-yl, 1-methylpyrrolidin-2-yl, 1-ethylpyrrolidin-2-yl, 1-propylpyrrolidin-2-yl, 1-isopropylpyrrolidin-2-yl, 1-(2-methoxyethyl)pyrrolidin-2-yl, 1-(2-methoxypropyl)pyrrolidin-2-yl, 1-(2-ethoxyethyl)pyrrolidin-2-yl, 1-(2-ethoxypropyl)pyrrolidin-2-yl, 1-(2-hydroxymethyl)pyrrolidin-2-yl, 1-(2-hydroxyethyl)pyrrolidin-2-yl, or 1-(2-hydroxypropyl)pyrrolidin-2-yl;
R₂ is methyl, ethyl, propyl, isopropyl, butyl, isobutyl, tert-butyl, fluoromethyl, 2-fluoroethyl, 3-fluoropropyl, trifluoromethyl, difluoromethyl, 3,3,3-trifluoropropyl, 3,3,3-trifluoroethyl, 2,2-difluoroethyl, 3,3-difluoropropyl, hydroxymethyl, hydroxyethyl, hydroxypropyl, hydroxybutyl, 2-hydroxy-2-methylpropyl, 3-hydroxy-3-methylbutyl, methoxymethyl, methoxyethyl, methoxypropyl, methoxybutyl, ethoxymethyl, ethoxyethyl, ethoxypropyl, or ethoxybutyl;
R₃ is phenyl, 2-fluorophenyl, 3-fluorophenyl, 4-fluorophenyl, 2-methylphenyl, 3-methylphenyl, 4-methylphenyl, 2-methoxyphenyl, 3-methoxyphenyl, 4-methoxyphenyl, pyridin-2-yl, pyridin-3-yl, pyridin-4-yl, 6-methylpyridin-2-yl, 6-methoxypyridin-2-yl, 5-methylpyridin-2-yl, 5-methoxypyridin-2-yl, 4-methylpyridin-2-yl, 4-methoxypyridin-2-yl, 3-methylpyridin-2-yl, 3-methoxypyridin-2-yl, 6-fluoropyridin-2-yl, or 6-chloropyridin-2-yl;
R₄ is fluorine or chlorine.

Still alternatively, R₁ is pyrrolidin-1-yl, pyrrolidin-2-yl, 1-methylpyrrolidin-2-yl, 1-ethylpyrrolidin-2-yl, 1-propylpyrrolidin-2-yl, 1-isopropylpyrrolidin-2-yl, 1-(2-methoxyethyl)pyrrolidin-2-yl, 1-(2-methoxypropyl)pyrrolidin-2-yl, 1-(2-ethoxyethyl)pyrrolidin-2-yl, 1-(2-ethoxypropyl)pyrrolidin-2-yl, 1-(2-hydroxymethyl)pyrrolidin-2-yl, 1-(2-hydroxyethyl)pyrrolidin-2-yl, or 1-(2-hydroxypropyl)pyrrolidin-2-yl;
R₂ is methyl, ethyl, propyl, isopropyl, butyl, isobutyl, tert-butyl, fluoromethyl, 2-fluoroethyl, 3-fluoropropyl, trifluoromethyl, difluoromethyl, 3,3,3-trifluoropropyl, 3,3,3-trifluoroethyl, 2,2-difluoroethyl, 3,3-difluoropropyl, hydroxymethyl, hydroxyethyl, hydroxypropyl, hydroxybutyl, 2-hydroxy-2-methylpropyl, 3-hydroxy-3-methylbutyl, methoxymethyl, methoxyethyl, methoxypropyl, methoxybutyl, ethoxymethyl, ethoxyethyl, ethoxypropyl, or ethoxybutyl;
R₃ is pyridin-2-yl;
R₄ is chlorine.

Still alternatively, R₁ is 1-methylpyrrolidin-2-yl, 1-ethylpyrrolidin-2-yl, 1-isopropylpyrrolidin-2-yl;
R₂ is methyl, ethyl, propyl, methoxyethyl, methoxypropyl, 2,2-difluoroethyl;
R₃ is pyridin-2-yl;
R₄ is chlorine.

Exemplary compounds involved in this application are listed below:

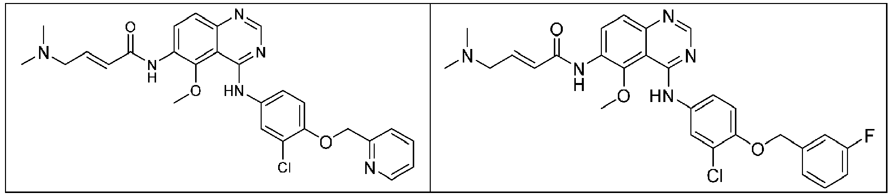

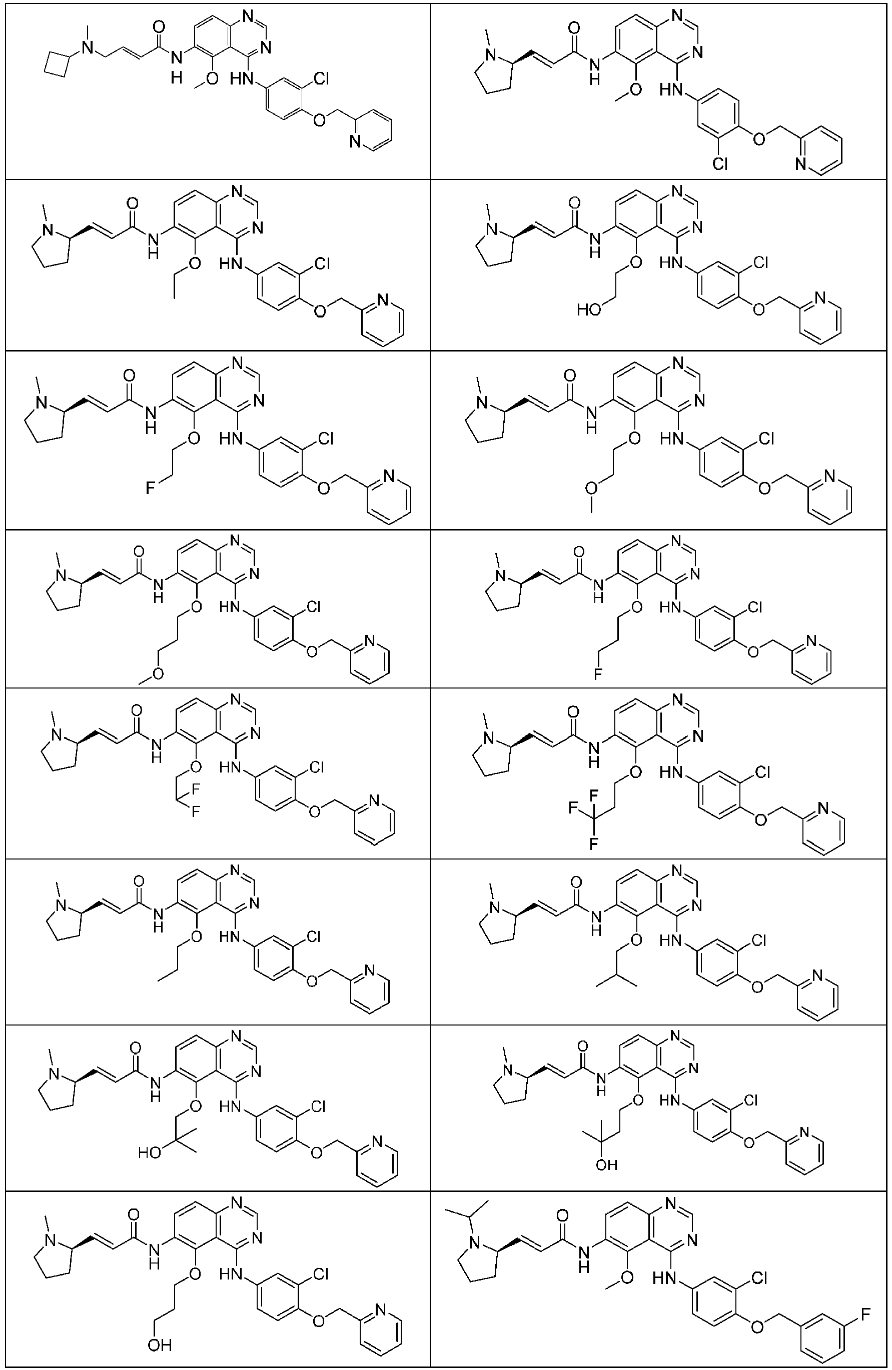

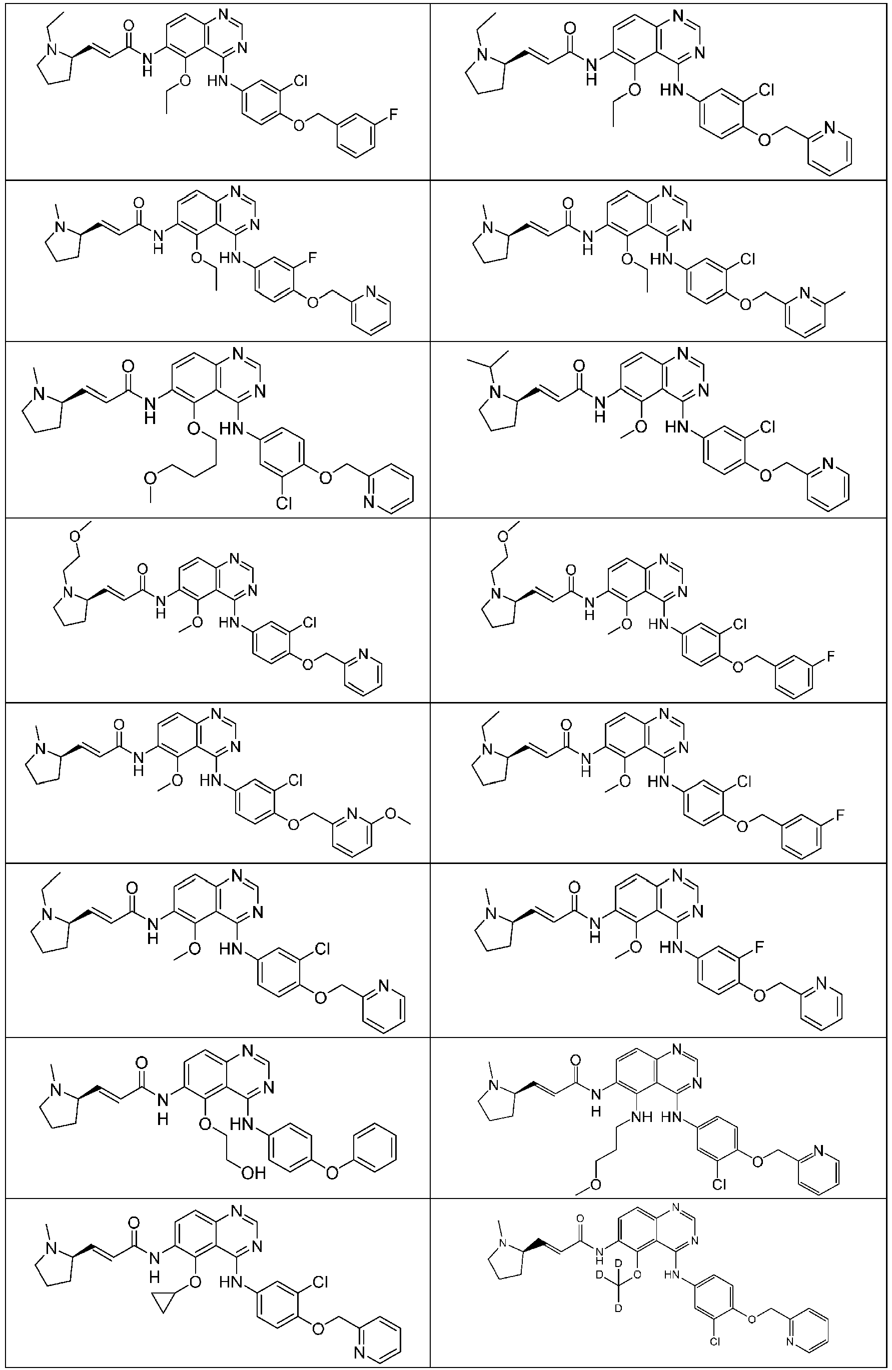

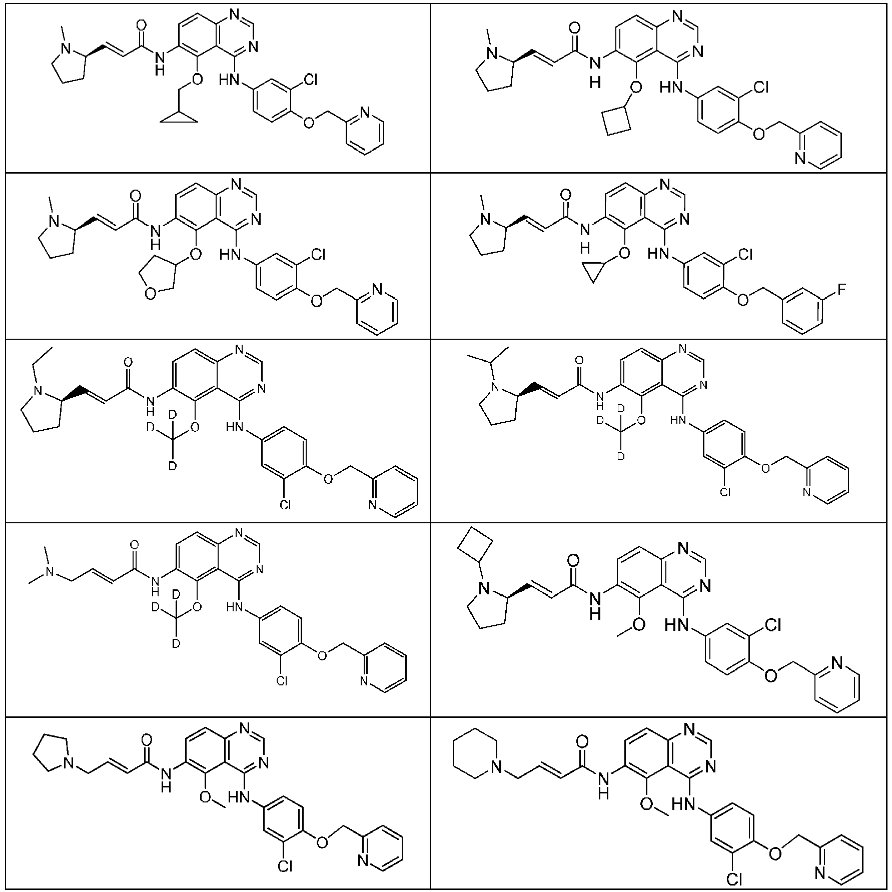

Another aspect of the present application provides a pharmaceutical composition, which comprises the compound described in the present application, a stereoisomer thereof, a pharmaceutically acceptable salt thereof, or a deuterated derivative thereof, and one or more pharmaceutically acceptable carriers or excipients.

The pharmaceutical composition of the present application may also contain one or more other therapeutic agents.

The present application also relates to use of the compound, a stereoisomer thereof, a pharmaceutically acceptable salt thereof, or a deuterated derivative thereof in the manufacture of a medicament for the treatment of a disease related to tyrosine kinase HER2, especially a disease related to exon 20 mutations of the tyrosine kinase HER2.

The disease is alternatively cancer or autoimmune disease, especially ocular fundus disease, xerophthalmia, psoriasis, leucoderma, dermatitis, alopecia areata, rheumatoid arthritis, colitis, multiple sclerosis, systemic lupus erythematosus, Crohn's disease, atherosclerosis, pulmonary fibrosis, liver fibrosis, myelofibrosis, non-small cell lung cancer, small cell lung cancer, breast cancer, pancreatic cancer, glioma, glioblastoma, ovarian cancer, cervical cancer, colorectal cancer, melanoma, endometrial cancer, prostate cancer, bladder cancer, leukemia, gastric cancer, liver cancer, gastrointestinal stromal tumor, thyroid cancer, chronic granulocytic leukemia, acute myelocytic leukemia, non-Hodgkin's lymphoma, nasopharyngeal cancer, esophageal cancer, brain tumor, B-cell and T-cell lymphoma, lymphoma, multiple myeloma, biliary carcinosarcoma, or cholangiocarcinoma.

The present disclosure also relates to a method for treating a disease or condition mediated by kinases such as HER2, which comprises administering to a patient in need thereof (a human being or other mammal, in particular a human being) a therapeutically effective amount of the compound described in the present application or a salt thereof, wherein the disease or condition mediated by kinases such as HER2 includes those mentioned above.

### BRIEF DESCRIPTION OF THE DRAWINGS

The foregoing and other purposes, features and other advantages of the present disclosure will be more clearly understood from the following detailed description in conjunction with the drawings, wherein
FIG. 1 illustrates the effect of NERATINIB on T/C values of tumor-bearing mice over time;
FIG. 2 illustrates the effect of PYROTINIB on T/C values of tumor-bearing mice over time;
FIG. 3 illustrates the effect of the compound of Example 4 of the present application on T/C values of tumor-bearing mice over time;
FIG. 4 illustrates the effect of the compound of Example 8 of the present application on T/C values of tumor-bearing mice over time;
FIG. 5 illustrates the effect of the compound of Example 9 of the present application on T/C values of tumor-bearing mice over time;
FIG. 6 illustrates the effect of the compound of Example 11 of the present application on T/C values of tumor-bearing mice over time;
FIG. 7 illustrates the effect of the compound of Example 24 of the present application on T/C values of tumor-bearing mice over time;
FIG. 8 illustrates the effect of the compound of Example 29 of the present application on T/C values of tumor-bearing mice over time;
FIG. 9 illustrates the effect of the compound of Example 33 of the present application on T/C values of tumor-bearing mice over time.

### DETAILED DESCRIPTION OF THE INVENTION

Unless otherwise stated, the following terms used in this application (including the specification and claims) have the definitions given below. In this application, the use of "or" or "and" means "and/or" unless stated otherwise. In addition, the use of the term "comprising" and other forms such as "including", "containing" and "having" is not limiting. The chapter headings used herein are for organizational purposes only and should not be interpreted as limitations on the topics described.

Unless otherwise specified, an alkyl group refers to a saturated linear and branched hydrocarbon group having a specified number of carbon atoms, and the term C₁-C₆ alkyl refers to an alkyl moiety containing from 1 to 6 carbon atoms. Similarly, C₁-C₃ alkyl refers to an alkyl moiety containing from 1 to 3 carbon atoms. For example, C₁-C₆ alkyl includes methyl, ethyl, propyl, isopropyl, n-butyl, isobutyl, sec-butyl, tert-butyl, n-pentyl, 3-(2-methyl)butyl, 2-pentyl, 2-methylbutyl, neopentyl, n-hexyl, 2-hexyl and 2-methylpentyl, etc.

When a substituent term such as "alkyl" is used in combination with other substituent term, such as in terms "C₁-C₃ alkoxy C₁-C₆ alkylthio" or "hydroxy-substituted C₁-C₆ alkyl", the linking substituent terms (e.g., alkyl or alkylthio) are intended to encompass a divalent moiety, wherein the point of attachment is through the linking substituent. Examples of "C₁-C₃ alkoxy C₁-C₆ alkylthio" include, but are not limited to, methoxymethylthio, methoxyethylthio and ethoxypropylthio, etc. Examples of "hydroxy-substituted C₁-C₆ alkyl" include, but are not limited to, hydroxymethyl, hydroxyethyl, and hydroxyisopropyl, etc.

An alkoxy group is an alkyl-O- group formed by a linear or branched alkyl group described previously and -O-, e.g., methoxy, ethoxy, and the like. Similarly, an alkylthio group is an alkyl-S- group formed by a linear or branched alkyl group as described previously and - S-, e.g., methylthio, ethylthio, and the like.

Alkenyl and alkynyl groups include linear or branched alkenyl or alkynyl groups, and the term C₂-C₆ alkenyl or C₂-C₆ alkynyl refers to linear or branched hydrocarbon groups having at least one alkenyl or alkynyl group.

The term "haloalkyl", such as "C₁-C₆ haloalkyl", refers to a group having one or more halogen atoms, which may be the same or different, on one or more carbon atoms of an alkyl moiety comprising 1 to 6 carbon atoms. Examples of "C₁-C₆ haloalkyl" may include, but are not limited to, -CF₃ (trifluoromethyl), -CCl₃ (trichloromethyl), 1,1-difluoroethyl, 2,2,2-trifluoroethyl and hexafluoroisopropyl, etc. Similarly, the term "C₁-C₆ haloalkoxy" refers to a haloalkyl-O- group formed by the C₁-C₆ haloalkyl and -O-, which can be, for example, trifluoromethoxy, trichloromethoxy, etc.

The term "cycloalkyl" refers to a non-aromatic, saturated, cyclic hydrocarbon group containing a specified number of carbon atoms. For example, the term "(C₃-C₆)cycloalkyl" refers to a non-aromatic cyclic hydrocarbon ring having 3-6 ring carbon atoms. Exemplary "(C₃-C₆)cycloalkyl" includes cyclopropyl, cyclobutyl, cyclopentyl, and cyclohexyl.

The term "aryl" refers to a group or moiety comprising an aromatic monocyclic or bicyclic hydrocarbon radical, which contains from 6 to 12 carbon ring atoms and has at least one aromatic ring. Examples of "aryl" are phenyl, naphthyl, indenyl and dihydroindenyl (indanyl). Generally, in the compounds of the present disclosure, the aryl is phenyl.

Unless otherwise specified, the term "heteroalicyclic group" as used herein refers to an unsubstituted or substituted stable 4- to 7-membered non-aromatic monocyclic saturated ring system consisting of carbon atoms and 1 to 3 heteroatoms selected from N, O, and S, wherein the N or S heteroatom can be randomly oxidized, and the N heteroatom can also be randomly quaternized. Examples of such heterocycles include, but are not limited to, azetidinyl, oxetanyl, thietanyl, pyrrolidinyl, pyrrolinyl, pyrazolidinyl, pyrazolinyl, imidazolidinyl, imidazolinyl, oxazolinyl, thiazolinyl, tetrahydrofuranyl, dihydrofuranyl, tetrahydrothienyl, 1,3-dioxolanyl, piperidinyl, piperazinyl, tetrahydropyranyl, dihydropyranyl, tetrahydrothiopyranyl, 1,3-dioxanyl, 1,4-dioxanyl, 1,3-oxathiolanyl, 1,3-oxathianyl, 1,3-dithianyl, 1,4-oxathiolanyl, 1,4-oxathianyl, 1,4-dithianyl, morpholinyl, and thiomorpholinyl.

The term "heteroaryl" as used herein represents a group or moiety containing an aromatic monocyclic or bicyclic radical (containing 5 to 10 ring atoms), which includes 1 to 3 heteroatoms independently selected from nitrogen, oxygen and sulfur. The term also includes bicyclic heterocyclic aryl, which contains an aryl ring moiety fused to a heterocycloalkyl ring moiety, or a heteroaryl ring moiety fused to a cycloalkyl ring moiety. Unless otherwise specified, it represents an unsubstituted or substituted stable 5- or 6-membered monocyclic aromatic ring system, and can also represents an unsubstituted or substituted benzene fused heteroaromatic ring system or bicyclic heteroaromatic ring system with 9 or 10 ring atoms, which are composed of carbon atoms and 1 to 3 heteroatoms selected from N, O, and S, where N and S heteroatoms can be oxidized, and N heteroatoms can also be quaternized. Heteroaryl groups can be connected to any heteroatom or carbon atom to form a stable structure. Illustrative examples of heteroaryl groups include, but are not limited to, furanyl, thienyl, pyrrolyl, imidazolyl, pyrazolyl, triazolyl, tetrazolyl, thiazolyl, oxazolyl, isoxazolyl, oxadiazolyl, thiadiazolyl, isothiazolyl, pyridyl, oxo-pyridyl (pyridyl-N-oxide), pyridazinyl, pyrazinyl, pyrimidinyl, triazinyl, benzofuranyl, isobenzofuranyl, 2,3-dihydrobenzofuranyl, 1,3-benzodioxolyl, dihydrobenzodioxinyl, benzothienyl, indazinyl, indolyl, isoindolyl, indolinyl, benzimidazolyl, dihydrobenzimidazolyl, benzoxazolyl, dihydrobenzoxazolyl, benzothiazolyl, benzoisothiazolyl, dihydrobenzisothiazolyl, indazolyl, imidazopyridyl, pyrazolopyridyl, benzotriazolyl, triazolopyridyl, purinyl, quinolinyl, tetrahydroquinolinyl, isoquinolinyl, tetrahydroisoquinolinyl, quinoxalinyl, cinnolinyl, phthalazinyl, quinazolinyl, 1,5-naphthyridinyl, 1,6-naphthyridinyl, 1,7-naphthyridinyl, 1,8-naphthyridinyl, and pteridyl.

The term "carbonyl" refers to a -C(O)- group. The terms "halogen" and "halo" refer to chlorine, fluorine, bromine or iodine substituent. "Oxo" refers to an oxygen moiety with double bond; for example, "oxo" may be directly attached to a carbon atom to form a carbonyl moiety (C=O). "Hydroxy" is intended to refer to a radical -OH. The term "cyano" as used herein refers to a group -CN.

The term "deuterated derivative" refers to a derivative obtained by deuteration of optionally one or more hydrogen atoms in the compound of this application.

The term "each independently" means that where more than one substituents are selected from a number of possible substituents, those substituents may be the same or different.

It is clear that the compound of formula I, or stereisomer, crystalline form or prodrug thereof, and pharmaceutically acceptable salt thereof, may exist in a solvated or non-solvated form. For example, the solvated form can be a water-soluble form. The present disclosure includes all the solvated and non-solvated forms.

In this disclosure, the term "isomer" refers to different compounds having the same molecular formula, and may include various isomeric forms such as stereoisomers and tautomers. "Stereoisomers" are isomers that differ only in the arrangement of their atoms in space. Some compounds described herein contain one or more asymmetric centers and thus can give rise to enantiomers, diastereomers, and other stereoisomeric forms which can be defined as (R)- or (S)-based on absolute stereochemistry. The chemical entities, pharmaceutical compositions, and methods disclosed herein are intended to include all of these possible isomers, including racemic mixtures, optically pure forms, and intervenient mixtures between them. Optically active (R)- and (S)-isomers can be prepared using chiral synthons or chiral reagents or resolved using conventional techniques. The optical activity of a compound can be analyzed by any suitable method, including but not limited to chiral chromatography and polarimetry, and the degree of dominance of one stereoisomer over other isomers can be determined.

Individual stereoisomers (or isomers-enriched mixtures) of a compound of this disclosure may be resolved using methods known to those skilled in the art. For example, such resolution may be carried out by: (1) formation of diastereoisomeric salts, complexes or other derivatives; (2) selective reaction with a stereoisomer-specific reagent, for example by enzymatic oxidation or reduction; or (3) gas-liquid or liquid chromatography in a chiral environment, for example, on a chiral support such as silica bound with chiral ligands or in the presence of a chiral solvent. The skilled artisan will appreciate that where the desired stereoisomer is converted into another chemical entity by one of the separation procedures described above, a further step is required to liberate the desired form. Alternatively, specific stereoisomers may be synthesized by asymmetric synthesis using optically active reagents, substrates, catalysts or solvents, or by converting one enantiomer to the other by asymmetric transformation.

When a compound described herein contains an olefinic double bond, it means that the compound includes various cis- or trans-isomers, unless otherwise stated.

"Tautomers" are structurally different isomers that are interconvertible to each other through tautomerization. "Tautomerization" is a form of isomerization and includes a prototropic change or proton transfer tautomerization, which can be considered as a subset of acid-base chemistry. "prototropic change tautomerization" or "proton transfer tautomerization" involves the migration of a proton accompanied by a bond-level transformation, which is often exchange of a single bond with an adjacent double bond. When tautomerization is possible (for example, in solution), tautomers can reach chemical equilibrium. An example of tautomerization is keto-enol tautomerization.

The compound of the present disclosure as an active ingredient, and the method of preparing the same, are both included in the present disclosure. Moreover, the crystalline form of some of the compounds may exist as polymorphs, and such forms may also be included in the present disclosure. Additionally, some of the compounds may form solvates with water (i.e., hydrates) or common organic solvents, and such solvates are also included within the scope of the disclosure.

The compounds of the disclosure may be used in the free form for treatment or, when appropriate, in the form of a pharmaceutically acceptable salt or other derivative for treatment. As used herein, the term "pharmaceutically acceptable salt" refers to organic and inorganic salts of the compounds of the present disclosure which are suitable for use in human and lower animals without undue toxicity, irritation, allergic response, etc., and have reasonable benefit/risk ratio. Pharmaceutically acceptable salts of amines, carboxylic acids, phosphonates, and other types of compounds are well known in the art. The salt can be formed by reacting a compound of the disclosure with a suitable free base or acid, including, but not limited to, inorganic acids such as hydrochloric acid, hydrobromic acid, phosphoric acid, sulfuric acid, perchloric acid or organic acids such as acetic acid, oxalic acid, maleic acid, tartaric acid, citric acid, succinic acid, malonic acid. Or the salts may be obtained by methods well known in the art, such as ion exchange. Other pharmaceutically acceptable salts include adipate, alginate, ascorbate, aspartate, besylate, benzoate, bisulfate, borate, butyrate, camphorate, camphorsulfonate, citrate, digluconate, lauryl sulfate, ethanesulfonate, formate, fumarate, glucoheptonate, glycerol phosphate, glyconate, hemisulfate, hexanoate, hydroiodide, 2-hydroxyethanesulfonate, lactobionate, lactate, laurate, lauryl sulfate, malate, maleate, mesylate, 2-naphthalenesulfonate, nicotinate, nitrate, oleate, palmitate, pamoate, pectate, persulphate, per-3-phenylpropionate, phosphate, picrate, propionate, stearate, sulfate, thiocyanate, p-toluenesulfonate, undecanoate, and the like. Representative alkali or alkaline earth metal salts include salts of sodium, lithium, potassium, calcium, magnesium, and the like. Other pharmaceutically acceptable salts include suitable non-toxic salts of ammonium, quaternary ammonium, and amine cations formed from halides, hydroxides, carboxylates, sulfates, phosphates, nitrates, lower alkyl sulfonates and aryl sulfonates.

Further, the term "prodrug" as used herein means that a compound can be converted into the compound of the present disclosure in vivo. Such transformation is affected by hydrolysis of the prodrug in the blood or enzymatic conversion to the parent compound in the blood or tissue.

Pharmaceutical compositions of this disclosure comprise the compound described herein or a pharmaceutically acceptable salt thereof; an additional active agent selected from a kinase inhibitory agent (small molecule, polypeptide, antibody, etc.), an immunosuppressant, an anticancer agent, an anti-viral agent, anti-inflammatory agent, antifungal agent, antibiotic, and an anti-vascular hyper proliferation compound; and any pharmaceutically acceptable carrier, adjuvant or excipient.

The compounds of the present disclosure may be used alone or in combination with one or more of other compounds of the present disclosure or with one or more of other agents. When administered in combination, the therapeutic agents can be formulated for simultaneous or sequential administration at different times, or the therapeutic agents can be administered as a single composition. By "combination therapy", it refers to the use of a compound of the present disclosure in combination with another agent, in a manner whereby each agent is co-administered simultaneously or administered sequentially, in either case, for the purpose of achieving the optimal effect of drugs. Co-administration includes dosage form for simultaneous delivery, as well as separate dosage forms for each compound. Thus, administration of the compounds of the disclosure can be combined with other therapies known in the art, for example, radiation therapy or cytostatic agents, cytotoxic agents, other anticancer agents, and the like as used in the treatment of cancer, in order to improve the symptoms of cancer. The administration sequence is not limited in the present disclosure. The compounds of the present disclosure may be administered before, simultaneously, or after other anticancer or cytotoxic agents.

To prepare the pharmaceutical ingredient of the present disclosure, one or more compounds of Formula (I) or salts thereof as an active ingredient can be intimately mixed with a pharmaceutical carrier, which is carried out according to conventional pharmaceutical formulation techniques. The carrier can be used in a wide variety of forms depending on the form of preparation which is designed for different modes of administration (for example, oral or parenteral administration). Suitable pharmaceutically acceptable carriers are well known in the art. A description of some of these pharmaceutically acceptable carriers can be found in the Handbook of Pharmaceutical Excipients, published jointly by the American Pharmaceutical Association and the Pharmaceutical Society of Great Britain.

The pharmaceutical composition of the present disclosure may have the following forms, for example, those suitable for oral administration, such as tablets, capsules, pills, powders, sustained release forms, solutions or suspensions; those for parenteral injections such as clear solutions, suspensions, emulsion; or those for topical application such as ointments, creams; or as a suppository for rectal administration. The pharmaceutical ingredients may also be presented in unit dosage form for single administration in a precise dosage. The pharmaceutical ingredient will include a conventional pharmaceutical carrier or excipient and a compound as an active ingredient prepared according to the present disclosure, and may also include other medical or pharmaceutical preparations, carriers, adjuvants, and the like.

Therapeutic compounds can also be administered to mammals other than humans. The drug dosage for a mammal will depend on the species of the animal and its disease condition or its disordered condition. The therapeutic compound can be administered to the animal in the form of a capsule, a bolus, a tablet or liquid. The therapeutic compound can also be introduced into the animal by injection or infusion. These drug forms are prepared in a traditional manner complying with standard veterinary practice. As an alternative, the pharmaceutical synthetic drugs can be fed to the animal in a mixture with the animal feed, so that the concentrated feed additive or premix can be prepared by mixing ordinary animal feed.

It is a further object of the present disclosure to provide a method for treating cancer in a subject in need thereof, comprising administering to the subject a therapeutically effective amount of a composition containing the compound of the present disclosure.

The present disclosure also includes use of the compound of the present disclosure or a pharmaceutically acceptable derivative thereof in the manufacture of a medicament for the treatment of diseases related to tyrosine kinases EGFR and HER2, wherein the diseases may be cancer (including non-solid tumors, solid tumors, primary or metastatic cancer, as noted elsewhere herein, and including cancer resistant or refractory to one or more other therapies), especially may be lung cancer or breast cancer.

### EXAMPLES

The present disclosure also provides methods for preparing the corresponding compounds. Various synthetic methods can be used to prepare the compounds described herein, including the following methods. The compounds of the present disclosure, or pharmaceutically acceptable salts, isomers or hydrates thereof can be synthesized using the methods described below, synthetic methods known in the art of organic chemistry synthesis, or variants of these methods understood by those skilled in the art. Alternative methods include, but are not limited to, the methods described below.

In order to make the objectives, technical solutions and advantages of the present disclosure clearer, the present disclosure will be further described in detail below in conjunction with specific examples. It should be understood that the specific examples described here are only used to explain the present disclosure and are not intended to limit the present invention. If no specific technology or conditions are indicated in examples, the technology or conditions described in the literature in the art or the product specification shall be followed. If manufacturers are not indicated for reagents or instruments used, the reagents or instruments are all conventional products that are commercially available. The term "and/or" as used herein includes any and all combinations of one or more related listed items. Examples are provided below to better illustrate the invention, and all temperatures refer to °C unless otherwise noted. The names of some compounds in this disclosure are generated by Chemdraw and translated.

### Synthesis of intermediates

### Intermediate 1.

### (R,E)-3-(1-(2-methoxyethyl)pyrrolidin-2-yl)acrylic acid

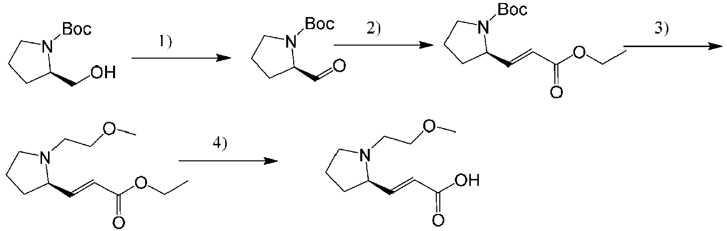

### Step 1): synthesis of tert-butyl (R)-2-formylpyrrolidine-1-carboxylate;

Tert-butyl (R)-2-(hydroxymethyl)pyrrolidine-1-carboxylate (4 g, 20 mmol) and Dess-Martin reagent (8.5 g, 20 mmol) were added to dichloromethane (70 ml) in an ice-water bath and the mixture was stirred at room temperature for 2 h. The mixture was filtered, washed, dried and concentrated to give 4 g of oil, which was used directly in the next step.

### Step 2): synthesis of Tert-butyl (R,E)-2-(3-ethoxy-3-oxoprop-1-en-1-yl)pyrrolidine-1-carboxylate;

Triethyl phosphonoacetate (3.6 mL, 18.1 mmol) was added dropwise to a solution of NaH (782 mg, 19.6 mmol) in THF (15 mL) at 25°C. After 0.5 h, the solution was cooled to 0 °C and a solution of Boc-L-proline (3 g, 15.1 mmol) in THF (60 mL) was added dropwise. The mixture was stirred at 0 °C for another 2 h, and then quenched by adding water. The aqueous phase was back-extracted several times with DCM, and the combined organic phase was dried over anhydrous sodium sulfate and concentrated. The resulting yellow oil (4 g, quantitative) was used directly without further purification. LCMS: 270[M + H]⁺.

### Step 3): synthesis of Ethyl (R,E)-3-(1-(2-methoxyethyl)pyrrolidin-2-yl)acrylate;

Tert-butyl (R,E)-2-(3-ethoxy-3-oxoprop-1-en-1-yl)pyrrolidine-1-carboxylate (0.27 g, 1 mmol) was added to a solution of trifluoroacetic acid (1 ml) in dichloromethane (4 ml), and the mixture was stirred to react at room temperature for 2 h. The mixture was concentrated to give a yellow oil. The yellow oil was added to a solution of bromoethyl methyl ether (0.3 g, 2 mmol) and potassium carbonate (0.45 g, 3 mmol) in acetonitrile (10 ml), and the mixture was heated to 80 °C to react for 12 hours. The mixture was cooled, extracted with ethyl acetate, and washed three times with water. The organic phase was dried and concentrated to give 0.12 g of a light yellow oil; LC-MS:228[M+H]⁺.

### Step 4): synthesis of (R,E)-3-(1-(2-methoxyethyl)pyrrolidin-2-yl)acrylic acid;

Ethyl (R,E)-3-(1-(2-methoxyethyl)pyrrolidin-2-yl)acrylate (0.12 g, 0.5 mmol) and sodium hydroxide (0.04 g, 1 mmol) were added to a solution of methanol (1 ml) and water (1 ml), and the reaction was stirred at room temperature overnight. After the reaction was completed, the mixture was extracted with ethyl acetate. The aqueous phase was adjusted to pH 4-5 with dilute hydrochloric acid, concentrated, washed with a mixture of dichloromethane and methanol, and filtered. The filtrate was concentrated, and then slurried with acetonitrile to give the product as a white solid; LC-MS: 200[M+H]⁺.

### Intermediate 2. (R,E)-3-(1-cyclobutylpyrrolidin-2-yl)acrylic acid

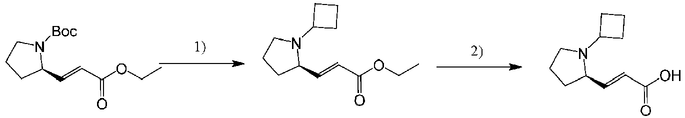

### Step 1): synthesis of Ethyl (R,E)-3-(1-cyclobutylpyrrolidin-2-yl)acrylate

Tert-butyl (R,E)-2-(3-ethoxy-3-oxoprop-1-en-1-yl)pyrrolidine-1-carboxylate (0.27 g, 1 mmol) was added to a solution of trifluoroacetic acid (1 ml) in dichloromethane (3 ml). The mixture was stirred to react at room temperature for two hours, and concentrated to give a yellow oil. The yellow oil, cyclobutyl-1-one (0.1 g, 1.5 mmol) was added to 1,2-dichloroethane, and the mixture was stirred for half an hour. Then sodium cyanoborohydride (0.13 g, 2 mmol) was added to react. After the completion of the reaction, the pH was adjusted to 8-9, and the mixture was extracted with dichloromethane. The organic phase was washed with water, dried, concentrated and purified by column chromatography to give the target product.

### Step 2): synthesis of (R,E)-3-(1-cyclobutylpyrrolidin-2-yl)acrylic acid

Ethyl (R,E)-3-(1-cyclobutylpyrrolidin-2-yl)acrylate (0.12 g, 0.5 mmol) and sodium hydroxide (0.04 g, 1 mmol) were added to a solution of methanol (1 ml) and water (1 ml), and the mixture was stirred to react at room temperature overnight. After the reaction was completed, the mixture was extracted with ethyl acetate. The aqueous phase was adjusted to pH 4-5 with dilute hydrochloric acid, concentrated, washed with a mixture of dichloromethane and methanol, and filtered. The filtrate was concentrated, and then slurrried with acetonitrile to give the product as a white solid; LC-MS:196[M+H]⁺.

### Synthesis of acryloyl chloride intermediate

The acryloyl chloride intermediates were synthesized by reacting the corresponding acrylic acid compounds in the presence of oxalyl chloride. The synthesis of (R,E)-3-(1-methylpyrrolidin-2-yl)acryloyl chloride was illustrated below as an example, and the rest of the acryloyl chloride intermediates were synthesized by the same method.

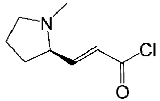

(R,E)-3-(1-methylpyrrolidin-2-yl)acrylic acid (150 mg, 1 mmol), oxalyl chloride (1.05 mmol) and DMF (1 drop) were respectively added to anhydrous dichloromethane (2 ml) under the condition of an ice-water bath, and the mixture was stirred to react at room temperature for 2 h. The mixture was concentrated to give a solid, which can be used directly in the next step of the reaction.

The following acyl chloride intermediates were synthesized from the corresponding acids:

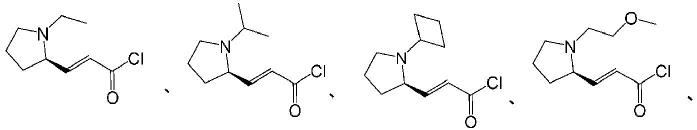

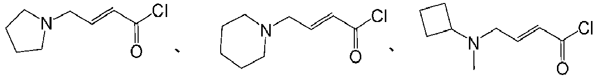

### Example 1. (E)-N-(4-((3-chloro-4-(pyridin-2-ylmethoxy)phenyl)amino)-5-methoxy quinazolin-6-yl)-4-(dimethylamino)but-2-enamide

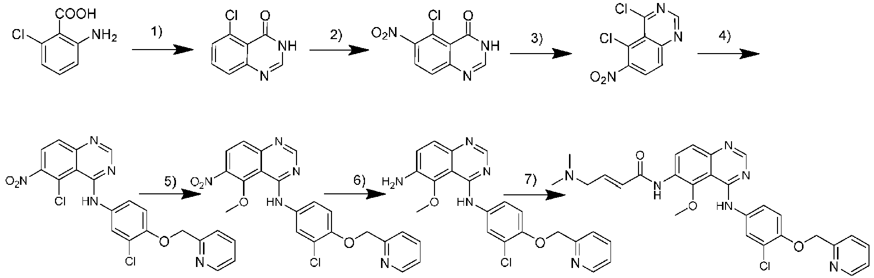

### Step 1): synthesis of 5-chloroquinazolin-4(3H)-one

2-Amino-6-chlorobenzoic acid (17.2g, 100mmol) and formamidine acetate (15.6g, 150mmol) were respectively added to a solution of ethanol (150mL), and the mixture was heated to reflux and stirred to react for 24 hours. The mixture was cooled and filtered to give 14 g of a white solid, 78% yield; MS:181[M+H]⁺.

### Step 2): synthesis of 5-chloro-6-nitroquinazolin-4(3H)-one

5-chloroquinazolin-4(3H)-one (7.2 g, 40 mmol) was dissolved in concentrated sulfuric acid (40mL), and potassium nitrate (4.2 g, 41.6 mmol) powder was added in batches at 0 °C. The mixture was stirred to react at room temperature for 24 hours. The product was added into crushed ice in batches, and the mixture was stirred for 1 hour. The mixture was filtered to give a light-yellow solid. The resultant solid was added to ethanol. The mixture was refluxed, then cooled and filtered to give 8.6 g of the product as a white solid, 95% yield.

### Step 3): synthesis of 4,5-dichloro-6-nitroquinazoline

5-Chloro-6-nitroquinazolin-4(3H)-one (4.5 g, 20 mmol) was added to dichlorosulfoxide (45 mL), and DMF (2 mL) was added. The mixture was heated to 80 °C to react at reflux. Upon the product was completely dissolved, the mixture was reacted for another 2 hours, and then concentrated. Toluene was added, and the mixture was concentrated again to give 4.9 g of the product as a white solid.

### Step 4): synthesis of 5-chloro-N-(3-chloro-4-(pyridin-2-ylmethoxy)phenyl)-6-nitro quinazolin-4-amine

4,5-Dichloro-6-nitroquinazoline (4.9 g, 20 mmol) was added into anhydrous acetonitrile, and 3-chloro-4-(pyridin-2-ylmethoxy)aniline (7 g, 30 mmol) and triethylamine (3 g, 30 mmol) were respectively added at 0 °C. The mixture was heated to 50 °C to react for 5 hours. The mixture was cooled, concentrated, and washed with methanol to give 6.5 g of the product as a white solid, 74% yield; MS:442[M+H]⁺.

### Step 5): synthesis of N-(3-chloro-4-(pyridin-2-ylmethoxy)phenyl)-5-methoxy-6-nit roquinazolin-4-amine

5-Chloro-N-(3-chloro-4-(pyridin-2-ylmethoxy)phenyl)-6-nitroquinazolin-4-amine (4.4 g, 10 mmol) was added to a mixed solution of DMF (15 mL) and sodium methoxide solution (30% sodium methoxide in methanol, 15 mL) at 0 °C. The mixture was stirred to react for 2 hours and quenched by adding ice. The mixture was filtered and dried to give 4.1 g of the product as a yellow solid, 94% yield; MS:438[M+H]⁺.

### Step 6): synthesis of N⁴-(3-chloro-4-(pyridin-2-ylmethoxy)phenyl)-5-methoxyquinazolin-4,6-diamine

N-(3-chloro-4-(pyridin-2-ylmethoxy)phenyl)-5-methoxy-6-nitroquinazolin-4-amine (2.2 g, 5 mmol) was added to ethanol, and iron powder and aqueous ammonium chloride solution were added. The mixture was heated to 50 °C to react for 2 hours, then cooled, filtered, and rinsed with a large amount of dichloromethane. The filtrate was washed with brine, dried and concentrated to give 2g of the product as a light-purple solid, 98% yield; MS:408[M+H]⁺.

### Step 7): synthesis of (E)-N-(4-((3-chloro-4-(pyridin-2-ylmethoxy)phenyl)amino)-5-methoxyquinazolin-6-yl)-4-(dimethylamino)but-2-enamide

N⁴-(3-chloro-4-(pyridin-2-ylmethoxy)phenyl)-5-methoxyquinazolin-4,6-diamine (40 mg, 0.1 mmol) was added to NMP solution (1 mL), and a solution of (E)-4-(dimethylamino)but-2-enoyl chloride (24 mg, 0.15 mmol) in dichloromethane (1 mL) was added at 0 °C. The mixture was stirred to react for half an hour, and quenched by adding water. The mixture was adjusted to pH of 9 with sodium bicarbonate, and then extracted with dichloromethane. The organic phase was washed with saturated saline, dried and concentrated to give an oil. The oil was purified by column chromatography to give 18 mg of the product as a white solid; ¹H NMR (400 MHz, DMSO-*d*₆) δ 9.94 (s, 1H), 9.86 (s, 1H), 8.60 (d, *J=* 4.8 Hz, 1H), 8.51 (s, 1H), 8.29 (d, *J* = 9.0 Hz, 1H), 8.11 (d, *J* = 2.6 Hz, 1H), 7.88 (t, *J* = 7.7 Hz, 1H), 7.65 - 7.53 (m, 3H), 7.37 (d, *J* = 7.6 Hz, 1H), 7.27 (d, *J* = 9.0 Hz, 1H), 6.81 (d, *J* = 15.4, 5.9 Hz, 1H), 6.58 (d, *J* = 15.5 Hz, 1H), 5.32 (s, 2H), 3.93 (s, 3H), 3.09 (d, *J* = 5.9 Hz, 2H), 2.20 (s, 6H). MS:519[M+H]⁺.

### Example 2. (E)-N-(4-((3-chloro-4-((3-fluorobenzyloxy)phenyl)amino)-5-methoxyquinazolin-6-yl)-4-(dimethylamino)but-2-enamide

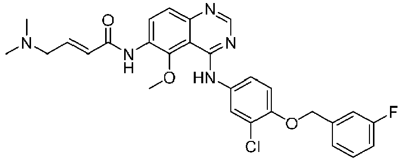

The compound was synthesized using the same method as in Example 1, except that 3-chloro-4-((3-fluorobenzyl)oxy)aniline was used for the reaction instead of 3-chloro-4-(pyridin-2-ylmethoxy)aniline in Step 4) of Example 1; ¹H NMR (400 MHz, DMSO-*d*₆) δ 9.94 (s, 1H), 9.86 (s, 1H), 8.51 (s, 1H), 8.29 (d, *J=* 9.0 Hz, 1H), 8.09 (d, *J=* 2.6 Hz, 1H), 7.66 - 7.53 (m, 2H), 7.48 (td, *J=* 8.0, 6.0 Hz, 1H), 7.37 - 7.14 (m, 4H), 6.81 (dt, *J=* 15.4, 5.9 Hz, 1H), 6.58 (d, *J=* 15.5 Hz, 1H), 5.28 (s, 2H), 3.92 (s, 3H), 3.11 (d, *J=* 6.1 Hz, 2H), 2.22 (s, 6H). MS:536[M+H]⁺.

### Example 3. (E)-N-(4-((3-chloro-4-(pyridin-2-ylmethoxy)phenyl)amino)-5-methoxyquinazolin-6-yl)-4-(cyclobutyl(methyl)amino)but-2-enamide

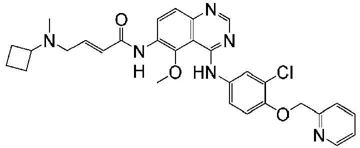

The compound was synthesized using the same method as in Example 1, except that (E)-4-(cyclobutyl(methyl)amino)but-2-enoyl chloride was used for the reaction instead of (E)-4-(dimethylamino)but-2-enoyl chloride in Step 7) of Example 1; ¹H NMR (400 MHz, DMSO-*d*₆) δ 9.93 (s, 1H), 9.86 (s, 1H), 8.63 - 8.57 (m, 1H), 8.51 (s, 1H), 8.28 (d, *J* = 9.1 Hz, 1H), 8.11 (d, *J=* 2.6 Hz, 1H), 7.88 (t, *J=* 7.7 Hz, 1H), 7.65 - 7.52 (m, 3H), 7.41 - 7.33 (m, 1H), 7.26 (d, *J* = 9.0 Hz, 1H), 6.82 (d, *J* = 15.4 Hz, 1H), 6.57 (d, *J=* 15.4 Hz, 1H), 5.31 (s, 2H), 3.93 (s, 3H), 3.05 (d, *J=* 6.1 Hz, 2H), 2.87 (p, *J=* 8.0 Hz, 1H), 2.08 - 1.92 (m, 5H), 1.88 - 1.73 (m, 2H), 1.60 (dd, *J=* 20.5, 10.0Hz, 2H). MS:559[M+H]⁺.

### Example 4. (R,E)-N-(4-((3-chloro-4-(pyridin-2-ylmethoxy)phenyl)amino)-5-methoxyquinazolin-6-yl)-3-(1-methylpyrrolidin-2-yl)acrylamide

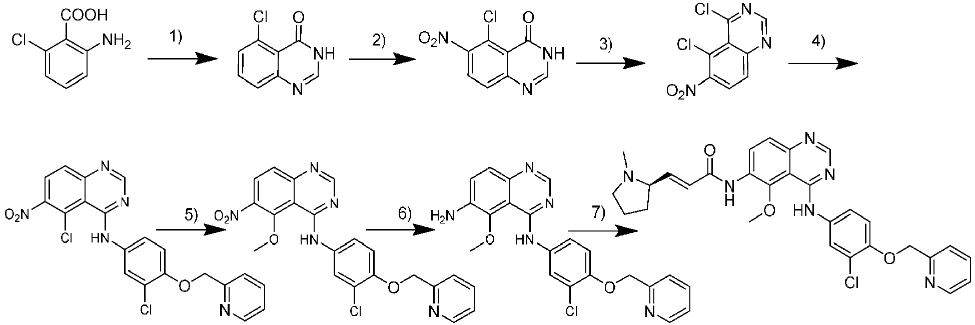

### Step 1) to Step 6): see Example 1;

Step 7): N⁴-(3-chloro-4-(pyridin-2-ylmethoxy)phenyl)-5-methoxyquinazolin-4,6-diamine (40 mg, 0.1 mmol) was added to NMP solution, and a solution of (R,E)-3-(1-methylpyrrolidin-2-yl)acryloyl chloride (26 mg) in dichloromethane (1 mL) was added at 0 °C. The mixture was stirred to react for half an hour, and then quenched by adding water. The mixture was adjusted to pH of 9 with sodium bicarbonate, and then extracted with dichloromethane. The organic phase was washed with saturated saline, dried and concentrated to give an oil. The oil was purified by column chromatography to give 24 mg of the product as a white solid; ¹H NMR (400 MHz, DMSO-*d*₆) δ 9.92 (s, 1H), 9.86 (s, 1H), 8.60 (d, *J=* 4.9 Hz, 1H), 8.51 (s, 1H), 8.30 (d, *J=* 9.1 Hz, 1H), 8.11 (d, *J=* 2.6 Hz, 1H), 7.88 (t, *J=* 7.7 Hz, 1H), 7.65 - 7.53 (m, 3H), 7.37 (d, *J=* 7.6 Hz, 1H), 7.27 (d, *J=* 9.0 Hz, 1H), 6.70 (dd, *J=* 15.3, 7.5 Hz, 1H), 6.56 (d, *J=* 15.3 Hz, 1H), 5.31 (s, 2H), 3.93 (s, 3H), 3.04 (d, *J=* 9.9 Hz, 1H), 2.75 (t, *J=* 7.8 Hz, 1H), 2.22 (s, 3H), 2.22 - 2.13 (m, 1H), 2.01 (d, *J=* 13.5 Hz, 1H), 1.79 - 1.68 (m, 2H), 1.58 (d, *J=* 12.3 Hz, 1H). MS:545[M+H]⁺.

### Example 5. (R,E)-N-(4-((3-chloro-4-(pyridin-2-ylmethoxy)phenyl)amino)-5-ethoxyquinazolin-6-yl)-3-(1-methylpyrrolidin-2-yl)acrylamide

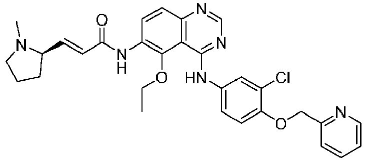

The compound was synthesized using the same method as in Example 4, except that sodium ethoxide was used for the reaction instead of sodium methoxide in step 5) of Example 4; ¹H NMR (400 MHz, DMSO-*d*₆) δ 9.99 (s, 1H), 9.91 (s, 1H), 8.60 (d, *J=* 4.8 Hz, 1H), 8.54 (s, 1H), 8.23 - 8.16 (m, 2H), 7.89 (t, *J=* 7.7 Hz, 1H), 7.62 - 7.51 (m, 3H), 7.37 (d, *J=* 7.6 Hz, 1H), 7.28 (d, *J=* 9.0 Hz, 1H), 6.69 (dd, *J* = 15.3, 7.7 Hz, 1H), 6.52 (d, *J=* 15.4 Hz, 1H), 5.31 (s, 2H), 4.13 (q, *J* = 7.0 Hz, 2H), 3.04 (d, *J* = 9.9 Hz, 1H), 2.77 (q, *J=* 7.9 Hz, 1H), 2.22 (s, 4H), 2.08 -1.95 (m, 1H), 1.74 (t, *J* = 8.5 Hz, 2H), 1.65 - 1.51 (m, 1H), 1.41 (t, *J* = 7.0 Hz, 3H). MS:559[M+H]⁺.

### Example 6. (R,E)-N-(4-((3-chloro-4-(pyridin-2-ylmethoxy)phenyl)amino)-5-(2-hydroxyethoxy)quinazolin-6-yl)-3-(1-methylpyrrolidin-2-yl)acrylamide

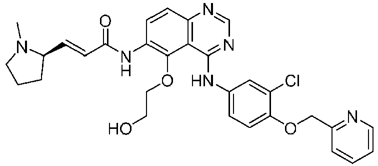

The compound was synthesized using the same method as in Example 4, except that sodium 2-hydroxyethoxide was used for the reaction instead of sodium methoxide in step 5) of Example 4; ¹H NMR (400 MHz, DMSO-*d*₆) δ 10.16 (s, 1H), 9.98 (s, 1H), 8.60 (d, *J=* 4.9 Hz, 1H), 8.50 (s, 1H), 8.33 (d, *J* = 9.1 Hz, 1H), 8.09 (d, *J* = 2.5 Hz, 1H), 7.89 (td, *J=* 7.7, 1.7 Hz, 1H), 7.67 (d, *J=* 9.0 Hz, 1H), 7.57 (t, *J=* 8.0 Hz, 2H), 7.37 (d, *J=* 7.5 Hz, 1H), 7.24 (d, *J=* 9.0 Hz, 1H), 6.69 (dd, *J=* 15.3, 7.7 Hz, 1H), 6.42 (d, *J=* 15.3 Hz, 1H), 5.78 - 5.59 (m, 1H), 5.30 (s, 2H), 4.08 (t, *J=* 4.1 Hz, 2H), 3.87 (q, *J=* 4.3 Hz, 2H), 3.03 (d, *J=* 9.7 Hz, 1H), 2.75 (q, *J=* 8.0 Hz, 1H), 2.20 (s, 3H), 2.17 (t, *J=* 8.8 Hz, 1H), 2.07 - 1.94 (m, 1H), 1.74 (t, *J=* 8.7 Hz, 2H), 1.58 (d, *J=* 17.2 Hz, 1H). MS:575[M+H]⁺.

### Example 7. (R,E)-N-(4-((3-chloro-4-(pyridin-2-ylmethoxy)phenyl)amino)-5-(2-fluoroethoxy)quinazolin-6-yl)-3-(1-methylpyrrolidin-2-yl)acrylamide

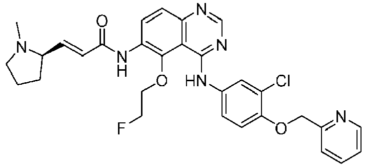

The compound was synthesized using the same method as in Example 4, except that sodium 2-fluoroethoxide was used for the reaction instead of sodium methoxide in step 5) of Example 4; ¹H NMR (400 MHz, DMSO-*d*₆) δ 9.91 (s, 1H), 9.84 (s, 1H), 8.60 (d, *J* = 4.7 Hz, 1H), 8.54 (s, 1H), 8.25 (d, *J=* 9.0 Hz, 1H), 8.08 (d, *J=* 2.6 Hz, 1H), 7.88 (t, *J=* 7.7 Hz, 1H), 7.58 (d, *J=* 8.6 Hz, 3H), 7.37 (d, *J=* 7.6 Hz, 1H), 7.27 (d, *J=* 9.1 Hz, 1H), 6.69 (dd, *J=* 15.3, 7.7 Hz, 1H), 6.48 (d, *J=* 15.3 Hz, 1H), 5.30 (s, 2H), 4.96 (d, *J=* 4.8 Hz, 1H), 4.88 - 4.81 (m, 1H), 4.40 - 4.33 (m, 1H), 4.32 - 4.25 (m, 1H), 3.04 (d, *J=* 9.7 Hz, 1H), 2.76 (q, *J* = 8.0 Hz, 1H), 2.21 (s, 3H), 2.18 (t, *J* = 8.8 Hz, 1H), 2.06 - 1.94 (m, 1H), 1.74 (d, *J* = 12.0 Hz, 2H), 1.65 - 1.53 (m, 1H). MS:577[M+H]⁺.

Example 8. (R,E)-N-(4-((3-chloro-4-(pyridin-2-ylmethoxy)phenyl)amino)-5-(2-methoxyethoxy)quinazolin-6-yl)-3-(1-methylpyrrolidin-2-yl)acrylamide

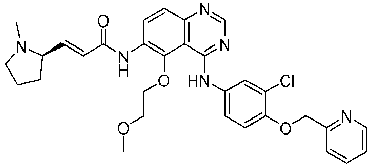

The compound was synthesized using the same method as in Example 4, except that sodium 2-methoxyethoxide was used for the reaction instead of sodium methoxide in step 5) of Example 4; ¹H NMR (400 MHz, DMSO-*d*₆) δ 10.03 (s, 1H), 9.87 (s, 1H), 8.60 (d, *J=* 4.8 Hz, 1H), 8.51 (s, 1H), 8.30 (d, *J* = 9.1 Hz, 1H), 8.00 (d, *J* = 2.6 Hz, 1H), 7.88 (td, *J* = 7.7 Hz, 1H), 7.68 (d, *J=* 9.0 Hz, 1H), 7.58 (d, *J=* 8.4 Hz, 2H), 7.37 (d, *J=* 7.6 Hz, 1H), 7.29 (d, *J=* 9.0 Hz, 1H), 6.70 (dd, *J* = 15.3, 7.6 Hz, 1H), 6.45 (d, *J=* 15.3 Hz, 1H), 5.30 (s, 2H), 4.20 - 4.13 (m, 2H), 3.83 - 3.76 (m, 2H), 3.30 (s, 3H), 3.04 (d, *J=* 9.9 Hz, 1H), 2.77 (q, *J=* 8.0 Hz, 1H), 2.22 (s, 4H), 2.08 - 1.95 (m, 1H), 1.79 - 1.69 (m, 2H), 1.65 - 1.51 (m, 1H). MS:589[M+H]⁺.

### Example 9. (R,E)-N-(4-((3-chloro-4-(pyridin-2-ylmethoxy)phenyl)amino)-5-(3-methoxypropoxy)quinazolin-6-yl)-3-(1-methylpyrrolidin-2-yl)acrylamide

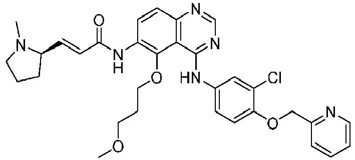

The compound was synthesized using the same method as in Example 4, except that sodium 3-methoxypropoxide was used for the reaction instead of sodium methoxide in step 5) of Example 4; ¹H NMR (400 MHz, DMSO-*d*₆) δ 9.83 (d, *J=* 17.8 Hz, 2H), 8.63 - 8.57 (m, 1H), 8.52 (s, 1H), 8.17 (d, *J* = 9.0 Hz, 1H), 8.09 (d, *J* = 2.6 Hz, 1H), 7.89 (t, *J* = 7.7 Hz, 1H), 7.62 - 7.52 (m, 3H), 7.37 (d, *J=* 7.4 Hz, 1H), 7.28 (d, *J=* 8.9 Hz, 1H), 6.69 (dd, *J=* 15.3, 7.7 Hz, 1H), 6.46 (d, *J=* 15.3 Hz, 1H), 5.31 (s, 2H), 4.07 (t, *J=* 6.2 Hz, 2H), 3.53 (t, *J=* 6.0 Hz, 2H), 3.16 (s, 3H), 3.04 (d, *J=* 9.7 Hz, 1H), 2.76 (q, *J=* 7.9 Hz, 1H), 2.21 (s, 3H), 2.20 - 2.13 (m, 1H), 2.08 (t, *J=* 6.2 Hz, 2H), 2.04 - 1.95 (m, 1H), 1.78 - 1.70 (m, 2H), 1.65 - 1.51 (m, 1H). MS:603[M+H]⁺.

### Example 10. (R,E)-N-(4-((3-chloro-4-(pyridin-2-ylmethoxy)phenyl)amino)-5-(3-fluoropropoxy)quinazolin-6-yl)-3-(1-methylpyrrolidin-2-yl)acrylamide

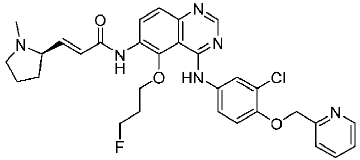

The compound was synthesized using the same method as in Example 4, except that sodium 3-fluoropropoxide was used for the reaction instead of sodium methoxide in step 5) of Example 4; ¹H NMR (400 MHz, DMSO-*d*₆) δ 9.86 (s, 1H), 9.80 (s, 1H), 8.60 (dt, *J=* 4.8 Hz, 1H), 8.53 (s, 1H), 8.16 - 8.09 (m, 2H), 7.89 (t, *J=* 7.7 Hz, 1H), 7.62 - 7.52 (m, 3H), 7.37 (d, *J* = 7.6 Hz, 1H), 7.27 (d, *J=* 9.0 Hz, 1H), 6.70 (dd, *J* = 15.3, 7.7 Hz, 1H), 6.47 (d, *J=* 15.3 Hz, 1H), 5.31 (s, 2H), 4.75 (t, *J* = 5.7 Hz, 1H), 4.63 (t, *J* = 5.7 Hz, 1H), 4.11 (t, *J* = 6.3 Hz, 2H), 3.07 (d, *J=* 10.1 Hz, 1H), 2.82 (d, *J=* 8.8 Hz, 1H), 2.27 - 2.18 (m, 6H), 2.10 - 1.95 (m, 1H), 1.75 (t, *J=* 9.3 Hz, 2H), 1.60 (d, *J=* 18.2 Hz, 1H). MS:591[M+H]⁺.

### Example 11. (R,E)-N-(4-((3-chloro-4-(pyridin-2-ylmethoxy)phenyl)amino)-5-(2,2-difluoroethoxy)quinazolin-6-yl)-3-(1-methylpyrrolidin-2-yl)acrylamide

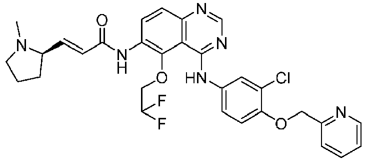

The compound was synthesized using the same method as in Example 4, except that sodium 2,2-difluoroethoxide was used for the reaction instead of sodium methoxide in step 5) of Example 4; ¹H NMR (400 MHz, DMSO-*d*₆) δ 9.95 (s, 1H), 9.64 (s, 1H), 8.64 - 8.52 (m, 2H), 8.16 - 8.04 (m, 2H), 7.89 (t, *J=* 7.7 Hz, 1H), 7.64 - 7.51 (m, 3H), 7.37 (d, *J=* 7.7 Hz, 1H), 7.29 (d, *J=* 9.1 Hz, 1H), 6.70 (dd, *J=* 15.3, 7.6 Hz, 1H), 6.55 - 6.36 (m, 2H), 5.31 (s, 2H), 4.39 (t, *J=* 15.8 Hz, 2H), 3.05 (d, *J=* 9.7 Hz, 1H), 2.78 (q, *J* = 7.9 Hz, 1H), 2.22 (s, 3H), 2.18 (d, *J* = 8.9 Hz, 1H), 2.10 - 1.95 (m, 1H), 1.80 - 1.69 (m, 2H), 1.66 - 1.52 (m, 1H). MS:595[M+H]⁺.

### Example 12. (R,E)-N-(4-((3-chloro-4-(pyridin-2-ylmethoxy)phenyl)amino)-5-(3,3,3-trifluoropropoxy)quinazolin-6-yl)-3-(1-methylpyrrolidin-2-yl)acrylamide

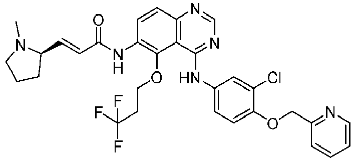

The compound was synthesized using the same method as in Example 4, except that sodium 3,3,3-trifluoropropoxide was used for the reaction instead of sodium methoxide in step 5) of Example 4; ¹H NMR (400 MHz, DMSO-*d*₆) δ 9.87 (s, 1H), 9.57 (s, 1H), 8.60 (d, *J=* 4.9 Hz, 1H), 8.51 (s, 1H), 8.11 (d, *J=* 9.0 Hz, 1H), 7.99 (d, *J=* 2.5 Hz, 1H), 7.89 (t, *J=* 7.7 Hz, 1H), 7.63 - 7.56 (m, 2H), 7.51 (d, *J =* 8.9 Hz, 1H), 7.38 (d, *J* = 7.5 Hz, 1H), 7.29 (d, *J =* 9.0 Hz, 1H), 6.70 (dd, *J =* 15.3, 7.6 Hz, 1H), 6.48 (d, *J=* 15.3 Hz, 1H), 5.31 (s, 2H), 4.22 (t, *J =* 6.0 Hz, 2H), 3.04 (t, *J=* 11.7 Hz, 3H), 2.80 (d, *J=* 8.1 Hz, 1H), 2.23 (s, 3H), 2.19 (d, *J=* 9.7 Hz, 1H), 2.07 -1.95 (m, 1H), 1.75 (ddt, *J=* 12.1 Hz, 2H), 1.66 -1.53 (m, 1H). MS:627[M+H]⁺.

### Example 13. (R,E)-N-(4-((3-chloro-4-(pyridin-2-ylmethoxy)phenyl)amino)-5-propoxyquinazolin-6-yl)-3-(1-methylpyrrolidin-2-yl)acrylamide

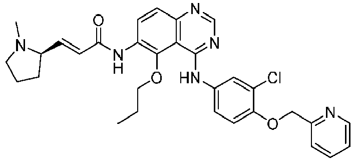

The compound was synthesized using the same method as in Example 4, except that sodium propoxide was used for the reaction instead of sodium methoxide in step 5) of Example 4; ¹H NMR (400 MHz, DMSO-*d*₆) δ 9.92 (s, 1H), 9.86 (s, 1H), 8.63 - 8.56 (m, 1H), 8.54 (s, 1H), 8.17 (d, *J=* 2.7 Hz, 1H), 8.09 (d, *J=* 9.0 Hz, 1H), 7.88 (t, *J=* 7.7 Hz, 1H), 7.62 - 7.49 (m, 3H), 7.37 (dd, *J* = 7.6, 4.9 Hz, 1H), 7.28 (d, *J=* 8.9 Hz, 1H), 6.69 (dd, *J* = 15.3, 7.6 Hz, 1H), 6.46 (d, *J=* 15.3 Hz, 1H), 5.30 (s, 2H), 3.99 (t, *J=* 6.7 Hz, 2H), 3.03 (d, *J=* 9.8 Hz, 1H), 2.75 (q, *J* = 7.9 Hz, 1H), 2.20 (s, 3H), 2.16 (t, *J=* 8.8 Hz, 1H), 2.00 (dt, *J=* 13.0, 8.3 Hz, 1H), 1.91 - 1.65 (m, 4H), 1.57 (dt, *J=* 15.2, 9.2 Hz, 1H), 0.97 (t, *J=* 7.4 Hz, 3H). MS:573[M+H]⁺.

### Example 14. (R,E)-N-(4-((3-chloro-4-(pyridin-2-ylmethoxy)phenyl)amino)-5-isobutoxyquinazolin-6-yl)-3-(1-methylpyrrolidin-2-yl)acrylamide

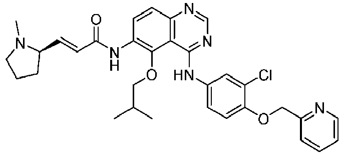

The compound was synthesized using the same method as in Example 4, except that sodium isobutoxide was used for the reaction instead of sodium methoxide in step 5) of Example 4; ¹H NMR (400 MHz, DMSO-*d*₆) δ 9.81 (d, *J=* 8.7 Hz, 2H), 8.60 (d, *J=* 4.8 Hz, 1H), 8.54 (s, 1H), 8.12 (d, *J* = 2.6 Hz, 1H), 7.99 (d, *J* = 8.9 Hz, 1H), 7.89 (t, *J* = 7.7 Hz, 1H), 7.62 - 7.47 (m, 3H), 7.37 (d, *J=* 7.6 Hz, 1H), 7.29 (d, *J=* 9.0 Hz, 1H), 6.68 (dd, *J=* 15.4, 7.7 Hz, 1H), 6.40 (d, *J=* 15.4 Hz, 1H), 5.30 (s, 2H), 3.77 (d, *J=* 6.4 Hz, 2H), 3.04 (dd, *J=* 9.5, 7.0 Hz, 1H), 2.77 (q, *J=* 7.9 Hz, 1H), 2.27 - 2.11 (m, 5H), 2.01 (d, *J=* 12.9 Hz, 1H), 1.74 (t, *J* = 8.5 Hz, 2H), 1.64 - 1.50 (m, 1H), 0.99 (d, *J=* 6.7 Hz, 6H). MS:587[M+H]⁺.

### Example 15. (R,E)-N-(4-((3-chloro-4-(pyridin-2-ylmethoxy)phenyl)amino)-5-(2-hydroxy-2-methylpropoxy)quinazolin-6-yl)-3-(1-methylpyrrolidin-2-yl)acrylamide

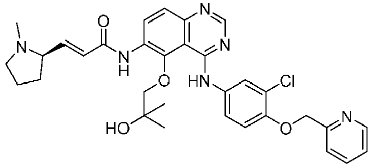

The compound was synthesized using the same method as in Example 4, except that sodium 2-methyl-2-hydroxypropoxide was used for the reaction instead of sodium methoxide in step 5) of Example 4; ¹H NMR (400 MHz, DMSO-*d*₆) δ 10.18 (s, 1H), 9.91 (s, 1H), 8.63 - 8.57 (m, 1H), 8.52 (s, 1H), 8.20 - 8.09 (m, 2H), 7.89 (td, *J* = 7.7 Hz, 1H), 7.68 (dd, *J=* 9.0 Hz, 1H), 7.58 (dd, *J* = 8.4, 4.8 Hz, 2H), 7.37 (dd, *J =* 7.6, 4.8 Hz, 1H), 7.24 (d, *J =* 9.0 Hz, 1H), 6.67 (dd, *J=* 15.3, 7.8 Hz, 1H), 6.30 (d, *J* = 15.3 Hz, 1H), 5.60 (s, 1H), 5.30 (s, 2H), 3.79 (s, 2H), 3.08 - 2.98 (m, 1H), 2.75 (d, *J=* 7.9 Hz, 1H), 2.19 (s, 3H), 2.17 (q, *J=* 9.0 Hz, 1H), 2.05 - 1.96 (m, 1H), 1.79 - 1.67 (m, 2H), 1.57 (d, *J=* 8.2 Hz, 1H), 1.26 (s, 6H). MS:603[M+H]⁺.

### Example 16. (R,E)-N-(4-((3-chloro-4-(pyridin-2-ylmethoxy)phenyl)amino)-5-(3-hydroxy-3-methylbutoxy)quinazolin-6-yl)-3-(1-methylpyrrolidin-2-yl)acrylamide

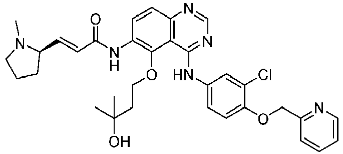

The compound was synthesized using the same method as in Example 4, except that sodium 3-methyl-3-hydroxybutoxide was used for the reaction instead of sodium methoxide in step 5) of Example 4; ¹H NMR (400 MHz, DMSO-*d*₆) δ 10.00 (d, *J=* 6.7 Hz, 2H), 8.60 (d, *J =* 4.8 Hz, 1H), 8.53 (s, 1H), 8.36 (d, *J=* 9.0 Hz, 1H), 8.18 (d, *J=* 2.6 Hz, 1H), 7.89 (t, *J=* 7.7 Hz, 1H), 7.62 - 7.51 (m, 3H), 7.38 (dd, *J* = 7.6, 4.8 Hz, 1H), 7.30 (d, *J=* 9.0 Hz, 1H), 6.69 (dd, *J* = 15.3, 7.8 Hz, 1H), 6.51 (d, *J=* 15.3 Hz, 1H), 5.31 (s, 2H), 4.80 (s, 1H), 4.16 (t, *J* = 6.7 Hz, 2H), 3.04 (dd, *J=* 9.8, 7.2 Hz, 1H), 2.77 (q, *J=* 7.9 Hz, 1H), 2.26 - 2.09 (m, 4H), 2.01 (q, *J*= 5.6, 4.5 Hz, 3H), 1.80 - 1.68 (m, 2H), 1.65 - 1.51 (m, 1H), 1.15 (s, 6H). MS:617[M+H]⁺.

### Example 17. (R,E)-N-(4-((3-chloro-4-(pyridin-2-ylmethoxy)phenyl)amino)-5-(3-hydroxypropoxy)quinazolin-6-yl)-3-(1-methylpyrrolidin-2-yl)acrylamide

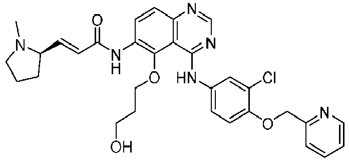

The compound was synthesized using the same method as in Example 4, except that sodium 3-hydroxypropoxide was used for the reaction instead of sodium methoxide in step 5) of Example 4; ¹H NMR (400 MHz, DMSO-*d*₆) δ 9.91 (d, *J* = 6.8 Hz, 2H), 8.68 - 8.57 (m, 1H), 8.52 (s, 1H), 8.33 (d, *J=* 9.1 Hz, 1H), 8.15 (d, *J=* 2.6 Hz, 1H), 7.89 (t, *J=* 7.7 Hz, 1H), 7.62 - 7.52 (m, 3H), 7.37 (dd, *J=* 7.5, 4.8 Hz, 1H), 7.27 (d, *J=* 9.0 Hz, 1H), 6.71 (dd, *J=* 15.3, 7.9 Hz, 1H), 6.50 (d, *J=* 15.3 Hz, 1H), 5.30 (s, 2H), 4.97 (t, *J=* 5.2 Hz, 1H), 4.12 (t, *J=* 6.2 Hz, 2H), 3.67 (q, *J=* 5.5 Hz, 2H), 3.08 (d, *J=* 9.6 Hz, 1H), 2.88 (s, 1H), 2.26 (br, 4H), 2.01 (dt, *J* = 12.3, 6.1 Hz, 3H), 1.77 (dd, *J=* 11.2, 5.7 Hz, 2H), 1.63 (d, *J* = 9.5 Hz, 1H). MS:589[M+H]⁺.

### Example 18. (R,E)-N-(4-((3-chloro-4-((3-fluorobenzyloxy)phenyl)amino)-5-methoaxyquinazolin-6-yl)-3-(1-isopropylpyrrolidine-2-yl)acrylamide

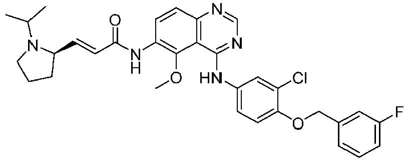

The compound was synthesized using the same method as in Example 4, except that 3-chloro-4-((3-fluorobenzyl)oxy)aniline was used as the raw material instead of 3-chloro-4-(pyridin-2-ylmethoxy)aniline in step 4) of Example 4, and (R,E)-3-(1-isopropylpyrrolidin-2-yl)acryloyl chloride was used for the reaction instead of (R,E)-3-(1-methylpyrrolidin-2-yl)acryloyl chloride in step 7); ¹H NMR (400 MHz, DMSO-*d*₆) δ 9.98 (s, 1H), 9.86 (s, 1H), 8.51 (s, 1H), 8.27 (d, *J=* 9.1 Hz, 1H), 8.09 (d, *J=* 2.6 Hz, 1H), 7.66 - 7.53 (m, 2H), 7.47 (td, *J=* 8.0, 5.9 Hz, 1H), 7.37 - 7.23 (m, 3H), 7.18 (td, *J=* 8.7, 2.6 Hz, 1H), 6.76-6.60 (m, 2H), 5.28 (s, 2H), 3.92 (s, 3H), 2.87 (s, 2H), 1.99 (s, 2H), 1.75 (br, 3H), 1.09 (br, 7H). MS:590[M+H]⁺.

### Example 19. (R,E)-N-(4-((3-chloro-4-((3-fluorobenzyloxy)phenyl)amino)-5-ethoxyquinazolin-6-yl)-3-(1-ethylpyrrolidin-2-yl)acrylamide

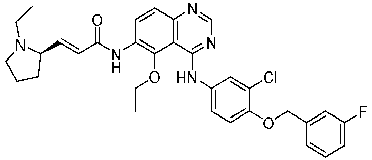

The compound was synthesized using the same method as in Example 4, except that 3-chloro-4-((3-fluorobenzyl)oxy)aniline was used instead of 3-chloro-4-(pyridin-2-ylmethoxy)aniline in step 4) of Example 4, sodium ethoxide was used instead of sodium methoxide in step 5), and (R,E)-3-(1-ethylpyrrolidin-2-yl)acryloyl chloride was used for the reaction instead of (R,E)-3-(1-methylpyrrolidin-2-yl)acryloyl chloride in step 7); ¹H NMR (400 MHz, DMSO-*d*₆) δ 9.99 (br, 2H), 8.54 (s, 1H), 8.18 (q, *J=* 4.1 Hz, 2H), 7.60 - 7.42 (m, 3H), 7.37 - 7.24 (m, 3H), 7.18 (t, *J=* 8.8 Hz, 1H), 6.72 (s, 1H), 6.56 (s, 1H), 5.27 (s, 2H), 4.13 (q, *J=* 7.1 Hz, 2H), 3.17 (s, 1H), 2.96 (s, 1H), 2.73 (s, 1H), 2.27 - 1.93 (m, 3H), 1.78 (s, 2H), 1.60 (s, 1H), 1.41 (t, *J=* 7.0 Hz, 3H), 1.07 (s, 3H). MS:590[M+H]⁺.

### Example 20. (R,E)-N-(4-((3-chloro-4-(pyridin-2-ylmethoxy)phenyl)amino)-5-ethoxyquinazolin-6-yl)-3-(1-ethylpyrrolidin-2-yl)acrylamide

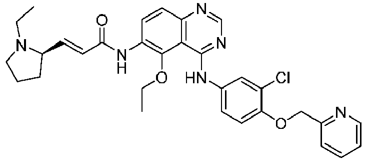

The compound was synthesized using the same method as in Example 4, except that sodium ethoxide was used instead of sodium methoxide in step 5), and (R,E)-3-(1-ethylpyrrolidin-2-yl)acryloyl chloride was used for the reaction instead of (R,E)-3-(1-methylpyrrolidin-2-yl)acryloyl chloride in step 7); ¹H NMR (400 MHz, DMSO-*d*₆) δ 10.00 (s, 2H), 8.60 (d, *J=* 4.9 Hz, 1H), 8.54 (s, 1H), 8.23 - 8.14 (m, 2H), 7.88 (t, *J=* 7.7 Hz, 1H), 7.62 - 7.51 (m, 3H), 7.37 (dd, *J=* 7.6, 4.8 Hz, 1H), 7.28 (d, *J* = 9.0 Hz, 1H), 6.84 - 6.64 (m, 1H), 6.56 (d, *J=* 15.1 Hz, 1H), 5.31 (s, 2H), 4.14 (q, *J=* 7.0 Hz, 2H), 3.27 - 2.94 (m, 2H), 2.77 (s, 1H), 2.25 (s, 2H), 2.03 (s, 1H), 1.79 (s, 2H), 1.62 (s, 1H), 1.41 (t, *J=* 7.0 Hz, 3H), 1.08 (t, *J=* 7.2 Hz, 3H). MS:573[M+H]⁺.

### Example 21. (R,E)-N-(5-ethoxy-4-((3-fluoro-4-(pyridin-2-ylmethoxy)phenyl)amino)quinazolin-6-yl)-3-(1-methylpyrrolidine-2-yl)acrylamide

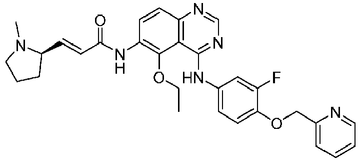

The compound was synthesized using the same method as in Example 4, except that 3-fluoro-4-(pyridin-2-ylmethoxy)aniline was used instead of 3-chloro-4-(pyridin-2-ylmethoxy)aniline in step 4) of Example 4, and sodium ethoxide was used instead of sodium methoxide in step 5) for the reaction; ¹H NMR (400 MHz, DMSO-*d*₆) δ 10.05 (s, 1H), 10.01 (s, 1H), 8.60 (d, *J=* 5.0 Hz, 1H), 8.54 (s, 1H), 8.17 (d, *J=* 9.0 Hz, 1H), 8.07 (d, *J=* 13.6 Hz, 1H), 7.87 (t, *J=* 7.7 Hz, 1H), 7.56 (d, *J=* 8.6 Hz, 2H), 7.41 - 7.33 (m, 2H), 7.28 (t, *J=* 9.2 Hz, 1H), 6.70 (dd, *J=* 15.4, 7.7 Hz, 1H), 6.56 (d, *J=* 15.4 Hz, 1H), 5.27 (s, 2H), 4.13 (q, *J=* 7.0 Hz, 2H), 3.07 (s, 1H), 2.84 (s, 1H), 2.25 (s, 4H), 2.03 (dq, *J=* 13.7, 8.6, 8.1 Hz, 1H), 1.77 (t, *J* = 8.5 Hz, 2H), 1.60 (s, 1H), 1.42 (t, *J=* 7.0 Hz, 3H). MS:543[M+H]⁺.

### Example 22. (R,E)-N-(4-((3-chloro-4-((6-methylpyridin-2-yl)methoxy)phenyl)amino)-5-ethoxyquinazolin-6-yl)-3-(1-methylpyrrolidin-2-yl)acrylamide

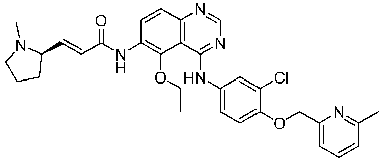

The compound was synthesized using the same method as in Example 4, except that 3-chloro-4-((6-methylpyridin-2-yl)methoxy)aniline was used instead of 3-chloro-4-(pyridin-2-ylmethoxy)aniline in step 4) of Example 4, and sodium ethoxide was used instead of sodium methoxide in step 5) for the reaction; ¹H NMR (400 MHz, DMSO-*d*₆) δ 10.01 (d, *J* = 8.4 Hz, 2H), 8.53 (s, 1H), 8.23 - 8.13 (m, 2H), 7.76 (t, *J* = 7.7 Hz, 1H), 7.59 - 7.51 (m, 2H), 7.36 (d, *J* = 7.7 Hz, 1H), 7.24 (dd, *J=* 14.4, 8.3 Hz, 2H), 6.70 (dd, *J=* 15.3, 7.7 Hz, 1H), 6.56 (d, *J=* 15.3 Hz, 1H), 5.25 (s, 2H), 4.14 (q, *J=* 7.0 Hz, 2H), 3.07 (s, 1H), 2.84 (s, 1H), 2.49 (br, 3H), 2.25 (br, 4H), 2.03 (dq, *J=* 13.3, 7.8 Hz, 1H), 1.81 - 1.70 (m, 2H), 1.62 (q, *J=* 8.7 Hz, 1H), 1.41 (t, *J=* 7.0 Hz, 3H). MS:573[M+H]⁺.

### Example 23. (R,E)-N-(4-((3-chloro-4-(pyridin-2-ylmethoxy)phenyl)amino)-5-(4-methoxybutoxy)quinazolin-6-yl)-3-(1-methylpyrrolidin-2-yl)acrylamide

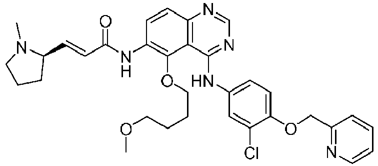

The compound was synthesized using the same method as in Example 4, except that sodium 4-methoxybutoxide was used instead of sodium methoxide in step 5) for the reaction; ¹H NMR (400 MHz, DMSO-*d*₆) δ 9.95 (s, 1H), 9.91 (s, 1H), 8.60 (d, *J=* 4.8 Hz, 1H), 8.53 (s, 1H), 8.14 (d, *J=* 2.6 Hz, 1H), 8.07 (d, *J=* 9.0 Hz, 1H), 7.88 (t, *J=* 7.7 Hz, 1H), 7.61 - 7.50 (m, 3H), 7.37 (dd, *J=* 7.6, 4.8 Hz, 1H), 7.28 (d, *J=* 9.0 Hz, 1H), 6.70 (dd, *J=* 15.3, 7.7 Hz, 1H), 6.49 (d, *J* = 15.3 Hz, 1H), 5.31 (s, 2H), 4.04 (t, *J=* 6.7 Hz, 2H), 3.29 (t, *J* = 6.2 Hz, 2H), 3.15 (s, 3H), 3.05 (d, *J* = 9.2 Hz, 1H), 2.81 (s, 1H), 2.23 (s, 4H), 2.01 (d, *J=* 13.1, 7.5 Hz, 1H), 1.91 - 1.82 (m, 2H), 1.75 (d, *J=* 15.6 Hz, 2H), 1.68 - 1.56 (m, 3H). MS:617[M+H]⁺.

### Example 24. (R,E)-N-(4-((3-chloro-4-(pyridin-2-ylmethoxy)phenyl)amino)-5-methoxyquinazolin-6-yl)-3-(1-isopropylpyrrolidin-2-yl)acrylamide

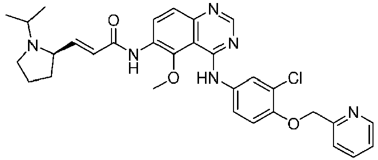

The compound was synthesized using the same method as in Example 4, except that (R,E)-3-(1-isopropylpyrrolidin-2-yl)acryloyl chloride was used instead of (R,E)-3-(1-methylpyrrolidin-2-yl)acryloyl chloride in step 7) for the same reaction; ¹H NMR (400 MHz, DMSO-*d*₆) δ 10.16 - 9.92 (m, 1H), 9.86 (s, 1H), 8.60 (d, *J =* 4.8, 1.2 Hz, 1H), 8.51 (s, 1H), 8.28 (d, *J=* 9.1 Hz, 1H), 8.11 (d, *J=* 2.6 Hz, 1H), 7.88 (t, *J=* 7.7 Hz, 1H), 7.65 - 7.53 (m, 3H), 7.37 (dd, *J* = 7.6, 4.9 Hz, 1H), 7.27 (d, *J=* 9.0 Hz, 1H), 6.78 (br, 1H), 6.63 (br, 1H), 5.31 (s, 2H), 3.93 (s, 3H), 2.90 (br, 2H), 2.00 (br, 2H), 1.76 (br, 3H), 1.09 (br, 7H). MS:573[M+H]⁺.

### Example 25. (R,E)-N-(4-((3-chloro-4-(pyridin-2-ylmethoxy)phenyl)amino)-5-methoxyquinazolin-6-yl)-3-(1-(2-methoxyethyl)pyrrolidin-2-yl)acrylamide

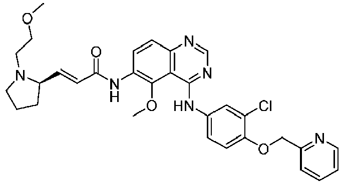

The compound was synthesized using the same method as in Example 4, except that (R,E)-3-(1-(2-methoxyethyl)pyrrolidin-2-yl)acryloyl chloride was used instead of (R,E)-3-(1-methylpyrrolidin-2-yl)acryloyl chloride in step 7) for the reaction; ¹H NMR (400 MHz, DMSO-*d*₆) δ 9.99 (s, 1H), 9.87 (s, 1H), 8.60 (d, *J=* 4.9Hz, 1H), 8.51 (s, 1H), 8.27 (d, *J=* 9.0 Hz, 1H), 8.11 (d, *J=* 2.6 Hz, 1H), 7.89 (t, *J=* 7.7 Hz, 1H), 7.65 - 7.52 (m, 3H), 7.41 - 7.33 (m, 1H), 7.27 (d, *J=* 9.0 Hz, 1H), 6.69 (dd, *J=* 15.3, 7.6 Hz, 1H), 6.57 (d, *J=* 15.4 Hz, 1H), 5.32 (s, 2H), 3.92 (s, 3H), 3.43 (t, *J=* 6.1 Hz, 2H), 3.24 (s, 3H), 3.15 (s, 1H), 3.02 (d, *J=* 9.8 Hz, 1H), 2.84 - 2.76 (m, 1H), 2.25 (s, 2H), 1.98 (dd, *J=* 12.1, 7.0 Hz, 1H), 1.80 - 1.71 (m, 2H), 1.55 (t, *J=* 10.1 Hz, 1H). MS:589[M+H]⁺.

### Example 26. (R,E)-N-(4-((3-chloro-4-((3-fluorobenzyloxy)phenyl)amino)-5-methoxyquinazolin-6-yl)-3-(1-(2-methoxyethyl))pyrrolidin-2-yl)acrylamide

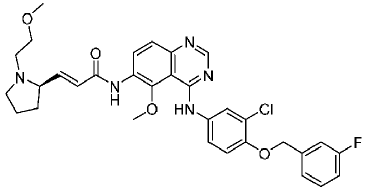

The compound was synthesized using the same method as in Example 4, except that 3-chloro-4-((3-fluorobenzyl)oxy)aniline was used as the raw material instead of 3-chloro-4-(pyridin-2-ylmethoxy)aniline in step 4) of Example 4, and (R,E)-3-(1-(2-methoxyethyl)pyrrolidin-2-yl)acryloyl chloride was used instead of (R,E)-3-(1-methylpyrrolidin-2-yl)acryloyl chloride in step 7) for the reaction; ¹H NMR (400 MHz, DMSO-*d*₆) δ 9.96 (s, 1H), 9.86 (s, 1H), 8.51 (s, 1H), 8.28 (d, *J=* 9.1 Hz, 1H), 8.09 (d, *J=* 2.6 Hz, 1H), 7.62 (d, *J=* 8.9Hz, 1H), 7.56 (d, *J=* 9.0 Hz, 1H), 7.47 (td, *J=* 8.0, 5.9 Hz, 1H), 7.37 - 7.24 (m, 3H), 7.18 (td, *J=* 8.7 Hz, 1H), 6.69 (dd, *J=* 15.3, 7.7 Hz, 1H), 6.56 (d, *J=* 15.4 Hz, 1H), 5.28 (s, 2H), 3.92 (s, 3H), 3.43 (t, *J=* 6.1 Hz, 2H), 3.24 (s, 3H), 3.15 (s, 1H), 3.00 (s, 1H), 2.84 - 2.75 (m, 1H), 2.42 - 2.17 (m, 2H), 1.97 (q, *J=* 8.7, 8.2 Hz, 1H), 1.76 (t, *J* = 7.4 Hz, 2H), 1.55 (s, 1H). MS:606[M+H]⁺.

### Example 27. (R,E)-N-(4-((3-chloro-4-((6-methoxypyridin-2-yl)methoxy)phenyl)amino)-5-methoxyquinazolin-6-yl)-3-(1-methylpyrrolidin-2-yl)acrylamide

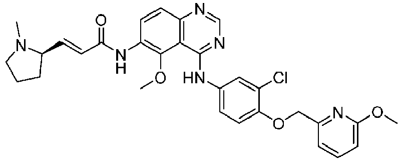

The compound was synthesized using the same method as in Example 4, except that 3-chloro-4-((6-methoxypyridin-2-yl)methoxy)aniline was used as the raw material instead of 3-chloro-4-(pyridin-2-ylmethoxy)aniline in step 4) of Example 4 for the reaction; ¹H NMR (400 MHz, DMSO-*d*₆) δ 10.06 (s, 1H), 9.87 (s, 1H), 8.51 (s, 1H), 8.26 (d, *J=* 9.0 Hz, 1H), 8.11 (d, *J=* 2.6 Hz, 1H), 7.76 (dd, *J=* 8.3, 7.3 Hz, 1H), 7.65 - 7.52 (m, 2H), 7.27 (d, *J=* 9.0 Hz, 1H), 7.14 (d, *J=* 7.2 Hz, 1H), 6.81 - 6.57 (m, 3H), 5.22 (s, 2H), 3.93 (s, 3H), 3.86 (s, 3H), 3.06 (t, *J* = 8.2 Hz, 1H), 2.84 (s, 1H), 2.24 (br, 4H), 2.01 (br, 1H), 1.75 (t, *J* = 9.0 Hz, 2H), 1.67 - 1.54 (m, 1H). MS:575[M+H]⁺.

### Example 28. (R,E)-N-(4-((3-chloro-4-((3-fluorobenzyloxy)phenyl)amino)-5-methoxyquinazolin-6-yl)-3-(1-ethylpyrrolidin-2-yl)acrylamide

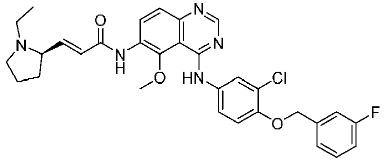

The compound was synthesized using the same method as in Example 4, except that 3-chloro-4-((3-fluorobenzyl)oxy)aniline was used as the raw material instead of 3-chloro-4-(pyridin-2-ylmethoxy)aniline in step 4) of Example 4, and (R,E)-3-(1-ethylpyrrolidin-2-yl)acryloyl chloride was used instead of (R,E)-3-(1-methylpyrrolidin-2-yl)acryloyl chloride in step 7) for the reaction; ¹H NMR (400 MHz, DMSO-*d*₆) δ 10.04 (s, 1H), 9.86 (s, 1H), 8.51 (s, 1H), 8.27 (d, *J=* 9.0 Hz, 1H), 8.09 (d, *J=* 2.6 Hz, 1H), 7.66 - 7.53 (m, 2H), 7.47 (td, *J=* 8.0, 5.9 Hz, 1H), 7.37 - 7.23 (m, 3H), 7.18 (td, *J=* 8.7, 2.7 Hz, 1H), 6.76 (s, 1H), 6.63 (s, 1H), 5.28 (s, 2H), 3.93 (s, 3H), 3.31 - 3.07 (m, 1H), 2.83 (d, *J=* 29.9 Hz, 1H), 2.40 - 2.12 (m, 3H), 2.05 (s, 1H), 1.82 (s, 2H), 1.65 (s, 1H), 1.10 (s, 3H). MS:576[M+H]⁺.

### Example 29. (R,E)-N-(4-((3-chloro-4-(pyridin-2-ylmethoxy)phenyl)amino)-5-methoxyquinazolin-6-yl)-3-(1-ethylpyrrolidin-2-yl)acrylamide

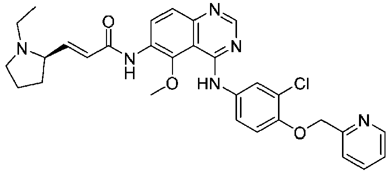

The compound was synthesized using the same method as in Example 4, except that (R,E)-3-(1-ethylpyrrolidin-2-yl)acryloyl chloride was used instead of (R,E)-3-(1-methylpyrrolidin-2-yl)acryloyl chloride in step 7) for the reaction; ¹H NMR (400 MHz, DMSO-*d*₆) δ 9.99 (s, 1H), 9.86 (s, 1H), 8.60 (dt, *J* = 4.8, 1.2 Hz, 1H), 8.51 (s, 1H), 8.28 (d, *J=* 9.0 Hz, 1H), 8.11 (d, *J* = 2.6 Hz, 1H), 7.88 (td, *J=* 7.7, 1.8 Hz, 1H), 7.64 - 7.53 (m, 3H), 7.41 - 7.33 (m, 1H), 7.27 (d, *J* = 9.0 Hz, 1H), 6.73 (dd, *J* = 15.4, 7.8 Hz, 1H), 6.58 (d, *J=* 15.5 Hz, 1H), 5.32 (s, 2H), 3.92 (s, 3H), 3.18 (s, 1H), 2.75 (s, 1H), 2.22 (s, 3H), 2.02 (s, 1H), 1.78 (d, *J* = 8.8 Hz, 2H), 1.61 (s, 1H), 1.07 (t, *J=* 7.4 Hz, 3H). MS:559[M+H]⁺.

### Example 30. (R,E)-N-(4-((3-fluoro-4-(pyridin-2-ylmethoxy)phenyl)amino)-5-methoxyquinazolin-6-yl)-3-(1-methylpyrrolidin-2-yl)acrylamide

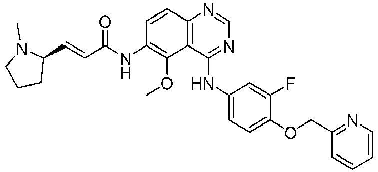

The compound was synthesized using the same method as in Example 4, except that 3-fluoro-4-(pyridin-2-ylmethoxy)aniline was used instead of 3-chloro-4-(pyridin-2-ylmethoxy)aniline in step 4) of Example 4 for the reaction; ¹H NMR (400 MHz, DMSO-*d*₆) δ 10.01 (s, 1H), 9.90 (s, 1H), 8.63 - 8.56 (m, 1H), 8.53 (s, 1H), 8.29 (d, *J =* 9.0 Hz, 1H), 8.00 (dd, *J=* 13.5, 2.6 Hz, 1H), 7.87 (td, *J* = 7.7, 1.8 Hz, 1H), 7.56 (dd, *J=* 8.4, 4.7 Hz, 2H), 7.49 - 7.33 (m, 2H), 7.27 (t, *J=* 9.3 Hz, 1H), 6.71 (dd, *J=* 15.2, 7.5 Hz, 1H), 6.59 (d, *J=* 15.4 Hz, 1H), 5.28 (s, 2H), 3.92 (s, 3H), 3.08 (s, 1H), 2.85 (s, 1H), 2.26 (s, 4H), 2.04 (s, 1H), 1.77 (s, 2H), 1.62 (s, 1H). MS:529[M+H]⁺.

### Example 31. (R,E)-N-(5-(2-hydroxyethoxy)-4-((4-phenyloxyphenyl)amino)quinazolin-6-yl)-3-(1-methylpyrrolidin-2-yl)acrylamide

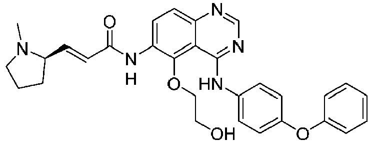

The compound was synthesized using the same method as in Example 4, except that 4-phenyloxyaniline was used instead of 3-chloro-4-(pyridin-2-ylmethoxy)aniline in step 4) of Example 4, and sodium 2-hydroxyethoxide was used instead of sodium methoxide in step 5) for the reaction; ¹H NMR (400 MHz, DMSO-*d*₆) δ 10.22 (s, 1H), 9.98 (s, 1H), 8.49 (s, 1H), 8.34 (d, *J=* 9.1 Hz, 1H), 7.88 (d, *J=* 9.1 Hz, 2H), 7.57 (d, *J=* 9.1 Hz, 1H), 7.39 (t, *J=* 7.8 Hz, 2H), 7.17 - 6.98 (m, 5H), 6.69 (dd, *J=* 15.3, 7.7 Hz, 1H), 6.43 (d, *J=* 15.3 Hz, 1H), 5.69 (t, *J* = 4.5 Hz, 1H), 4.10 (t, *J=* 4.1 Hz, 2H), 3.92 - 3.85 (m, 2H), 3.09 - 2.99 (m, 1H), 2.76 (q, *J=* 8.0 Hz, 1H), 2.21 (s, 3H), 2.17 (t, *J* = 8.8 Hz, 1H), 2.01 (dtd, *J* = 13.0, 8.2, 5.6 Hz, 1H), 1.74 (td, *J=* 8.8, 4.5 Hz, 2H), 1.59 (td, *J=* 11.6, 4.8 Hz, 1H). MS:526[M+H]⁺.

### Example 32. (R,E)-N-(4-((3-chloro-4-(pyridin-2-ylmethoxy)phenyl)amino)-5-((3-methoxypropyl)amino)quinazolin-6-yl)-3-(1-methylpyrrolidin-2-yl)acrylamide

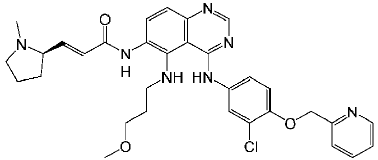

The compound was synthesized using the same method as in Example 4, except that 3-methoxypropylamine was used instead of sodium methoxide in step 5) for the reaction;

¹H NMR (400 MHz, DMSO-*d*₆) δ 10.61 (s, 1H), 10.23 (s, 1H), 8.83 (s, 1H), 8.61 (dd, *J* = 5.0, 1.6 Hz, 1H), 8.12 - 8.01 (m, 2H), 7.90 (td, *J=* 7.7, 1.8 Hz, 1H), 7.59 (q, *J=* 11.3, 7.4 Hz, 3H), 7.43 - 7.32 (m, 2H), 7.29 - 6.94 (m, 1H), 6.85 (dd, *J=* 15.3, 8.8 Hz, 1H), 6.62 (d, *J* = 15.3 Hz, 1H), 5.35 (s, 2H), 5.22 (d, *J* = 12.6 Hz, 1H), 4.13 (d, *J* = 9.4 Hz, 1H), 3.72 (s, 1H), 3.34 (t, *J=* 6.0 Hz, 2H), 3.16 (d, *J=* 7.7 Hz, 1H), 3.11 (s, 3H), 2.96 (t, *J=* 6.9 Hz, 2H), 2.83 (s, 2H), 2.75 - 2.62 (m, 1H), 2.33 (q, *J=* 4.1, 3.0 Hz, 1H), 2.16 - 1.95 (m, 1H), 1.97 - 1.84 (m, 1H), 1.75 (t, *J=* 6.5 Hz, 2H). MS:602[M+H]⁺.

### Example 33. (R,E)-N-(4-((3-chloro-4-(pyridin-2-ylmethoxy)phenyl)amino)-5-(methoxy-d3)quinazolin-6-yl)-3-(1-methylpyrrolidin-2-yl)acrylamide

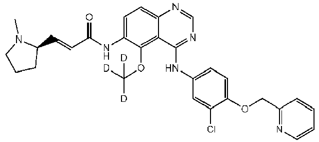

The compound was synthesized using the same method as in Example 4, except that sodium trideuterooxide was used instead of sodium methoxide in step 5) for the reaction;

¹H NMR (400 MHz, DMSO-*d*₆) δ 9.93 (s, 1H), 9.87 (s, 1H), 8.60 (d, *J* = 4.7 Hz, 1H), 8.51 (s, 1H), 8.29 (d, *J* = 9.1 Hz, 1H), 8.11 (d, *J* = 2.6 Hz, 1H), 7.88 (td, *J=* 7.7, 1.8 Hz, 1H), 7.64 - 7.52 (m, 3H), 7.37 (dd, *J=* 7.5, 4.9 Hz, 1H), 7.26 (d, *J=* 9.0 Hz, 1H), 6.70 (dd, *J=* 15.3, 7.6 Hz, 1H), 6.57 (d, *J=* 15.4 Hz, 1H), 5.31 (s, 2H), 3.04 (dd, *J=* 9.9, 7.2 Hz, 1H), 2.78 (q, *J* = 7.9 Hz, 1H), 2.22 (br, 4H), 2.01 (dt, *J=* 13.2, 8.2 Hz, 1H), 1.74 (td, *J=* 9.0, 5.5 Hz, 2H), 1.59 (dd, *J=* 12.3, 9.4 Hz, 1H). MS:548[M+H]⁺.

### Example 34. (R,E)-N-(4-((3-chloro-4-(pyridin-2-ylmethoxy)phenyl)amino)-5-(methoxy-d3)quinazolin-6-yl)-3-(1-ethylpyrrolidin-2-yl)acrylamide

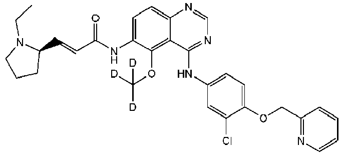

The compound was synthesized using the same method as in Example 4, except that sodium trideuterooxide was used instead of sodium methoxide in step 5), and (R,E)-3-(1-ethylpyrrolidin-2-yl)acryloyl chloride was used instead of (R,E)-3-(1-methylpyrrolidin-2-yl)acryloyl chloride in step 7) for the reaction;

¹H NMR (400 MHz, DMSO-*d*₆) δ 9.94 (s, 1H), 9.87 (s, 1H), 8.60 (d, *J=* 4.7 Hz, 1H), 8.51 (s, 1H), 8.28 (d, *J* = 9.1 Hz, 1H), 8.11 (d, *J* = 2.6 Hz, 1H), 7.88 (td, *J* = 7.7, 1.9 Hz, 1H), 7.64 - 7.52 (m, 3H), 7.37 (dd, *J=* 7.5, 4.8 Hz, 1H), 7.27 (d, *J=* 9.0 Hz, 1H), 6.70 (dd, *J=* 15.3, 7.7 Hz, 1H), 6.55 (d, *J* = 15.4 Hz, 1H), 5.31 (s, 2H), 3.14 (s, 1H), 2.95 (s, 1H), 2.71 (dd, *J =* 13.0, 7.9 Hz, 1H), 2.14 (s, 2H), 1.99 (dd, *J=* 12.8, 6.9 Hz, 1H), 1.75 (q, *J=* 8.1, 7.6 Hz, 2H), 1.57 (t, *J=* 10.0 Hz, 1H), 1.04 (t, *J=* 7.1 Hz, 3H). MS:562[M+H]⁺.

### Example 35. (R,E)-N-(4-((3-chloro-4-(pyridin-2-ylmethoxy)phenyl)amino)-5-(methoxy-d3)quinazolin-6-yl)-3-(1-isopropylpyrrolidin-2-yl)acrylamide

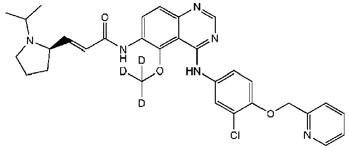

The compound was synthesized using the same method as in Example 4, except that sodium trideuterooxide was used instead of sodium methoxide in step 5), and (R,E)-3-(1-isopropylpyrrolidin-2-yl)acryloyl chloride was used instead of (R,E)-3-(1-methylpyrrolidin-2-yl)acryloyl chloride in step 7) for the reaction;

¹H NMR (400 MHz, DMSO-*d*₆) δ 9.88 (d, *J* = 9.2 Hz, 2H), 8.60 (d, *J=* 4.8 Hz, 1H), 8.51 (s, 1H), 8.28 (d, *J* = 9.1 Hz, 1H), 8.11 (d, *J* = 2.7 Hz, 1H), 7.88 (t, *J=* 7.7 Hz, 1H), 7.64 - 7.52 (m, 3H), 7.37 (dd, *J=* 7.5, 4.8 Hz, 1H), 7.26 (d, *J=* 8.9 Hz, 1H), 6.73 (dd, *J=* 15.3, 7.5 Hz, 1H), 6.55 (d, *J=* 15.2 Hz, 1H), 5.31 (s, 2H), 3.41- 3.36 (m, 1H), 2.91- 2.78 (m, 2H), 2.55 (d, *J* = 7.8 Hz, 1H), 1.95 (dd, *J=* 12.1, 7.4 Hz, 1H), 1.70 (q, *J* = 8.0 Hz, 2H), 1.55 (q, *J* = 7.3, 6.6 Hz, 1H), 1.05 (d, *J=* 6.6 Hz, 3H), 0.95 (d, *J=* 6.3 Hz, 3H). MS:576[M+H]⁺.

### Example 36. (E)-N-(4-((3-chloro-4-(pyridin-2-ylmethoxy)phenyl)amino)-5-(methoxy-d3)quinazolin-6-yl)-4-(dimethylamino)but-2-enamide

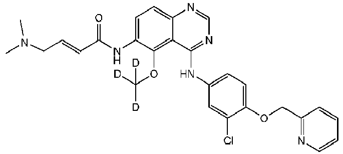

The compound was synthesized using the same method as in Example 1, except that sodium trideuterooxide was used instead of sodium methoxide in step 5) for the reaction;

¹H NMR (400 MHz, DMSO-*d*₆) δ 9.94 (s, 1H), 9.87 (s, 1H), 8.60 (dt, *J* = 4.7, 1.3 Hz, 1H), 8.51 (s, 1H), 8.28 (d, *J* = 9.0 Hz, 1H), 8.11 (d, *J* = 2.6 Hz, 1H), 7.88 (td, *J* = 7.7, 1.8 Hz, 1H), 7.65 - 7.52 (m, 3H), 7.37 (ddd, *J=* 7.6, 4.8, 1.2 Hz, 1H), 7.27 (d, *J* = 9.0 Hz, 1H), 6.80 (dt, *J=* 15.4, 5.9 Hz, 1H), 6.58 (d, *J =* 15.5 Hz, 1H), 5.32 (s, 2H), 3.09 (dd, *J=* 6.0, 1.5 Hz, 2H), 2.20 (s, 6H). MS:522[M+H]⁺.

### Example 37. (R,E)-N-(4-((3-chloro-4-(pyridin-2-ylmethoxy)phenyl)amino)-5-cyclopropoxyquinazolin-6-yl)-3-(1-methylpyrrolidin-2-yl)acrylamide

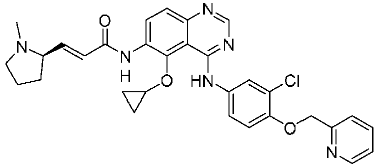

The compound was synthesized using the same method as in Example 4, except that sodium cyclopropoxide was used instead of sodium methoxide in step 5) for the reaction;

¹H NMR (400 MHz, DMSO-*d*₆) δ 10.46 (s, 1H), 10.40 (s, 1H), 8.84 (s, 1H), 8.66 - 8.60 (m, 1H), 8.34 (d, *J* = 9.0 Hz, 1H), 7.98 - 7.88 (m, 2H), 7.65 (dd, *J=* 24.5, 8.4 Hz, 2H), 7.51 (dd, *J=* 8.9, 2.6 Hz, 1H), 7.46 - 7.32 (m, 2H), 6.90 - 6.74 (m, 2H), 5.37 (s, 2H), 4.30 (tt, *J=* 6.3, 3.0 Hz, 1H), 4.10 (t, *J=* 8.0 Hz, 1H), 3.71 (br, 1H), 3.17 (br, 1H), 2.83 (s, 3H), 2.33 (dd, *J* = 12.6, 8.2 Hz, 1H), 2.17 - 1.87 (m, 3H), 0.98 (br, 2H), 0.68 - 0.59 (m, 2H). MS:571[M+H]⁺.

### Example 38. (R,E)-N-(4-((3-chloro-4-(pyridin-2-ylmethoxy)phenyl)amino)-5-(cyclopropylmethoxy)quinazolin-6-yl)-3-(1-methylpyrrolidone-2-yl)acrylamide

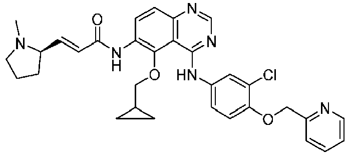

The compound was synthesized using the same method as in Example 4, except that sodium cyclopropylmethoxide was used instead of sodium methoxide in step 5) for the reaction;

¹H NMR (400 MHz, DMSO-*d*₆) δ 10.18 (s, 1H), 9.94 (s, 1H), 8.60 (d, *J* = 4.9 Hz, 1H), 8.54 (s, 1H), 8.20 (d, *J=* 2.6 Hz, 1H), 8.13 (d, *J=* 9.0 Hz, 1H), 7.89 (t, *J=* 7.7 Hz, 1H), 7.57 (dd, *J* = 9.0, 7.3 Hz, 3H), 7.37 (dd, *J=* 7.5, 4.8 Hz, 1H), 7.28 (d, *J=* 9.0 Hz, 1H), 6.68 (dd, *J=* 15.3, 7.7 Hz, 1H), 6.48 (d, *J=* 15.4 Hz, 1H), 5.30 (s, 2H), 3.96 (d, *J=* 7.4 Hz, 2H), 3.04 (dd, *J* = 9.7, 7.2 Hz, 1H), 2.76 (q, *J* = 7.9 Hz, 1H), 2.21 (s, 3H), 2.17 (t, *J* = 8.8 Hz, 1H), 2.06 - 1.94 (m, 1H), 1.74 (dd, *J=* 14.6, 7.2 Hz, 2H), 1.65 - 1.52 (m, 1H), 1.29 (t, *J=* 7.7 Hz, 1H), 0.55 - 0.45 (m, 2H), 0.28 - 0.19 (m, 2H). MS:585[M+H]⁺.

### Example 39. (R,E)-N-(4-((3-chloro-4-(pyridin-2-ylmethoxy)phenyl)amino)-5-cyclobutoxyquinazolin-6-yl)-3-(1-methylpyrrolidin-2-yl)acrylamide

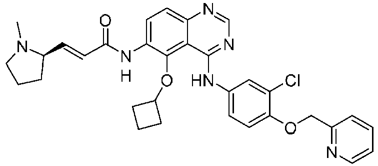

The compound was synthesized using the same method as in Example 4, except that sodium cyclobutoxide was used instead of sodium methoxide in step 5) for the reaction;

¹H NMR (400 MHz, DMSO-*d*₆) δ 9.89 (d, *J* = 18.8 Hz, 2H), 8.64 - 8.57 (m, 1H), 8.54 (s, 1H), 8.21 (d, *J=* 2.6 Hz, 1H), 8.13 (d, *J* = 9.0 Hz, 1H), 7.89 (t, *J=* 7.7 Hz, 1H), 7.62 - 7.51 (m, 3H), 7.42 - 7.33 (m, 1H), 7.28 (d, *J=* 9.0 Hz, 1H), 6.68 (dd, *J=* 15.3, 7.7 Hz, 1H), 6.47 (d, *J=* 15.4 Hz, 1H), 5.31 (s, 2H), 4.56 (t, *J=* 7.7 Hz, 1H), 3.04 (dd, *J=* 9.6, 7.1 Hz, 1H), 2.76 (q, *J=* 8.0 Hz, 1H), 2.29 (dd, *J* = 10.0, 8.3 Hz, 2H), 2.25 - 2.10 (m, 6H), 2.00 (d, *J* = 12.9 Hz, 1H), 1.81 - 1.68 (m, 2H), 1.69 - 1.53 (m, 2H), 1.37 (t, *J=* 10.5 Hz, 1H). MS:585[M+H]⁺.

### Example 40. (E)-N-(4-((3-chloro-4-(pyridin-2-ylmethoxy)phenyl)amino)-5-((tetrahydrofuran-3-yl)oxy)quinazolin-6-yl)-3-((R)-1-methylpyrrolidin-2-yl)acrylamide

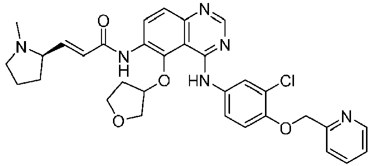

The compound was synthesized using the same method as in Example 4, except that sodium tetrahydrofuran-3-olate was used instead of sodium methoxide in step 5) for the reaction; ¹H NMR (400 MHz, DMSO-*d*₆) δ 9.98 (s, 1H), 9.83 (s, 1H), 8.60 (dt, *J=* 4.8 Hz, 1H), 8.54 (s, 1H), 8.17 (d, *J=* 2.7 Hz, 1H), 8.10 (d, *J=* 9.1 Hz, 1H), 7.89 (td, *J* = 7.7 Hz, 1H), 7.67 - 7.53 (m, 3H), 7.37 (dd, *J=* 7.6, 4.8 Hz, 1H), 7.27 (d, *J=* 9.0 Hz, 1H), 6.71 (dd, *J=* 15.4, 7.7 Hz, 1H), 6.46 (d, *J=* 15.4 Hz, 1H), 5.30 (s, 2H), 5.05 (d, *J=* 6.2 Hz, 1H), 4.16 (d, *J=* 10.8 Hz, 1H), 3.96 (q, *J=* 7.2 Hz, 1H), 3.68 (td, *J=* 8.5, 5.7 Hz, 1H), 3.55 (dd, *J* = 10.7, 4.6 Hz, 1H), 3.05 (dd, *J* = 9.7, 7.1 Hz, 1H), 2.83 - 2.76 (m, 1H), 2.26 - 2.01 (m, 7H), 1.75 (dd, *J* = 12.0, 9.0 Hz, 2H), 1.66 - 1.54 (m, 1H). MS:601[M+H]⁺.

### Example 41. (R,E)-N-(4-((3-chloro-4-((3-fluorobenzyl)oxy)phenyl)amino)-5-cyclopropoxyquinazolin-6-yl)-3-(1-methylpyrrolidone-2-yl)acrylamide

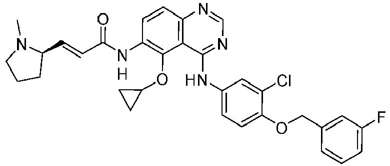

The compound was synthesized using the same method as in Example 4, except that 3-chloro-4-((3-fluorobenzyl)oxy)aniline was used instead of 3-chloro-4-(pyridin-2-ylmethoxy)aniline in Step 4) of Example 4, and sodium cyclopropoxide was used instead of sodium methoxide in step 5) for the reaction; ¹H NMR (400 MHz, DMSO-*d*₆) δ 10.40 (s, 1H), 10.29 (s, 1H), 10.11 (s, 1H), 8.74 (s, 1H), 8.28 (d, *J=* 9.0 Hz, 1H), 8.00 (d, *J=* 2.6 Hz, 1H), 7.65 (d, *J=* 9.0 Hz, 1H), 7.56 - 7.43 (m, 2H), 7.33 (dd, *J=* 8.2, 5.8 Hz, 3H), 7.20 (td, *J=* 8.7, 2.6 Hz, 1H), 6.89 - 6.74 (m, 2H), 5.31 (s, 2H), 4.26 (tt, *J=* 6.5, 3.1 Hz, 1H), 4.10 (s, 1H), 3.71 (s, 1H), 3.16 (d, *J=* 10.0 Hz, 1H), 2.83 (s, 3H), 2.33 (tq, *J=* 8.6, 4.1, 3.5 Hz, 1H), 2.16 - 1.84 (m, 2H), 0.97 (tq, *J=* 5.7, 3.3 Hz, 2H), 0.67 - 0.60 (m, 2H). MS:588[M+H]⁺.

### Example 42. (R,E)-N-(4-((3-chloro-4-(pyridin-2-ylmethoxy)phenyl)amino)-5-methoxyquinazolin-6-yl)-3-(1-cyclobutylpyrrolidin-2-yl)acrylamide

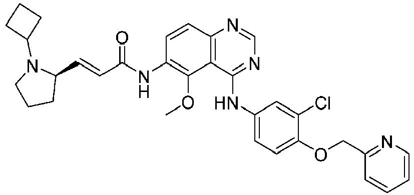

The compound was synthesized using the same method as in Example 4, except that (R,E)-3-(1-cyclobutylpyrrolidin-2-yl)acryloyl chloride was used instead of (R,E)-3-(1-methylpyrrolidin-2-yl)acryloyl chloride in Step 7) of Example 4 for the reaction; ¹H NMR (400 MHz, DMSO-*d*₆) δ 9.92 (s, 1H), 9.86 (s, 1H), 8.60 (d, *J=* 4.8 Hz, 1H), 8.51 (s, 1H), 8.27 (d, *J* = 9.0 Hz, 1H), 8.11 (d, *J =* 2.6 Hz, 1H), 7.89 (td, *J=* 7.7, 1.8 Hz, 1H), 7.65 - 7.52 (m, 3H), 7.37 (dd, *J=* 7.5, 4.9 Hz, 1H), 7.27 (d, *J=* 9.0 Hz, 1H), 6.75 (s, 1H), 6.54 (d, *J=* 15.2 Hz, 1H), 5.32 (s, 2H), 4.12 (q, *J=* 5.3 Hz, 2H), 3.92 (s, 3H), 3.38 (d, *J=* 7.2 Hz, 1H), 3.0 (s, 1H), 1.93 (s, 5H), 1.75 (s, 2H), 1.62 (s, 3H). MS: 585 [M+H]⁺.

### Example 43. (E)-N-(4-((3-chloro-4-(pyridin-2-ylmethoxy)phenyl)amino)-5-methoxyquinazolin-6-yl)-4-(pyrrolidine-1-yl)but-2-enamide

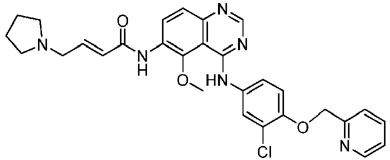

The compound was synthesized using the same method as in Example 4, except that (E)-4-(pyrrolidine-1-yl)but-2-enoyl chloride was used instead of (R,E)-3-(1-methylpyrrolidin-2-yl)acryloyl chloride in Step 7) of Example 4 for the reaction; ¹H NMR (400 MHz, DMSO-*d*₆) δ 9.95 (s, 1H), 9.87 (s, 1H), 8.60 (dt, *J=* 4.8, 1.4 Hz, 1H), 8.51 (s, 1H), 8.28 (d, *J* = 9.0 Hz, 1H), 8.12 (d, *J=* 2.6 Hz, 1H), 7.89 (td, *J=* 7.7, 1.8 Hz, 1H), 7.65 - 7.5 3 (m, 3H), 7.37 (ddd, *J* = 7.6, 4.9, 1.2 Hz, 1H), 7.27 (d, *J=* 9.0 Hz, 1H), 6.91 - 6.75 (m, 1H), 6.60 (d, *J=* 15.4 Hz, 1H), 5.32 (s, 2H), 3.93 (s, 3H), 3.28 (dd, *J* = 5.7, 1.7 Hz, 2H), 2.55 - 2.52 (m, 4H), 1.73 (br, 4H). MS: 545 [M+H]⁺.

### Example 44. (E)-N-(4-((3-chloro-4-(pyridin-2-ylmethoxy)phenyl)amino)-5-methoxyquinazolin-6-yl)-4-(piperidin-1-yl)but-2-enamide

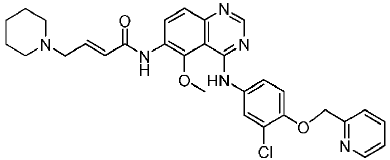

The compound was synthesized using the same method as in Example 4, except that (E)-4-(piperidin-1-yl)but-2-enoyl chloride was used instead of (R,E)-3-(1-methylpyrrolidin-2-yl)acryloyl chloride in Step 7) of Example 4 for the reaction; ¹H NMR (400 MHz, DMSO-*d*₆) δ 9.95 (s, 1H), 9.87 (s, 1H), 8.60 (ddd, *J* = 4.8, 1.8, 0.9 Hz, 1H), 8.52 (s, 1H), 8.27 (d, *J=* 9.0 Hz, 1H), 8.12 (d, *J* = 2.6 Hz, 1H), 7.89 (td, *J* = 7.7, 1.8 Hz, 1H), 7.65 - 7.53 (m, 3H), 7.37 (ddd, *J* = 7.6, 4.9, 1.2 Hz, 1H), 7.27 (d, *J=* 9.0 Hz, 1H), 6.81 (dt, *J* = 15.5, 6.0 Hz, 1H), 6.56 (d, *J* = 15.4 Hz, 1H), 5.32 (s, 2H), 3.92 (s, 3H), 3.12 (d, *J=* 5.9 Hz, 2H), 2.38 (s, 4H), 1.54 (p, *J=* 5.5 Hz, 4H), 1.41 (d, *J=* 6.7 Hz, 2H). MS: 559 [M+H]⁺.

In order to characterize the biochemical activity, pharmacological properties, etc. of the compounds of the present application, the compounds of the present application, as well as pyrotinib, neratinib, and control compounds 1 and 2, were assayed as follows. Among them, the synthesis of pyrotinib and control compound 2 is described in Chinese Patent CN102471312, and control compound 1 is a stereoisomer of the R configuration of the control compound 2.

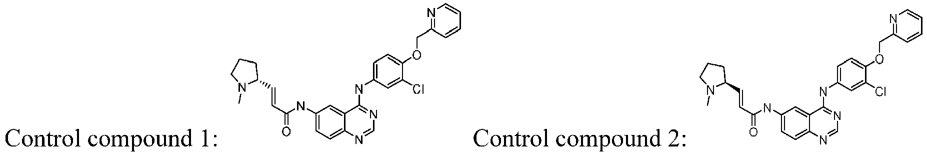

### Assay example 1. Assay of inhibition of EGFR^{WT} and HER2 kinase activity by small molecule compounds

Reagents and consumables: ULightTM-labeled Ploy GT Peptide (Perkin Elmer, Cat. No. TRF-0100-M); ULightTM-labeled JAK-1 (Try 1023) Peptide (Perkin Elmer, Cat. No. TRF-0121-M); Eu-W1024-labeled Anti-Phosphotyrosine Antibody (PT66) (Perkin Elmer, Cat. No. AD0068); 10× Detection Buffer (Perkin Elmer, Cat. No. CR97-100); Her2 Kinase (Carna Biosciences, Cat. No. 08-016); EGFR kinase (Carna Biosciences, Cat. No. 08-115); HEPES (GIBCO, Cat. No. 15630-080); EGTA (Sigma, Cat. No. 03777-10G); EDTA (Sigma, Cat. No. EDS- 100G); MgCl₂ (Sigma, Cat. No. 63069-100ML); DTT (Sigma, Cat. No. 43816-10ML); Tween-20 (Sigma, Cat. No. P7949-100ML); DMSO (Life Science, Cat. No. 0231-500ML); 384-well plates (Perkin Elmer, Cat. No. 607290); multifunctional plate reader (Perkin Elmer, Cat. No. Envision)

Preparation of compound solution: The assay compound was dissolved in DMSO to form a 10 mM stock solution. Prior to use, the compound was diluted to 0.25 mM in DMSO (100 times the final concentration of the diluent) and then subjected to a 3-fold concentration gradient dilution to obtain a total of 11 concentrations. The solution was diluted with buffer to a diluent with 4x the final concentration when the compound was added.

HER2 kinase assay: buffer was prepared, and 40 nM 4X Her2 kinase solution, 40 µM 4X ATP solution, and 400 nM 4×ULight^{™}-labeled Ploy GT Peptide substrate solution were prepared using the buffer. After the preparation was completed, the enzyme was mixed with different concentrations of compounds pre-prepared by dilution, and the mixture was left to stand at room temperature for 5 min. Each concentration was performed in duplicate. The corresponding substrate and ATP were added and the mixture was reacted at room temperature for 120 minutes (with negative and positive controls). After completion of the reaction, PT66 detection antibody was added. The mixture was incubated at room temperature for 60 minutes, and then detected by Envision.

EGFR^{WT} kinase assay: buffer was prepared, and 3.48 nM 4X EGFR kinase solution, 600 µM 4XATP solution, and 400 nM 4×ULight^{™}-labeled JAK-1 (Try1023) Peptide substrate solution were prepared using the buffer. After the preparation was completed, the enzyme was mixed with different concentrations of compounds pre-prepared by dilution, and the mixture was left to stand at room temperature for 5 min. Each concentration was performed in duplicate. The corresponding substrate and ATP were added and the mixture was reacted at room temperature for 120 minutes (with negative and positive controls). After completion of the reaction, PT66 detection antibody was added. The mixture was incubated at room temperature for 60 minutes, and then detected by Envision.

Data Calculation: the reading of well and inhibition rate were calculated using Excel, wherein reading of well = 10000*(well EU665 value)/(well EU615 value), and inhibition rate = [(reading of positive control well - reading of assay well) / (reading of positive control well - reading of negative control well)] * 100%. Compound concentrations and corresponding inhibition rates were input into GraphPad Prism for processing to calculate IC₅₀ values.

The results of the assay of the inhibitory activity of the compounds of the present application against EGFR^{WT} and HER2 tyrosine kinase are listed in Table 1, wherein A indicates that the IC₅₀ is less than or equal to 10 nM, B indicates that the IC₅₀ is greater than 10 nM and less than or equal to 100 nM, C indicates that the IC₅₀ is greater than 100 nM and less than or equal to 1000 nM, and NT indicates that there is no relevant result.

**Table 1. Results of the assay of the inhibitory activity of the compounds of the present disclosure against EGFR and HER2 kinases**

| Example No. | HER2 IC₅₀ nM | EGFR^{WT} IC₅₀ nM | Example No. | HER2 IC₅₀ | EGFR^{WT} IC₅₀ nM |
|---|---|---|---|---|---|
| 1 | A | A | 23 | A | C |
| 2 | A | A | 24 | A | C |
| 3 | A | A | 25 | A | C |
| 4 | A | A | 26 | A | B |
| 5 | A | B | 27 | A | B |
| 6 | A | A | 28 | A | A |
| 7 | A | A | 29 | A | A |
| 8 | A | A | 30 | A | A |
| 9 | A | A | 31 | A | A |
| 10 | A | A | 32 | A | C |
| 11 | A | A | 33 | A | A |
| 12 | A | A | 34 | A | A |
| 13 | A | A | 35 | A | B |
| 14 | A | A | 36 | A | A |
| 15 | A | A | 37 | A | B |
| 16 | A | A | 38 | A | A |
| 17 | A | A | 39 | A | A |
| 18 | A | A | 40 | A | A |
| 19 | A | A | 41 | A | NT |
| 20 | A | A | 42 | A | C |
| 21 | A | A | 43 | A | NT |
| 22 | A | A | 44 | A | NT |

As can be seen from the results in Table 1 above, all the compounds of the present application can inhibit both HER2 and EGFR kinases. Especially for HER2 kinase, the compounds of the present application show very excellent inhibitory activity as a whole, and can be used for the treatment of tumors and other diseases mediated by HER2 kinase.

### Assay example 2. Assay of inhibition of cell proliferation by small molecule compounds

The present application examined the in vitro anti-proliferative activity of the compounds of the present disclosure against in vitro cultured BT474, NCI-N87, Ba/F3-EGFRVIII and Ba/F3 HER2 A775_G776insYVMA and Ba/F3 EGFR D770_N771insSVD cell lines by CTG method.

Reagents and consumables: RPMI1640 (ThermoFisher, Cat. No. C11875500BT); fetal bovine serum (Hyclone, Cat. No. SV30087.03); 0.25% trypsin-EDTA (ThermoFisher, Cat. No. 25200-072); penicillin-streptomycin (Hyclone, Cat. No. SV30010); DMSO (Amresco, Cat. No. 0231-500ML); CTG assay kit (Promega, Cat. No. G924C); 96-well plate (Corning, Cat. No. 3603); multifunctional plate reader (Perkin Elmer, Cat. No. Envision)

Cell lines: BT474 (from the cell bank of Chinese Academy of Sciences), NCI-N87 (from ATCC), Ba/F3-EGFR-VIII, Ba/F3 HER2 A775_G776insYVMA and Ba/F3 EGFR D770_N771insSVD (all from KYinno Biotechnology (Beijing) Co., Ltd.); all of the above cells were cultured with RPMI1640 medium containing 10% fetal bovine serum, 100 U/mL penicillin, and 100 µg/mL streptomycin in the culture process.

### Specific assay methods:

1. The assay compound was dissolved with DMSO to form a stock solution and a gradient dilution was performed, followed by dilution with the corresponding medium to obtain a solution with 5-fold working concentration.
2. Cells in logarithmic growth phase were adjusted to specific cell densities by dilution with culture medium, and 80 µL of cell suspension was added to a 96-well plate, such that plating densities of cells of BT474, NCI-N87, Ba/F3-EGFR-VIII, Ba/F3 HER2 A775_G776insYVMA and Ba/F3 EGFR D770_N771insSVD were all 3000 cells/well. The cells of Ba/F3-EGFR-VIII, Ba/F3 HER2 A775_G776insYVMA and Ba/F3 EGFR D770_N771insSVD were used directly in the next step (treatment with compound), whereas the BT474 and NCI-N87 needed to be cultured overnight in a 37°C, 5% carbon dioxide gas incubator to adhere to the wall, and then treated with compounds.
3. 20 µL of compound solution was added to each well of the 96-well plate that had been inoculated with cells. The highest concentration of the assayed compound was 10 µM, 4-fold serial dilution, a total of 9 concentrations, and duplicate wells. A control group without compound was also set up.
4. The cells were cultured for another 72 hours, and then cell viability was detected by CTG assay kit. Signal values were read with a multifunctional plate reader (Perkin Elmer). GraphPad Prism software was used to create a dose-response curve and IC₅₀ was calculated.

The results of the assay of the anti-proliferative activity of the representative compounds of the present disclosure against cells of BT474, NCI-N87, Ba/F3-EGFR-VIII, Ba/F3 HER2 A775_G776insYVMA and Ba/F3 EGFR D770_N771insSVD are listed in Table 2, wherein A indicates that the IC₅₀ is less than or equal to 1 nM, B indicates that the IC₅₀ is greater than 1 nM and less than or equal to 5 nM, C indicates that the IC₅₀ is greater than 5 nM and less than or equal to 10 nM, D indicates that the IC₅₀ is greater than 10 nM and less than or equal to 100 nM, and NT indicates that there is no relevant result.

**Table 2. Results of the assay of the anti-proliferative activity of the representative compounds of the present disclosure against the cells of BT474, NCI-N87, Ba/F3-EGFR-VIII, Ba/F3 HER2 A775_G776insYVMA and Ba/F3 EGFR D770_N771insSVD.**

| Example | hBT 474 | hNCI-N87 | Ba/F3 HER2 A775-G776insYVMA | Ba/F3 EGFR D770_N771insS VD | Ba/F3 EGFRvIII |
|---|---|---|---|---|---|
| No. | IC₅₀ | IC₅₀ | IC₅₀ | IC₅₀ | IC₅₀ |
| 1 | NT | B | B | NT | B |
| 2 | NT | B | B | D | NT |
| 3 | NT | NT | B | NT | NT |
| 4 | C | A | A | D | NT |
| 5 | D | B | A | NT | NT |
| 6 | NT | NT | B | C | NT |
| 7 | NT | NT | B | NT | NT |
| 8 | NT | NT | B | D | NT |
| 9 | NT | NT | B | D | NT |
| 10 | NT | NT | B | D | NT |
| 11 | NT | NT | B | NT | NT |
| 12 | NT | NT | B | NT | NT |
| 13 | NT | NT | B | NT | NT |
| 14 | NT | NT | B | NT | NT |
| 15 | NT | NT | B | D | NT |
| 16 | NT | NT | B | NT | NT |
| 17 | NT | NT | B | D | NT |
| 18 | NT | NT | B | NT | NT |
| 19 | NT | NT | B | NT | NT |
| 20 | NT | NT | B | NT | NT |
| 21 | NT | NT | B | NT | NT |
| 22 | NT | NT | B | NT | NT |
| 23 | NT | NT | B | NT | NT |
| 24 | NT | NT | B | NT | NT |
| 25 | NT | NT | B | NT | NT |
| 26 | NT | NT | B | D | NT |
| 27 | NT | NT | B | NT | NT |
| 28 | NT | NT | B | D | NT |
| 29 | NT | NT | A | D | NT |
| 30 | NT | NT | B | NT | NT |
| 31 | NT | NT | B | NT | NT |
| 32 | NT | NT | B | NT | NT |
| 33 | NT | NT | A | NT | NT |
| 34 | NT | NT | B | NT | NT |
| 35 | NT | NT | B | NT | NT |
| 36 | NT | NT | B | NT | NT |
| 37 | NT | NT | B | NT | NT |
| 38 | NT | NT | B | NT | NT |
| 39 | NT | NT | B | NT | NT |
| 40 | NT | NT | B | NT | NT |
| 41 | NT | NT | B | NT | NT |
| Nera | | | D | | |
| Pyro | | | D | | |

The results in Table 2 show that the compounds of the present application all exhibit excellent anti-tumor proliferation activity against various cell lines assayed above. Especially for Ba/F3 HER2 A775-G776sYAMA cells, the compounds of the present application exhibit excellent activity that is several times or even tens of times better than that of the existing drugs neratinib and pyrotinib.

### Assay example 3. Assay of pharmacokinetics of small molecule compounds

In this assay, the pharmacokinetic characteristics of the compounds of the present application were assayed after administration of some of the compounds of the present application to SD rats by single oral administration and intravenous injection.

### (i) Reagents, instruments and animals used

**Table 3: Assay reagents**

| Reagent | Supplier | Cat. No. | Lot No. |
|---|---|---|---|
| DMSO | Innochem | D3851 | INAH03010 |
| Kolliphor^{®}HS 15 (Solutol) | SIGMA | 42966 | BCBZ6212 |
| Acetonitrile | Fisher | 75-05-8 | 186342 |
| Formic acid | DIKMA | 64-18-6 | 2486917 |
| Ammonium acetate | SIGMA | 431311 | MKCD5084 |
| Ultra-pure water | Manufactured | - | - |
| | in-house | | |

**Table 4. Assay instruments**

| Instrument | Brand | Model |
|---|---|---|
| Liquid chromatography-mass spectrometry (LC-MS) | AB Sciex | 5500 Q-trap |
| High speed refrigerated centrifuge | Thermo | ST 40R |
| Balance (centi milli-) | Mettler | XPE205 |
| Single well pipette | Eppendorf | Research plus series |
| Electric 12-hole pipette | Thermo | Novus series |

**Table 5: Rats used in the assay**

| | |
|---|---|
| Animal & strain: | SD Rat (Male) |
| Animal grade: | SPF grade |
| Animal source: | Beijing HFK BioSCIENCE Co., Ltd |
| Weight range, age: | 180 g-350 g on the first day of administration, 8 weeks old (based on body weight) |

### (ii) Preparation of sample formulation

1. Intravenous (IV) injection group: an appropriate amount of the assay compound was weighed, and completely dissolved in an appropriate volume of vehicle (DMSO/Solutol/H₂O = 5/10/85 v/v/v (the vehicle for Example 9 was DMSO/Solutol/H₂O = 5/10/85 v/v/v with 2 times mole of maleic acid)). The mixture was stirred, vortexed and/or sonicated. Once a solution was obtained, the vehicle was gradually added to a final volume to reach the target concentration. The solution was vortexed, and sonicated to obtain a homogeneous solution. The solution was filtered through a 0.22 µm PVDF membrane.

2. Oral (PO) group: an appropriate amount of the assay compound was weighed, and completely dissolved in an appropriate volume of vehicle (DMSO/Solutol/H₂O = 5/10/85 v/v/v (the vehicle for Example 9 was DMSO/Solutol/H₂O = 5/10/85 v/v/v with 2 times mole of maleic acid)). The mixture was stirred, vortexed and/or sonicated. Once a solution was obtained, the vehicle was gradually added to a final volume to reach the target concentration. The solution was vortexed, and sonicated to obtain a homogeneous solution.

### (iii) Administration and sampling of rats

The animals were randomly divided into groups according to their body weights, and the weights of the animals in each group were comparable after grouping (not exceeding ±20% of the average body weight). Meanwhile, the IV group was not fasted and the PO group was fasted overnight (>12 hrs) and given food 2 hrs after drug administration. All animals had free access to water. The dosing regimen and pharmacokinetic sampling regimen are given in Table 6 and Table 7 below, respectively.

**Table 6. Dosing regimen**

| Grou p | Method of administratio | Numbe r of animal s | Sample | Dose of administratio n (mg/kg) | Volume of administratio n (mL/kg) | Concentratio n of formulation (mg/mL) |
|---|---|---|---|---|---|---|
| 1 | IV | 3 | Whole blood | 3 | 3 | 1 |
| 2 | PO | 3 | Whole Blood | 10 | 10 | 1 |
| 3 | PO | 3 | Whole blood/brai n | 10 | 10 | 1 |

**Table 7. Pharmacokinetic sampling regimen**

| Group | Method of administration | Sampling | Anticoagulant | Pharmacokinetic time point |
|---|---|---|---|---|
| 1 | IV | Jugular Vein Cannulation (JVC) | K₂EDTA | Whole blood: pre-dose, 5, 15, 30 minutes, 1, 2, 4, 7 and 24 hours post-dose |
| 2 | PO | Jugular Vein Cannulation (JVC) | K₂EDTA | Whole blood: pre-dose, 15, 30 minutes, 1, 2, 4, 7 and 24 hours post-dose |
| 3 | PO | Blood was collected from the heart, the animal was anesthetized, and brain tissue was collected. | K₂EDTA | Whole blood: 2 hours |
| | | | | Brain tissue: 2 hours |

Rats were administered according to the above regimen, and blood and brain tissue samples were collected and processed at predetermined time points (collection and processing were performed according to conventional methods in the art).

### (iv) Analysis of samples

Six times volume of acetonitrile was added to the whole blood samples, and the mixture was vortexed for 1 min and then centrifuged at 4°C, 4500 rpm for 15 min. The supernatant was diluted 2-fold with ultra-pure water, and the samples were analyzed by LC/MS.

Brain tissues were weighed and homogenized by adding 4-fold homogenizing solution (acetonitrile/water=1/1 v/v). Six times volume of acetonitrile was added respectively to the brain tissue homogenate, and the mixture was vortexed for 1 min and then centrifuged at 4°C, 4500 rpm for 15 min. The supernatant was diluted 2-fold with ultra-pure water, and the samples were analyzed by LC/MS.

### (v) Data analysis:

Pharmacokinetic parameters was calculated using WinNonlin software. The following pharmacokinetic parameters was calculated from the plasma drug concentration-time data: CL (clearance); V_{d} (apparent volume of distribution); T_{1/2} (elimination half-life time); Cₘₐₓ (peak concentration); Tₘₐₓ (time to peak); AUC (area under plasma drug concentration-time curve); MRT (mean retention time); F% (bioavailability).

The assay results are shown in Tables 8-11 below, which give the values of each pharmacokinetic parameter of pyrotinib, neratinib, control compounds 1 and 2, and some of the compounds of the present application under both intravenous injection and oral administration, respectively. The results show that the control compounds 1 and 2, when administrated orally, are hardly absorbed and cannot be measured to obtain effective pharmacokinetic data; whereas all the compounds of the present application exhibit excellent pharmacokinetic properties and are better than pyrotinib and neratinib in view of bioavailability.

**Table 8: Pharmacogenetic parameters of some of the assay compounds in rats (IV 3 mg/kg)**

| Parameter | PYROTINIB | NERATINIB | Control compound 1 | Control compound 2 | Example 4 |
|---|---|---|---|---|---|
| T_{1/2} (hr) | 3.45 ± 0.24 | 4.38 ± 0.55 | 4.91 ± 0.28 | 6.50 ± 0.78 | 4.50 ± 0.21 |
| AUC₀₋ₜ (hr*ng/mL) | 8310 ± 892 | 2388 ± 267 | 678 ± 74 | 565 ± 40 | 968 ± 156 |
| AUC_{inf} (hr*ng/mL) | 8340 ± 890 | 2422 ± 252 | 693 ± 72 | 595 ± 37 | 979 ± 161 |
| Cl_{_obs} (mL/min/kg) | 6.04 ± 0.63 | 20.8 ± 2.0 | 72.7 ± 7.1 | 84.3 ± 5.1 | 52.1 ± 9.3 |
| V_{d} (L/kg) | 1.81 ± 0.26 | 7.94 ± 1.71 | 31.0 ± 4.6 | 47.5 ± 7.5 | 20.2 ± 2.6 |

**Table 9. Pharmacogenetic parameters of some of the assay compounds in rats (IV 3 mg/kg)**

| Parameter | Example 9 | Example 13 | Example 24 | Example 29 |
|---|---|---|---|---|
| T_{1/2} (hr) | 3.94 ± 0.18 | 4.12 ± 0.22 | 5.01 ± 0.45 | 4.86 ± 0.23 |
| AUC₀₋ₜ (hr*ng/mL) | 949 ± 128 | 953 ± 105 | 1513 ± 26 | 914 ± 129 |
| AUC_{inf} (hr*ng/mL) | 955 ± 130 | 964 ± 104 | 1553 ± 23 | 938 ± 134 |
| Cl_{_obs} (mL/min/kg) | 53.0 ± 6.7 | 52.3 ± 5.7 | 32.2 ± 0.5 | 54.0 ± 7.4 |
| V_{d} (L/kg) | 18.0 ± 2.3 | 18.7 ± 2.9 | 14.0 ± 1.3 | 22.7 ± 2.4 |

**Table 10. Pharmacogenetic parameters of some of the assay compounds in rats (PO 10 mg/kg)**

| Parameter | PYROTINIB | NERATINIB | Control compound 1 | Control compound 2* | Example 4 |
|---|---|---|---|---|---|
| Tₘₐₓ (hr) | 4.33 ± 2.52 | 2.67 ± 1.15 | NA | 0.25 ± NA | 5.33 ± 2.89 |
| Cₘₐₓ (ng/mL) | 246 ± 76 | 201 ± 66 | NA | 6.72 ± NA | 65.7 ± 19.9 |
| T_{1/2} (hr) | 3.60 ± 0.37 | 2.97 ± 0.27 | NA | 2.39 ± NA | 3.69 ± 0.31 |
| AUC₀₋ₜ (hr*ng/mL) | 2789 ± 1109 | 1466 ± 637 | NA | 18.7 ± NA | 888 ± 221 |
| AUC_{inf} (hr*ng/mL) | 2813 ± 1110 | 1471 ± 638 | NA | 23.8 ± NA | 902 ± 223 |
| F% | 10.1 ± 4.0 | 18.2 ± 7.89 | NA | 0.992 ± NA | 27.6 ± 6.8 |

| | | | | | |
|---|---|---|---|---|---|
| *: Only one of the 3 parallel assays had data, and the SD value could not be counted. | | | | | |

**Table 11. Pharmacogenetic parameters of some of the assay compounds in rats (PO 10 mg/kg)**

| Parameter | Example 9 | Example 13 | Example 24 | Example 29 |
|---|---|---|---|---|
| Tₘₐₓ (hr) | 5.00 ± 3.46 | 2.0 ± 0.0 | 5.33 ± 2.89 | 3.33 ± 3.21 |
| Cmax (ng/mL) | 80.3 ± 30.5 | 53.7 ± 12.4 | 86.3 ± 32.7 | 95.3 ± 16.5 |
| T_{1/2} (hr) | 4.13 ± 0.57 | 5.42 ± 0.34 | 7.81 ± 3.71 | 4.63 ± 0.26 |
| AUC₀₋ₜ (hr*ng/mL) | 1098 ± 406 | 719 ± 233 | 1264 ± 561 | 1100 ± 107 |
| AUC_{inf} (hr*ng/mL) | 1112 ± 403 | 750 ± 238 | 1474 ± 655 | 1134 ± 106 |
| F% | 34.9 ± 12.6 | 23.3 ± 7.4 | 25.0 ± 11.2 | 36.3 ± 3.4 |

| | | | | |
|---|---|---|---|---|
| NA: Exposure was too low to be counted. | | | | |

On the other hand, as shown in Table 11-1 below, the compounds of the present application exhibit excellent ability to penetrate the blood-brain barrier, and the brain/blood ratio is significantly increased compared with pyrotinib and neratinib. This also indicates that the compounds of the present application not only have excellent inhibitory activities on EGFR and HER2 kinases, but also have excellent ability to penetrate the blood-brain barrier, so that the compounds of the present application are expected to be applied to EGFR and/or HER2 kinase-mediated related tumors, especially brain metastases.

**Table 11-1. Concentrations and ratios of the assay compounds of this application in brain tissue and whole blood (PO 10 mg/kg, sampling time, 2h administration)**

| **Example** | **Blood drug concentration (ng/mL)** | **Brain tissue concentration (ng/g)** | **Brain tissue/blood ratio** |
|---|---|---|---|
| Pyratinib | 884 | 25.8 | 0.0291 |
| neratinib | 440 | 14.4 | 0.0366 |
| Control compound 2 | 1.14 | NA | NA |
| Example 13 | 273 | 127 | 0.465 |
| Example 29 | 121 | 43.5 | 0.360 |

| | | | |
|---|---|---|---|
| NA: Exposure was too low to be counted. | | | |

**Assay example 4.** Assay of efficacy of small molecular compounds in mice bearing tumors In this assay, some compounds of the application were orally administered to a nude mouse tumor-bearing model of Ba/F3 ERBB2 A775_G776insYVMA to study the effect of the compounds of the application on tumor growth.

**Table 12. Assay instruments:**

| **Instrument** | **Brand** | **Model** |
|---|---|---|
| Electronic balance (mouse) | AOHAOSI Instruments (Changzhou) Co. | Scout SE-SE202F |
| Single channel pipette | Mettler Toled | L-200XLS+ |
| Centrifuge | eppendort | Cantnfuge 5424 R |
| Clean bench | ESCO | ACB-6A1 |
| Vortex mixer | North TZ-Biotech | HQ-60W |
| Transfer window | LingyunBoji (Beijing) Technology Co. | unknown |
| IVC independent ventilation animal cage | LingyunBoji (Beijing) Technology Co. | LYBJ-IVCS |
| Electronic balance (mouse) | Changshu Shuangjie Assay Instrument Factory | T1000 |

**Table 13 Animals and cells used in the assay:**

| Genus | Strain | Sex | Quantit y | Weeks of age | Weight | Source | Animal ID No. |
|---|---|---|---|---|---|---|---|
| Mice | Balb/c | Male | 55 | 6-8 weeks | 18-20g | Charles River | 110011211 |
| | nude | | | | | 100936675 | |
| Ba/F3 ERBB2 A775_G776insY VMA | | | | KYinno Biotechnology (Beijing) Co., Ltd. | | | |

**Reagents:** RPMI1640 (ThermoFisher, Cat. No. C11875500BT); fetal bovine serum (Hyclone, Cat. No. SV30087.03); 0.25% trypsin-EDTA (ThermoFisher, Cat. No. 25200072); penicillin-streptomycin (Hyclone, Cat. No. SV30010); DSMO (Life Science, Cat. No. 0231-500ML); Solutol (Sigma, 70142-34-6 -1kg)

Preparation of the assay compound: an appropriate weight of the compound to be assayed was weighed, and completely dissolved in an appropriate volume of veihcle (DMSO). The mixture was vortexed or sonicated. Then an appropriate amount of Solutol was added and mixed well. Finally, sterilized drinking water was added and the mixture was mixed by stirring or vortexing to obtain a homogeneous solution or suspension.

**Method:** All the assays were authorized and agreed by the Animal Welfare Committee. Ba/F3 ERBB2 A775_G776insYVMA in a logarithmic growth period was inoculated subcutaneously into the right back of immunodeficient nude mice (BALB/c nude, female, 6-7 weeks of age, body weight 18±2g) with 4×10⁶ cells/rat. After the tumor grew to 150-200 mm³, the animals were randomly divided into a treatment group and a control group. For the treatment group, a solution of each assay compound was given, and for the control group, a vehicle solution without the assay compound was given. Both of the treatment group and the control group were administered once a day for about 2 weeks. The dose of each assay article was 30 mg/kg (in terms of effective compound concentration), and the assay compounds were all prepared freshly for use. During the assay, the diameter of loaded tumors was measured twice a week, and the mice were weighed at the same time. The tumor volume (TV) was calculated by the formula: TV = 1/2 × a × b², where a and b denote length and width, respectively. Relative tumor volume (RTV) was calculated based on the measured results by the formula: RTV = Vt/V0, where V0 is the tumor volume measured at grouping and administration and Vt is the tumor volume at each measurement. The evaluation index of antitumor activity was the relative tumor proliferation rate T/C (%) calculated by the formula: T/C (%) = (TRTV / CRTV) × 100 %, TRTV: RTV of the administration group; CRTV: RTV of the control group. T and C denote the average tumor volume at a certain time point in the administration group and the control group, respectively.

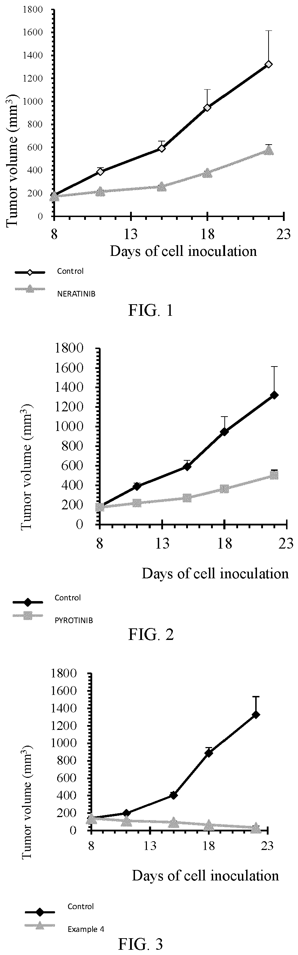

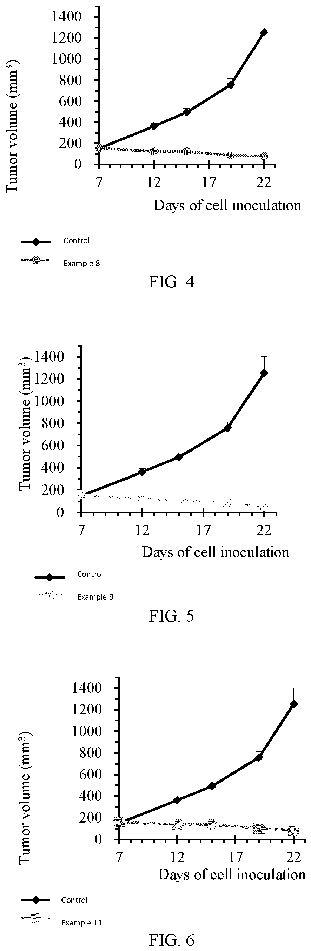

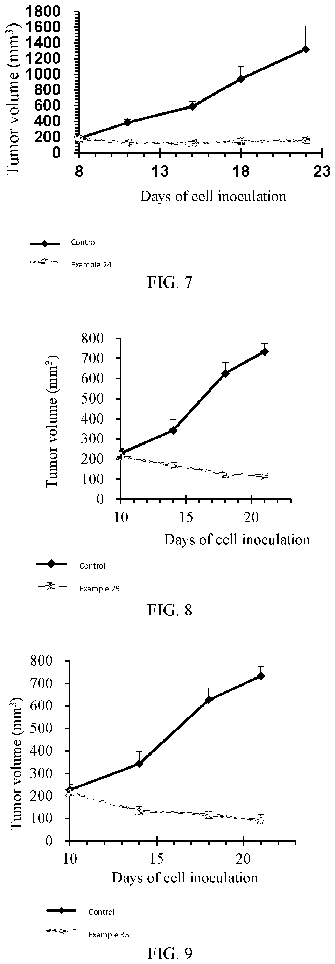

The assay results are shown in Table 14 below and FIGs. 1-9. As shown in the Table and Figures, Neratinib and Pyrotinib have certain tumor inhibitory effects at a dose of 30 mg/kg, with T/C values of 45.9% and 38.3%, respectively, at day 14. The compounds of Example 4, Example 8, Example 9, Example 11, Example 24, Example 29, and Example 33 of the present application have significant tumor inhibitory effects at a dose of 30 mg/kg, wherein the T/C values of the compounds of Example 4 and Example 24 are 2.6% and 12.4% at day 14, respectively, and the T/C values of the compounds of Example 8, Example 9, and Example 11 are 5.83%, 3.85%, and 5.71% at day 15, respectively, and the T/C values of the compounds of Example 29 and Example 33 are 18.7% and 12.4% at day 11, respectively. Combined with the above results of the assay of the proliferative activity of Ba/F3 HER2 A775-G776sYAMA cells, it can be seen that the compounds of the present application not only have anti-tumor cell proliferation activity in vitro against the exon 20 insertion mutation of HER2 (HER2 A775_G776insYVMA) that is superior to that of Pyrotinib and Neratinib, but also show far superior antitumor activity to Neratinib and Pyrotinib in nude mouse tumor-bearing model of Ba/F3 ERBB2 A775_G776insYVMA. All assayed compounds achieve significant tumor shrinkage, while tumors in the Neratinib and Pyrotinib groups still remain significant growth at the same dose (30 mg/kg).

**Table 14. T/C (%) of compounds of examples of the present application in the nude mouse tumor-bearing model of Ba/F3 ERBB2 A775_G776insYVMA**

| Group | Dosage, mode of administration | | T/C(%) |
|---|---|---|---|
| NERATINIB | 30mg/kg, QD×14 | PO | 45.9 |
| PYROTINIB | 30mg/kg, QD×14 | PO | 38.3 |
| Example 4 | 30mg/kg, QD×14 | PO | 2.6 |
| Example 8 | 30mg/kg, QD×15 | PO | 5.83 |
| Example 9 | 30mg/kg, QD×15 | PO | 3.85 |
| Example 11 | 30mg/kg, QD×15 | PO | 5.71 |
| Example 24 | 30mg/kg, QD×14 | PO | 12.4 |
| Example 29 | 30mg/kg, QD×11 | PO | 18.7 |
| Example 33 | 30mg/kg, QD×11 | PO | 12.4 |

| | | | |
|---|---|---|---|
| *QD denotes once-daily dosing | | | |

Taking all the assay results together, it can be found that the present application synthesizes a series of compounds by introducing a substituent at the 5-position of quinazoline and removing the substituent at the 7-position. The compounds of the present application exhibit good to excellent inhibitory activity against HER2 and EGFR kinases, as well as the proliferation of BT474, NCI-N87, Ba/F3-EGFR-VIII, Ba/F3 HER2 A775_G776insYVMA and Ba/F3 EGFR D770_N771insSVD cells. Especially for Ba/F3 HER2 A775_G776insYVMA cell model, the compounds of the present application all exhibit significantly better effects than that of Neratinib and Pyrotinib in the in vivo cell proliferation inhibition assay and the in vitro tumor-bearing model assay. Moreover, the compounds of the present application also exhibit excellent pharmacokinetic properties in the pharmacokinetic assay. The overall performance of the compounds of the present application is significantly better than that of Neratinib, Pyrotinib, and control compounds 1 and 2, and the compounds of the present application can be applied in the treatment of related diseases mediated by HER2, EGFR kinase or exon 20 mutation thereof or other mutation.

The above-mentioned embodiments are alternative embodiments of the present disclosure. It should be pointed out that for those skilled in the art, without departing from the principles of the present disclosure, several improvements and modifications can also be made to the embodiments of the present disclosure, and these improvements and modifications should also be regarded as within the protection scope of the present disclosure.

## Claims

1. A compound represented by formula (I), a stereoisomer thereof, a pharmaceutically acceptable salt thereof, or a deuterated derivative thereof, 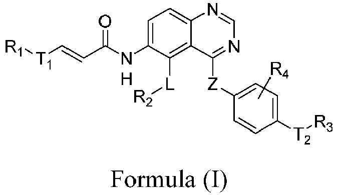 In formula (I),
Z is -NH- or -O-;
T₁ is -(CH₂)ₙ-, wherein n is an integer from 0 to 3;
R₁ is hydrogen, hydroxy, 4- to 7-membered heteroalicyclic group or -NR^{a}R^{b},
R^{a} and R^{b} are each independently hydrogen, C₁-C₆ alkyl, C₃-C₆ cycloalkyl, hydroxy-substituted C₁-C₆ alkyl, C₁-C₃ alkoxy-substituted C₁-C₆ alkyl, or C₃-C₆ cycloalkyl-substituted C₁-C₆ alkyl; the 4- to 7-membered heteroalicyclic group is a heteroalicyclic group containing 1-2 heteroatoms selected from N, O and S, wherein the heteroalicyclic group is unsubstituted or substituted with one or two of C₁-C₃ alkyl, C₁-C₄ alkylacyl, hydroxy, cyano, aminoacyl, mono- or di-substituted C₁-C₃ aminoacyl, C₁-C₃ alkoxy-substituted C₁-C₃ alkyl, and hydroxy-substituted C₁-C₃ alkyl;
L is -O-, -S- or -NH-;
R₂ is C₁-C₆ alkyl, C₁-C₆ haloalkyl, hydroxy-substituted C₁-C₆ alkyl, C₁-C₃ alkoxy-substituted C₁-C₆ alkyl, or -(CH₂)m-R₅,
R₅ is C₃-C₆ cycloalkyl or 4- to 6-membered heteroalicyclic group, and m is an integer from 0 to 3,
the 4- to 6-membered heteroalicyclic group is a heteroalicyclic group containing 1-2 heteroatoms selected from N, O and S;
T₂ is -M-(CH₂)p-, wherein M is O, S or NH, and p is an integer from 0 to 2,
R₃ is an aryl or heteroaryl group selected from phenyl, pyridyl, pyrimidinyl, and pyrrolyl, and the aryl or heteroaryl is unsubstituted or substituted with 1 to 3 substituents selected from halogen, cyano, hydroxy, C₁-C₃ alkyl, C₁-C₃ alkoxy, C₁-C₃ haloalkyl, C₃-C₄ cycloalkyl, C₂-C₃ alkynyl, C₂-C₃ alkenyl and -NR'R",
R', R" are each independently H or C₁-C₃ alkyl;
R₄ is hydrogen, halogen, C₁-C₃ alkyl or C₁-C₃ alkoxy.

2. The compound according to claim 1, a stereoisomer thereof, a pharmaceutically acceptable salt thereof, or a deuterated derivative thereof, wherein Z is -NH-.

3. The compound according to claim 1 or 2, a stereoisomer thereof, a pharmaceutically acceptable salt thereof, or a deuterated derivative thereof, wherein T₁ is -(CH₂)ₙ-, wherein n is an integer from 0 to 2,
R₁ is hydrogen, 4- to 6-membered heteroalicyclic group or -NR^{a}R^{b},
R^{a} and R^{b} are each independently hydrogen, C₁-C₃ alkyl, C₃-C₄ cycloalkyl, hydroxy-substituted C₁-C₃ alkyl, C₁-C₃ alkoxy-substituted C₁-C₃ alkyl, or C₃-C₆ cycloalkyl-substituted C₁-C₃ alkyl; the 4- to 6-membered heteroalicyclic group is pyrrolidinyl, piperidinyl, piperazinyl, morpholinyl, tetrahydrofuranyl, tetrahydropyranyl, or thiomorpholinyl, and the above groups are unsubstituted or substituted with one or two of methyl, ethyl, propyl, isopropyl, aldehyde group, acetyl, propionyl, hydroxy, cyano, aminoacyl, methoxymethyl, methoxyethyl, methoxypropyl, ethoxymethyl, ethoxyethyl, ethoxypropyl, propoxymethyl, propoxyethyl, propoxypropyl, isopropoxymethyl, isopropoxyethyl, isopropoxypropyl, hydroxymethyl, hydroxyethyl and hydroxypropyl;
or, T₁ is -(CH₂)ₙ-, wherein n is 0 or 1;
R₁ is dimethylamino, diethylamino, dipropylamino, diisopropylamino, methylethylamino, methylpropylamino, ethylpropylamino, methylcyclopropylamino, ethylcyclopropylamino, propylcyclopropylamino, isopropylcyclopropylamino, methylcyclobutylamino, ethylcyclobutylamino, propylcyclobutylamino, isopropylcyclobutylamino, pyrrolidin-1-yl, pyrrolidin-2-yl, 1-methylpyrrolidin-2-yl, 1-ethylpyrrolidin-2-yl, 1-propylpyrrolidin-2-yl, 1-isopropylpyrrolidin-2-yl, 1-(2-methoxyethyl)pyrrolidin-2-yl, 1-(2-methoxypropyl)pyrrolidin-2-yl, 1-(2-ethoxyethyl)pyrrolidin-2-yl, 1-(2-ethoxypropyl)pyrrolidin-2-yl, 1-(2-hydroxymethyl)pyrrolidin-2-yl, 1-(2-hydroxyethyl)pyrrolidin-2-yl, 1-(2-hydroxypropyl)pyrrolidin-2-yl, piperidin-1-yl, 1-methylpiperazin-4-yl, 1-ethylpiperazin-4-yl, 1-propylpiperazin-4-yl, 1-isopropylpiperazin-4-yl, 1-(2-hydroxyethyl)piperazin-4-yl, morpholinyl, tetrahydrofuran-2-yl, tetrahydrofuran-3-yl, tetrahydropyran-2-yl, tetrahydropyran-3-yl, or thiomorpholinyl.

4. The compound according to claim 1 or 2, a stereoisomer thereof, a pharmaceutically acceptable salt thereof, or a deuterated derivative thereof, wherein L is -O- or -NH-;
R₂ is C₁-C₄ alkyl, C₁-C₄ haloalkyl, hydroxy-substituted C₁-C₄ alkyl, C₁-C₃ alkoxy-substituted C₁-C₄ alkyl or -(CH₂)m-R₅,
R₅ is C₃-C₆ cycloalkyl or 4- to 6-membered heteroalicyclic group, and m is 0, 1 or 2,
the 4- to 6-membered heteroalicyclic group is a heteroalicyclic group containing 1-2 heteroatoms selected from N, O and S;
or, L is -O-;
R₂ is methyl, ethyl, propyl, isopropyl, butyl, isobutyl, tert-butyl, fluoromethyl, 2-fluoroethyl, 3-fluoropropyl, trifluoromethyl, difluoromethyl, 3,3,3-trifluoropropyl, 3,3,3-trifluoroethyl, 2,2-difluoroethyl, 3,3-difluoropropyl, hydroxymethyl, hydroxyethyl, hydroxypropyl, hydroxybutyl, 2-hydroxy-2-methylpropyl, 3-hydroxy-3-methylbutyl, methoxymethyl, methoxyethyl, methoxypropyl, methoxybutyl, ethoxymethyl, ethoxyethyl, ethoxypropyl, ethoxybutyl, cyclopropyl, cyclopropylmethyl, cyclobutyl, tetrahydrofuran-2-yl, or tetrahydrofuran-3 -yl.

5. The compound according to claim 1 or 2, a stereoisomer thereof, a pharmaceutically acceptable salt thereof, or a deuterated derivative thereof, wherein T₂ is -O-(CH₂)p-, wherein p is 0 or 1;
R₃ is phenyl, pyridyl, or pyrimidyl, and the phenyl, pyridyl, and pyrimidyl are unsubstituted or substituted with 1 to 2 substituents selected from fluorine, chlorine, methyl, methoxy, ethyl, ethoxy, propyl, propoxy, isopropyl, isopropoxy, cyano, hydroxy, fluoromethyl, 2-fluoroethyl, trifluoromethyl, difluoromethyl, 3,3,3-trifluoroethyl, and 2,2-difluoroethyl;
or, T₂ is -O-(CH₂)p-, wherein p is 1;
R₃ is phenyl, 2-fluorophenyl, 3-fluorophenyl, 4-fluorophenyl, 2-methylphenyl, 3-methylphenyl, 4-methylphenyl, 2-methoxyphenyl, 3-methoxyphenyl, 4-methoxyphenyl, pyridin-2-yl, pyridin-3-yl, pyridin-4-yl, 6-methylpyridin-2-yl, 6-methoxypyridin-2-yl, 5-methylpyridin-2-yl, 5-methoxypyridin-2-yl, 4-methylpyridin-2-yl, 4-methoxypyridin-2-yl, 3-methylpyridin-2-yl, 3-methoxypyridin-2-yl, 6-fluoropyridin-2-yl, or 6-chloropyridin-2-yl.

6. The compound according to claim 1 or 2, a stereoisomer thereof, a pharmaceutically acceptable salt thereof, or a deuterated derivative thereof, wherein R₄ is hydrogen, fluorine, chlorine, methyl, or methoxy;
or, R₄ is hydrogen, fluorine, or chlorine.

7. The compound according to claim 1, a stereoisomer thereof, a pharmaceutically acceptable salt thereof, or a deuterated derivative thereof, wherein the compound has the following structural formula, 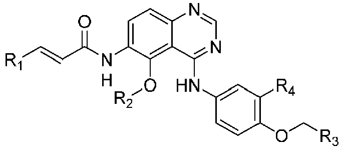
R₁ is pyrrolidinyl, piperidinyl, piperazinyl, morpholinyl, tetrahydrofuranyl, tetrahydropyranyl, or thiomorpholinyl, and the above groups are unsubstituted or substituted with one or two of methyl, ethyl, propyl, isopropyl, aldehyde group, acetyl, propionyl, hydroxy, cyano, aminoacyl, methoxymethyl, methoxyethyl, methoxypropyl, ethoxymethyl, ethoxyethyl, ethoxypropyl, propoxymethyl, propoxyethyl, propoxypropyl, isopropoxymethyl, isopropoxyethyl, isopropoxypropyl, hydroxymethyl, hydroxyethyl and hydroxypropyl;
R₂ is C₁-C₄ alkyl, C₁-C₄ haloalkyl, hydroxy-substituted C₁-C₄ alkyl, C₁-C₃ alkoxy-substituted C₁-C₄ alkyl or -(CH₂)m-R₅,
R₅ is C₃-C₆ cycloalkyl or 4- to 6-membered heteroalicyclic group, and m is 0, 1 or 2,
the 4- to 6-membered heteroalicyclic group is a heteroalicyclic group containing 1-2 heteroatoms selected from N, O and S;
R₃ is phenyl, pyridyl, or pyrimidyl, and the phenyl, pyridyl, and pyrimidyl are unsubstituted or substituted with 1 to 2 substituents selected from fluorine, chlorine, methyl, methoxy, ethyl, ethoxy, propyl, propoxy, isopropyl, isopropoxy, cyano, hydroxy, fluoromethyl, 2-fluoroethyl, trifluoromethyl, difluoromethyl, 3,3,3-trifluoroethyl, and 2,2-difluoroethyl;
R₄ is hydrogen, fluorine, chlorine, methyl, or methoxy.

8. The compound according to claim 7, a stereoisomer thereof, a pharmaceutically acceptable salt thereof, or a deuterated derivative thereof, wherein
R₁ is pyrrolidin-1-yl, pyrrolidin-2-yl, 1-methylpyrrolidin-2-yl, 1-ethylpyrrolidin-2-yl, 1-propylpyrrolidin-2-yl, 1-isopropylpyrrolidin-2-yl, 1-(2-methoxyethyl)pyrrolidin-2-yl, 1-(2-methoxypropyl)pyrrolidin-2-yl, 1-(2-ethoxyethyl)pyrrolidin-2-yl, 1-(2-ethoxypropyl)pyrrolidin-2-yl, 1-(2-hydroxymethyl)pyrrolidin-2-yl, 1-(2-hydroxyethyl)pyrrolidin-2-yl, or 1-(2-hydroxypropyl)pyrrolidin-2-yl;
R₂ is methyl, ethyl, propyl, isopropyl, butyl, isobutyl, tert-butyl, fluoromethyl, 2-fluoroethyl, 3-fluoropropyl, trifluoromethyl, difluoromethyl, 3,3,3-trifluoropropyl, 3,3,3-trifluoroethyl, 2,2-difluoroethyl, 3,3-difluoropropyl, hydroxymethyl, hydroxyethyl, hydroxypropyl, hydroxybutyl, 2-hydroxy-2-methylpropyl, 3-hydroxy-3-methylbutyl, methoxymethyl, methoxyethyl, methoxypropyl, methoxybutyl, ethoxymethyl, ethoxyethyl, ethoxypropyl, or ethoxybutyl;
R₃ is phenyl, 2-fluorophenyl, 3-fluorophenyl, 4-fluorophenyl, 2-methylphenyl, 3-methylphenyl, 4-methylphenyl, 2-methoxyphenyl, 3-methoxyphenyl, 4-methoxyphenyl, pyridin-2-yl, pyridin-3-yl, pyridin-4-yl, 6-methylpyridin-2-yl, 6-methoxypyridin-2-yl, 5-methylpyridin-2-yl, 5-methoxypyridin-2-yl, 4-methylpyridin-2-yl, 4-methoxypyridin-2-yl, 3-methylpyridin-2-yl, 3-methoxypyridin-2-yl, 6-fluoropyridin-2-yl, or 6-chloropyridin-2-yl;
R₄ is fluorine or chlorine;
or, R₁ is pyrrolidin-1-yl, pyrrolidin-2-yl, 1-methylpyrrolidin-2-yl, 1-ethylpyrrolidin-2-yl, 1-propylpyrrolidin-2-yl, 1-isopropylpyrrolidin-2-yl, 1-(2-methoxyethyl)pyrrolidin-2-yl, 1-(2-methoxypropyl)pyrrolidin-2-yl, 1-(2-ethoxyethyl)pyrrolidin-2-yl, 1-(2-ethoxypropyl)pyrrolidin-2-yl, 1-(2-hydroxymethyl)pyrrolidin-2-yl, 1-(2-hydroxyethyl)pyrrolidin-2-yl, or 1-(2-hydroxypropyl)pyrrolidin-2-yl;
R₂ is methyl, ethyl, propyl, isopropyl, butyl, isobutyl, tert-butyl, fluoromethyl, 2-fluoroethyl, 3-fluoropropyl, trifluoromethyl, difluoromethyl, 3,3,3-trifluoropropyl, 3,3,3-trifluoroethyl, 2,2-difluoroethyl, 3,3-difluoropropyl, hydroxymethyl, hydroxyethyl, hydroxypropyl, hydroxybutyl, 2-hydroxy-2-methylpropyl, 3-hydroxy-3-methylbutyl, methoxymethyl, methoxyethyl, methoxypropyl, methoxybutyl, ethoxymethyl, ethoxyethyl, ethoxypropyl, or ethoxybutyl;
R₃ is pyridin-2-yl;
R₄ is chlorine.

9. The compound according to claim 8, a stereoisomer thereof, a pharmaceutically acceptable salt thereof, or a deuterated derivative thereof, wherein
R₁ is 1-methylpyrrolidin-2-yl, 1-ethylpyrrolidin-2-yl, or 1-isopropylpyrrolidin-2-yl;
R₂ is methyl, ethyl, propyl, methoxyethyl, methoxypropyl, or 2,2-difluoroethyl;
R₃ is pyridin-2-yl;
R₄ is chlorine.

10. The compound according to claim 1, a stereoisomer thereof, a pharmaceutically acceptable salt thereof, or a deuterated derivative thereof, wherein the compound is selected from:

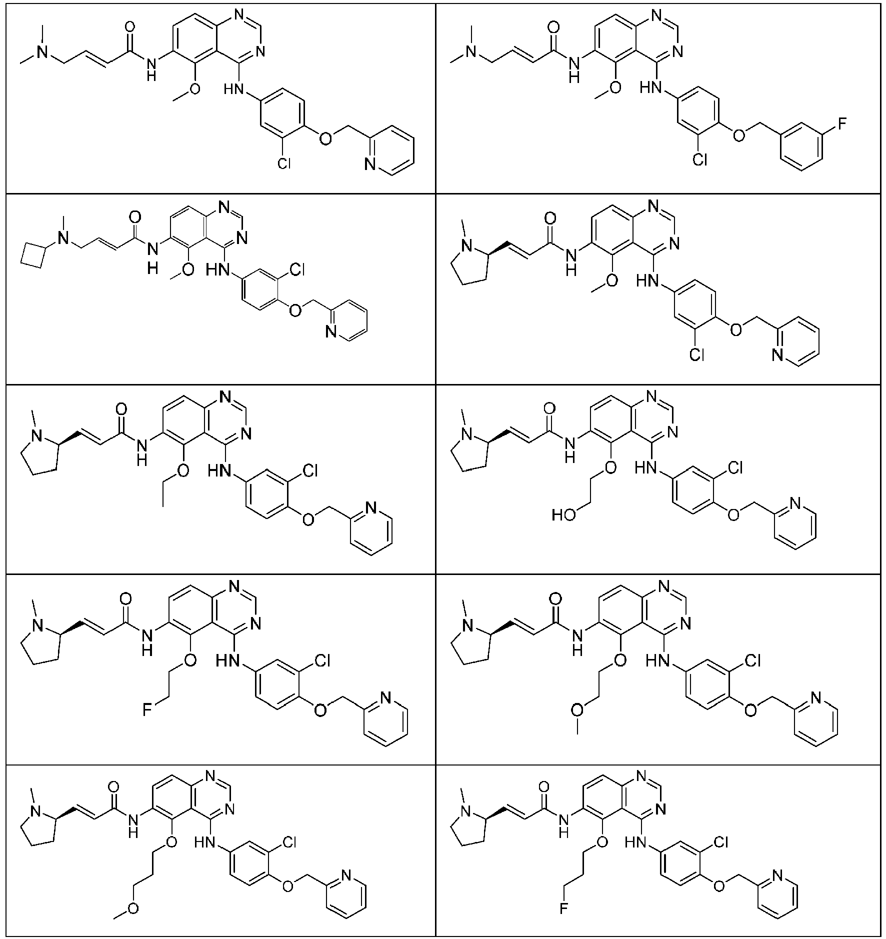

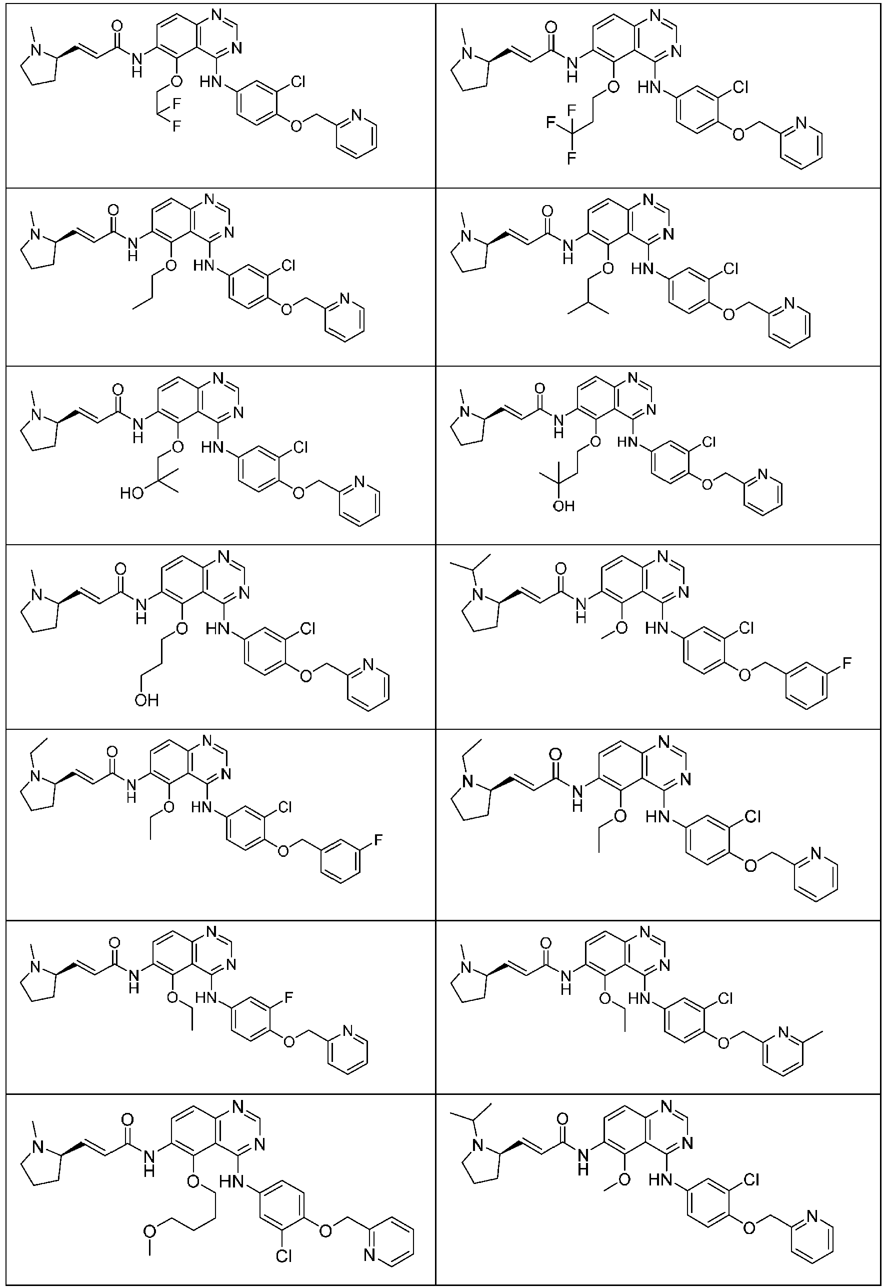

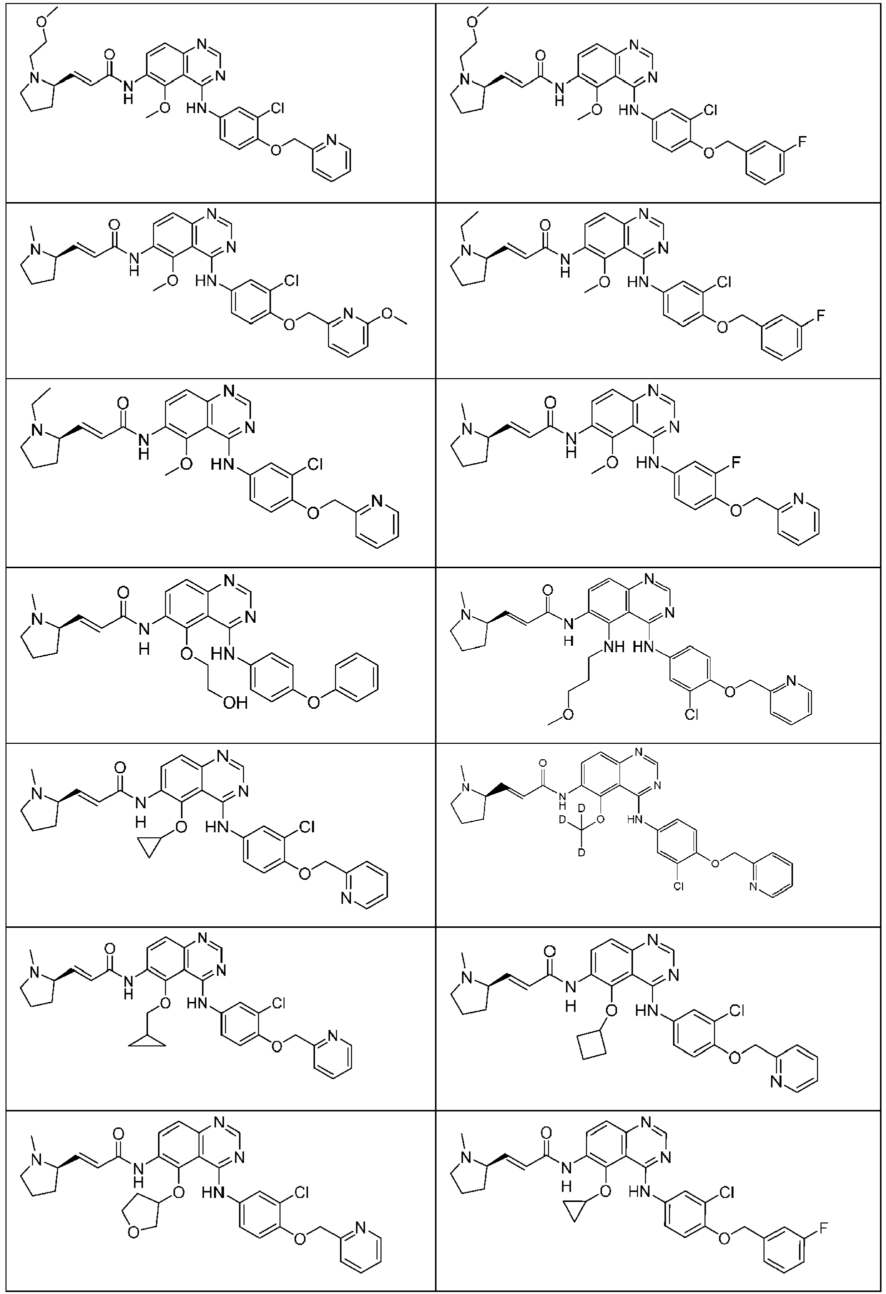

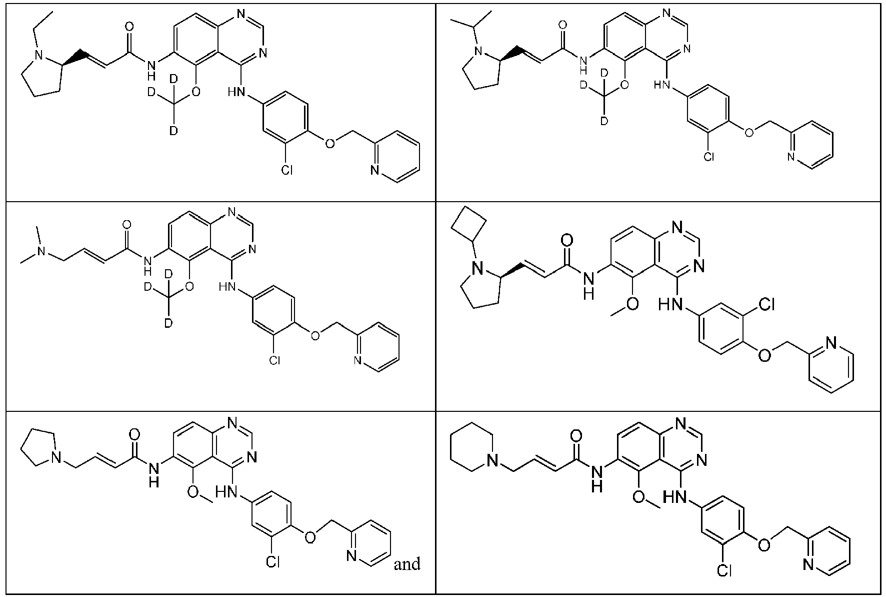

11. A pharmaceutical composition, which comprises the compound of any one of claims 1 to 10, a stereoisomer thereof, a pharmaceutically acceptable salt thereof, or a deuterated derivative thereof, and one or more pharmaceutically acceptable carriers or excipients.

12. The pharmaceutical composition according to claim 11, wherein the pharmaceutical composition also contains one or more other therapeutic agents.

13. Use of the compound of any one of claims 1 to 10, a stereoisomer thereof, a pharmaceutically acceptable salt thereof, or a deuterated derivative thereof in the manufacture of a medicament for the treatment of a disease related to tyrosine kinase HER2.

14. The use according to claim 13, wherein the disease related to tyrosine kinase HER2 is a disease related to mutations in exon 20 of the tyrosine kinase HER2.

15. The use according to claim 13 or 14, wherein the disease is cancer or autoimmune disease, especially ocular fundus disease, xerophthalmia, psoriasis, leucoderma, dermatitis, alopecia areata, rheumatoid arthritis, colitis, multiple sclerosis, systemic lupus erythematosus, Crohn's disease, atherosclerosis, pulmonary fibrosis, liver fibrosis, myelofibrosis, non-small cell lung cancer, small cell lung cancer, breast cancer, pancreatic cancer, glioma, glioblastoma, ovarian cancer, cervical cancer, colorectal cancer, melanoma, endometrial cancer, prostate cancer, bladder cancer, leukemia, gastric cancer, liver cancer, gastrointestinal stromal tumor, thyroid cancer, chronic granulocytic leukemia, acute myelocytic leukemia, non-Hodgkin's lymphoma, nasopharyngeal cancer, esophageal cancer, brain tumor, B-cell and T-cell lymphoma, lymphoma, multiple myeloma, biliary carcinosarcoma, or cholangiocarcinoma.
